(19) Europäisches Patentamt
European Patent Office
Office européen des brevets

(11) Publication number: 0 045 161
B1

(12) # EUROPEAN PATENT SPECIFICATION

(45) Date of publication of patent specification: 14.03.84

(21) Application number: 81303270.3

(22) Date of filing: 16.07.81

(51) Int. Cl.[3]: C 07 C 103/50, C 07 C 103/84, C 07 D 207/16, C 07 D 277/06, C 07 D 279/06, C 07 D 265/06, C 07 D 239/04, C 07 D 307/32, C 07 D 209/20

(54) ## Amides of 4-oxo-5-amidohexanoic acid derivatives.

(30) Priority: 24.07.80 GB 8024305
24.11.80 GB 8037651

(43) Date of publication of application:
03.02.82 Bulletin 82/5

(45) Publication of the grant of the patent:
14.03.84 Bulletin 84/11

(84) Designated Contracting States:
AT BE CH DE FR GB IT LI LU NL SE

(56) References cited:
EP - A - 0 010 347
EP - A - 0 021 883
FR - A - 2 340 933
GB - A - 2 028 794
US - A - 3 526 663

(73) Proprietor: IMPERIAL CHEMICAL INDUSTRIES PLC
Imperial Chemical House Millbank
London SW1P 3JF (GB)

(72) Inventor: Gravestock, Michael Barry
10 Dawlish Avenue Cheadle Hulme
Cheadle Cheshire SK8 6JF (GB)

(74) Representative: Slatcher, Reginald Peter et al,
Imperial Chemical Industries PLC Legal
Department: Patents PO Box 6
Welwyn Garden City Herts, AL7 1HD (GB)

EP 0 045 161 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Amides of 4-oxo-5-amidohexanoic acid derivatives

This invention relates to new amide derivatives and more particularly it relates to amide derivatives which are inhibitors of angiotensin converting enzyme (hereinafter ACE).

Various inhibitors of ACE are known. One is commercially available under the name captopril and has the chemical structure:—

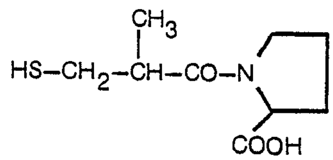

Another, known as MK 421, is at an advanced stage of clinical trial and this has the chemical structure:—

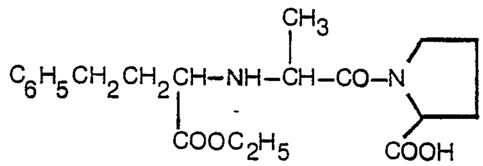

Various analogues of these compounds are described in the literature, especially in patent specifications. No ACE inhibitor is known, however, which bears a ketonic group in the chemical structure, and there are now herein for the first time provided such compounds.

According to the invention there is provided an amide derivative of the formula:—

$$R^1\text{—Y—}NR^2\text{—}CHR^3\text{—X—}CHR^{11}\text{—}CHR^4\text{—CO—}NR^5\text{—}CR^6R^{16}\text{—Q—}R^{10}$$

wherein either $R^1$ is hydrogen, or alkyl of up to 15 carbon atoms which is unsubstituted, or which bears one substituent selected from amino, benzyloxycarbonylamino, hydroxy, alkoxy, alkylthio and alkoxycarbonyl each of up to 5 carbon atoms, and aryl, aryloxy and arylthio substituents; or which bears two or three substituents one of which is aryl, another of which is aryl, hydroxy, amino, benzyloxycarbonylamino, trifluoromethyl, aryloxy or alkoxy of up to 5 carbon atoms and the third of which, if present, is aryl or trifluoromethyl;

or $R^1$ is aryl or, when Y is carbonyl or thiocarbonyl, is aryloxy, alkoxy or arylalkoxy wherein the alkoxy part has up to 5 carbon atoms;

or $R^1$ is halogenoalkyl of up to 6 carbon atoms or is alkenyl, halogenoalkenyl or cycloalkyl each of up to 6 carbon atoms which is unsubstituted or which bears an aryl substituent;

or $R^1$ is a substituent of the formula $R^8CONHCHR^9$— or $R^8COCH_2CHR^9$— wherein $R^8$ is alkyl or cycloalkyl each of up to 10 carbon atoms, or aryl and $R^9$ is hydrogen, or alkyl of up to 5 carbon atoms which is unsubstituted or which bears an aryl substituent, or $R^9$ is a group other than those stated above such that the compound $H_2NCHR^9COOH$ would be a naturally-occurring amino acid; wherein $R^2$ is hydrogen, alkyl of up to 5 carbon atoms which is unsubstituted or which bears an aryl substituent, or aryl;

wherein $R^3$ is alkyl or alkenyl each of up to 5 carbon atoms which is unsubstituted or which bears an aryl substituent, or $R^3$ is aryl or indolylmethyl; wherein $R^4$ is hydrogen or alkyl of up to 5 carbon atoms which is unsubstituted or which bears an aryl substituent; wherein either $R^5$ is hydrogen or aryl, or alkyl of up to 5 carbon atoms which is unsubstituted or which bears an aryl substituent, or $R^5$ is joined together with $R^6$ as defined below;

wherein either $R^6$ is hydrogen, aryl or heterocyclic, or alkyl of up to 5 carbon atoms which is unsubstituted or which bears a hydroxy, aryl or heterocyclic substituent;

or $R^6$ and $R^5$ are joined together to form alkylene or alkenylene of 2 to 5 carbon atoms; or an oxa, thia or aza-derivative of said alkylene or alkenylene; or a hydroxy- or oxo-substituted derivative of said alkylene or alkenylene; or $R^6$ and $R^{16}$, or $R^6$, $R^{16}$ and $R^5$, or $R^6$ and $R^{10}$ are joined together as defined below;

wherein $R^{16}$ is hydrogen or alkyl of up to 5 carbon atoms;

or $R^6$ and $R^{16}$ are joined together to form alkylene of 2 to 5 carbon atoms (that is, to form a spirocycloalkyl group);

or $R^{16}$ together with the first carbon atom of $R^6$ form a double bond wherein $R^6$ is otherwise alkyl, or wherein $R^6$ is otherwise substituted alkyl as defined above, or wherein $R^6$ and $R^5$ are otherwise joined together as defined above (that is, so that $R^5$, $R^6$ and $R^{16}$ together form alkylidene);

wherein Q is carbonyl (—CO—) or methylene (—$CH_2$—);

and wherein either $R^{10}$ is hydroxy, amino, aryloxy, arylthio, alkoxy, cycloalkoxy, alkylamino, dialkylamino, cyclic amino, hydroxyalkoxy, acyloxyalkoxy, aminoalkoxy, alkylaminoalkoxy, dialkylaminoalkoxy, cyclic aminoalkoxy, aminoalkylamino, alkylaminoalkylamino, dialkylaminoalkylamino, cyclic aminoalkylamino or arylalkoxy wherein each alkyl or alkoxy has up to 5 carbon atoms and wherein cyclic amino has up to 6 carbon atoms;

or wherein $R^{10}$ and $R^6$ are joined together such that $R^{10}$ is oxygen (—O—) joined to the terminal carbon atom of $R^6$ when it is alkyl;

wherein $R^{11}$ is hydrogen or alkyl of up to 3 carbon atoms;

wherein X is carbonyl (—CO—), hydroxymethylene (—CHOH—), thiocarbonyl (—CS—) or oximinomethylene (—C=N—$OR^{20}$ wherein $R^{20}$ is hydrogen or alkyl of up to 5 carbon atoms which is unsubstituted or which bears an aryl substituent);

and wherein Y is carbonyl, thiocarbonyl, sulphonyl (—$SO_2$—) or amido (—NHCO—);

or a salt thereof where appropriate.

It will be observed that there are various potentially asymmetrical carbon atoms in the amide of the invention, in particular the carbon atoms which bear the substituents $R^3$, $R^4$ and $R^{11}$ when these substituents are other than hydrogen, and the carbon atom which bears the substituents $R^6$ and $R^{16}$ when these are different one from the other, and that the amide may therefore exist in racemic and optically active forms. It is to be understood that this invention encompasses the racemic form and any optically-active form which possesses ACE- inhibiting properties, it being a matter of common general knowledge how an optically active compound may be prepared and how the ACE- inhibiting properties of a compound may be measured.

A suitable value for $R^1$ or $R^8$ when it is alkyl is, for example, methyl, isobutyl or n-tridecyl.

A suitable value for $R^2$, $R^3$, $R^4$, $R^5$, $R^6$, $R^9$, $R^{16}$ or $R^{20}$ when it is alkyl is, for example, methyl, ethyl, n-propyl or isobutyl.

A suitable value for $R^{11}$ when it is alkyl is, for example, methyl or ethyl.

A suitable value for $R^1$ or $R^{10}$ when it is alkoxy, or for the alkoxy substituent in $R^1$ when said group is alkyl substituted by alkoxy is, for example, methoxy, ethoxy or t-butoxy.

A suitable value for $R^1$ or $R^3$ when it is alkenyl, is, for example, allyl or 2-methylprop-2-enyl.

A suitable value for $R^1$ or $R^8$ when it is cycloalkyl is, for example, cyclopropyl, cyclopentyl or cyclohexyl.

A suitable value for $R^1$, $R^2$, $R^3$, $R^5$, $R^6$ or $R^8$ when it is aryl, or for the aryl substituent in the group $R^1$, $R^2$, $R^3$, $R^4$, $R^5$, $R^6$, $R^9$ or $R^{20}$ when said group is alkyl substituted by aryl, or for the aryl substituent in $R^1$ when said group is alkenyl, halogenoalkenyl or cycloalkyl substituted by aryl, or for the aryl substituent in $R^3$ when said group is alkenyl substituted by aryl, or for the aryl substituent in $R^1$ or $R^{10}$ when it is arylalkoxy, that is, alkoxy substituted by aryl, is, for example, unsubstituted phenyl or naphthyl, or phenyl or naphthyl substituted by one or more substituents selected from halogen, for example fluorine, chlorine, bromine and iodine, alkyl, alkenyl, alkynyl, alkylthio and alkoxy each of up to 5 carbon atoms, for example methyl, ethyl, t-butyl, allyl, propargyl, methylthio, methoxy and ethoxy, and hydroxy, methylenedioxy, amino, nitro, cyano, carboxy, carbamoyl, trifluoromethoxy and trifluoromethyl, aryl, for example phenyl and *p*-chlorophenyl, and substituents of the formula

| | | |
|---|---|---|
| —$COR^{30}$ | —$NHR^{30}$ | —$NR^{30}R^{31}$ |
| —$COOR^{30}$ | —$CONHR^{30}$ | —$CONR^{30}R^{31}$ |
| —$NHCOR^{30}$ | —$NHSO_2R^{30}$ | —$SO_2NR^{30}R^{31}$ |
| —$SR^{30}$ | —$SOR^{30}$ | —$SO_2R^{30}$ |
| —(alk)—$COR^{30}$ | | |
| —(alk)—$COOR^{30}$ | | |

wherein either $R^{30}$ and $R^{31}$, which may be the same or different, each is phenyl, or trifluoromethyl, or alkyl of up to 5 carbon atoms, for example methyl, ethyl or n-propyl, which is unsubstituted or which is substituted by phenyl, or $R^{30}$ and $R^{31}$ are joined such that together with the adjacent nitrogen atom they form pyrrolidino, carboxypyrrolidino, alkoxycarbonylpyrrolidino, piperidino, 4-methylpiperazino or morpholino, and wherein -alk- is alkylene of 1 to 4 carbon atoms.

A suitable value for $R^1$ or $R^{10}$ when it is aryloxy, or for the aryloxy substituent in $R^1$ when $R^1$ is alkyl substituted by aryloxy, is, for example, unsubstituted phenoxy or phenoxy substituted by one or more substituents selected from those stated above as suitable substituents in aryl.

A suitable value for $R^{10}$ when it is arylthio, or for the arylthio substituent in $R^1$ when $R^1$ is alkyl substituted by arylthio is, for example, unsubstituted phenylthio or phenylthio substituted by one or

more substituents selected from those stated in the penultimate paragraph as possible substituents in aryl.

A suitable value for the alkylthio or alkoxycarbonyl substituent in $R^1$ when it is alkyl substituted by alkylthio or alkoxycarbonyl is, for example, methylthio or ethoxycarbonyl.

A suitable value for $R^1$ when it is halogenoalkyl or halogenoalkenyl is, for example, trifluoromethyl or 1-chlorovinyl.

A suitable value for $R^6$ when it is heterocyclic, or for the heterocyclic substituent in $R^6$ when it is alkyl substituted by heterocyclic, is, for example indolyl.

A suitable value for alkylene or alkenylene formed by $R^5$ and $R^6$ joined together is, for example, ethylene, trimethylene, tetramethylene, pentamethylene, propenylene ($-CH_2-CH=CH-$), but-1-enylene ($-CH_2CH_2-CH=CH-$), 2-oxatrimethylene ($-CH_2-O-CH_2-$), 2-thiatrimethylene ($-CH_2-S-CH_2-$), 3-oxatetramethylene ($-CH_2CH_2-O-CH_2-$), 3-thiatetramethylene ($-CH_2CH_2-S-CH_2-$), 3-azatetramethylene ($-CH_2CH_2-NH-CH_2-$), 3-methyl-3-azatetramethylene

$$(-CH_2CH_2-N(CH_3)-CH_2-),$$

2-hydroxytrimethylene ($-CH_2CHOHCH_2-$), 1-oxotrimethylene ($-COCH_2CH_2-$) or 1-oxotetramethylene ($-COCH_2CH_2CH_2-$).

A suitable value for alkylene formed by $R^6$ and $R^{16}$ joined together, is, for example ethylene, trimethylene or tetramethylene.

A suitable value for alkylidene formed by $R^5$, $R^6$ and $R^{16}$ joined together is, for example, propan-1-yl-3-ylidene ($-CH_2CH_2CH=$).

A suitable value for $R^9$ when it is a group derived from a naturally-occurring amino acid is, for example, 3-aminopropyl (from L-ornithine), 4-aminobutyl (from L-lysine), hydroxymethyl (from L-serine), $\alpha$-hydroxyethyl (from L-threonine), $\beta$-hydroxyethyl (from L-homoserine), mercaptomethyl (from L-cysteine), $\beta$-methylthioethyl (from L-methionine), 3-guanidinopropyl (from L-arginine), imidazol-4-ylmethyl (from L-histidine), carboxymethyl (from L-aspartic acid) or 2-carboxyethyl (from glutamic acid).

A suitable value for $R^{10}$ when it is cycloalkoxy, hydroxyalkoxy or acyloxyalkoxy is, for example, cyclopentyloxy, cyclohexyloxy, 2-hydroxyethoxy, 3-hydroxypropoxy or pivaloyloxymethoxy.

A suitable value for $R^{10}$ when it is alkylamino, dialkylamino or cyclic amino is, for example, methylamino, ethylamino, dimethylamino, pyrrolidino, piperidino, 4-methylpiperazino or morpholino.

A suitable value for $R^{10}$ when it is aminoalkoxy, alkylaminoalkoxy, dialkylaminoalkoxy, cyclic aminoalkoxy, aminoalkylamino, alkylaminoalkylamino, dialkylaminoalkylamino or cyclic aminoalkylamino is, for example, ethoxy, propoxy, ethylamino or propylamino substituted in the 2- or 3-position respectively by amino, methylamino, ethylamino, dimethylamino, pyrrolidino, piperidino, 4-methylpiperazino or morpholino. Alternatively, $R^{10}$ when cyclic aminoalkoxy or aminoalkylamino may be a cyclic amine bearing an oxy- or amino-substituent on one of the carbon atoms of the cyclic amine, for example 1-methylpiperidin-4-yloxy or 1-methylpiperidin-4-ylamino.

When Q is carbonyl and $R^6$ and $R^{10}$ are joined together the group $-CR^6R^{16}-Q^3-R^{10}-$ may be, for example, the lactone group 2-oxotetrahydrofuran-3-yl or 2-oxo-tetrahydropyran-3-yl.

Preferably X, Y and Q are all carbonyl and $R^2$ and $R^{11}$ are both hydrogen.

One preferred amide derivative of the invention has the formula given above wherein $R^1$ is alkyl of up to 15 carbon atoms which is unsubstituted or which bears an aryl, aryloxy or arylthio substituent, or $R^1$ is aryl or aryloxy, or $R^1$ is a substituent of the formula $R^8CONHCHR^9$ or $R^8COCH_2CHR^9-$ wherein $R^8$ is alkyl or cycloalkyl each of up to 10 carbon atoms, or aryl, and $R^9$ is hydrogen, or alkyl of up to 5 carbon atoms which is unsubstituted or which bears an aryl substituent, or $R^9$ is a group other than those stated above such that the compound $H_2NCHR^9COOH$ would be a naturally-occurring amino acid;

wherein Y and $R^2$ have the meanings stated above;

wherein $R^3$ is alkyl or alkenyl each of up to 5 carbon atoms which is unsubstituted or which bears an aryl substituent, or $R^3$ is aryl or indolylmethyl;

wherein X is carbonyl, hydroxymethylene, thiocarbonyl or oximinomethylene ($>C=N-OH$);

wherein $R^{11}$ is hydrogen;

wherein $R^4$ is hydrogen or alkyl of up to 5 carbon atoms which is unsubstituted or which bears an aryl substituent;

wherein either $R^5$ is hydrogen, aryl or alkyl of up to 5 carbon atoms which is unsubstituted or which bears an aryl substituent, $R^6$ is hydrogen, alkyl of up to 5 carbon atoms which is unsubstituted or which bears an aryl or heterocyclic substituent, or $R^6$ is heterocyclic or aryl, and $R^{16}$ is hydrogen;

or $R^5$ and $R^6$ are joined together to form alkylene or alkenylene of 2 to 4 carbon atoms; or an oxa, thia or aza-derivative of said alkylene or alkenylene;

or $R^6$, $R^{16}$ and $R^5$ are joined together such that $R^{16}$ together with the first carbon atom of $R^6$ form a double bond wherein $R^6$ and $R^5$ are otherwise joined together as defined above;

wherein Q is carbonyl;

and wherein $R^{10}$ has the formula —$OR^7$, wherein $R^7$ is hydrogen or alkyl of up to 5 carbon atoms. Thus, a preferred amide derivative has the formula:—

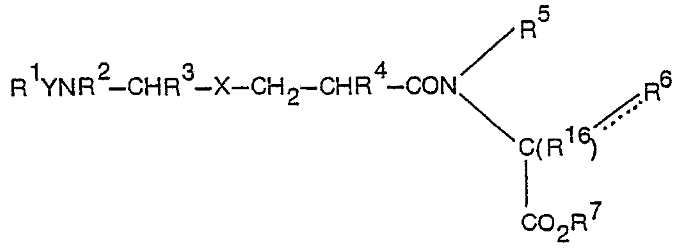

wherein $R^1$, $R^2$, $R^3$, $R^4$, $R^5$, $R^6$, $R^7$, $R^{16}$, X and Y have the meanings stated in this paragraph.

A particularly preferred amide derivative of the invention has the formula

$$R^1CONH—CHR^3—COCH_2—CHR^4—CONR^5—CHR^6—COR^{10}$$

wherein $R^1$ is methyl, benzyl, β-phenylethyl, benzyloxy, phenoxymethyl, phenylthiomethyl, phenyl, tolyl, methoxyphenyl, methylthiophenyl, monochlorophenyl, dichlorophenyl, fluorophenyl, iodophenyl, nitrophenyl, cyanophenyl, trifluoromethylphenyl, acetamidophenyl, acetylphenyl, methanesulphonylphenyl, biphenylyl, naphthyl, benzamidomethyl, cyclopentanecarbonamidomethyl or β-benzoylethyl;
$R^3$ is benzyl which is unsubstituted or bears a methyl, t-butyl, methoxy, fluoro, chloro, bromo, nitro, cyano, trifluoromethyl, amino, acetamido, methanesulphonamido, trifluoromethanesulphonamido or phenyl substituent, or
$R^3$ is 3-phenylpropyl or 3-phenylprop-2-enyl;
$R^4$ is hydrogen or methyl;
$R^5$ is hydrogen or methyl and $R^6$ is hydrogen, benzyl or indol-3-ylmethyl, or $R^5$ and $R^6$ together form trimethylene, tetramethylene, pentamethylene, 2-hydroxytrimethylene, 2-thiatrimethylene, 3-thiatetramethylene, 3-oxatetramethylene, 3-methyl-3-azatetramethylene; and
$R^{10}$ is hydroxy, amino, alkoxy of up to 5 carbon atoms, especially methoxy, ethoxy, isopropoxy or t-butoxy, or β-dimethylaminoethoxy or β-morpholinoethoxy.

An especially preferred amide derivative of the invention has the formula

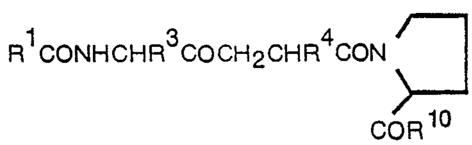

wherein $R^1$ is phenyl, *p*-acetamidophenyl or *p*-cyanophenyl, $R^3$ is benzyl, *p*-methylbenzyl or *p*-methoxybenzyl, $R^4$ is hydrogen or methyl and $R^{10}$ is hydroxy, amino, methoxy, ethoxy, isopropoxy or t-butoxy.

Specific compounds of the invention are hereinafter described in the Examples, and of these-preferred compounds are *N*-(5-acetamido-2-methyl-4-oxo-6-phenylhexanoyl-L-proline, *N*-(5-benzamido-2-methyl-4-oxo-6-phenylhexanoyl)-L-proline and *N*-(5-phenylacetamido-2-methyl-4-oxo-6-phenylhexanoyl)-L-proline, and salts thereof, and esters thereof with an alcohol of up to 5 carbon atoms, for example methyl and t-butyl esters thereof, and amides thereof. Of these preferred compounds are *N*-(5-benzamido-2-methyl-4-oxo-6-phenylhexanoyl)-L-proline, and its methyl ester, and its amide.

A suitable salt of an amide derivative of the invention wherein $R^{10}$ is hydroxy is, for example, an alkali metal or alkaline earth metal salt, for example a sodium, potassium, calcium or magnesium salt, or an ammonium or dicyclohexylamine salt.

An amide derivative of the invention may be manufactured by any chemical process known to be suitable for preparing compounds of related chemical types.

A preferred process for the manufacture of an amide derivative of the invention comprises the reaction of an acid of the formula:—

$$R^1—Y—NR^2—CHR^3—X—CHR^{11}—CHR^4—COOH$$

wherein $R^1$, $R^2$, $R^3$, $R^4$, $R^{11}$, X and Y have the meanings stated above, or of a reactive derivative thereof, with an amine of the formula:—

$$HNR^5—CR^6R^{16}—Q—R^{12}$$

wherein $R^5$, $R^6$, $R^{16}$ and Q have the meanings stated above and wherein $R^{12}$ has any of the meanings stated above for $R^{10}$ except those containing a free hydroxy or amino group.

The abovementioned reaction may be carried out by reacting the acid and amine together in the presence of a carbodiimide, for example dicyclohexylcarbodiimide, and N-hydroxybenzotriazole, in a diluent or solvent, for example tetrahydrofuran, methylene chloride or dimethylformamide, at ambient temperature or below.

The acid starting material for the above reaction wherein $R^2$ is hydrogen and X and Y are both carbonyl, that is, having the formula:—

$$R^1CONH—CHR^3—CO—CHR^{11}—CHR^4—COOH$$

may be obtained by the reaction of a compound of the formula:—

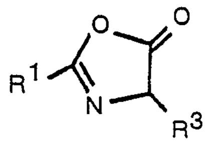

wherein $R^1$ and $R^3$ have the meanings stated above, with an acid chloride of the formula:—

$$ClCO—CHR^{11}—CHR^4—COOR^{13}$$

wherein $R^4$ and $R^{11}$ have the meanings stated above and wherein $R^{13}$ is alkyl of up to 5 carbon atoms, followed by the hydrolysis of the oxazolone ring and also hydrolysis to replace $R^{13}$ by hydrogen. The starting material wherein $R^2$ is alkyl, arylalkyl or aryl may be obtained by the reaction of the intermediate thus obtained, wherein $R^{13}$ is alkyl or has been replaced by hydrogen, with a compound of the formula $R^2Z$, wherein $R^2$ has the meaning stated above and wherein Z is a displaceable group such as a halogen atom or an alkanesulphonyl or arenesulphonyl group, for example a chlorine, bromine or iodine atom or a methanesulphonyl or toluene-*p*-sulphonyl group.

Alternatively, the starting material wherein $R^2$ is hydrogen and X is carbonyl or thiocarbonyl may be obtained by the reaction of a compound of the formula:—

$$H_2NCHR^3X^1CHR^{11}CHR^4COOR^{13}$$

wherein $R^3$, $R^4$, $R^{11}$ and $R^{13}$ have the meanings stated above and $X^1$ is carbonyl or thiocarbonyl, with a compound of the formula $R^1—Y—Z^1$, wherein $R^1$ and Y have the meanings stated above and wherein $Z^1$ is a displaceable group such as is defined above for Z, or $Z^1$ is hydroxy (in which case the reaction is carried out in the presence of a carbodiimide or an alkyl chloroformate), or, when Y is amido, with a compound of the formula $R^1NCO$, or, when Y is thiocarbonyl, with a compound of the formula $R^1CS—SCH_2COOH$.

An alternative process for the manufacture of an amide derivative of the invention wherein $R^2$ is hydrogen and X is carbonyl or thiocarbonyl comprises the reaction of an amine of the formula:

$$H_2NCHR^3—X^1—CHR^{11}CHR^4CONR^5—CR^6R^{16}—Q—R^{12}$$

wherein $R^3$, $R^4$, $R^5$, $R^6$, $R^{11}$, $R^{12}$, $R^{16}$, Q and $X^1$ have the meanings stated above, with a compound of the formula $R^1—Y—Z^1$, wherein $R^1$, Y and $Z^1$ have the meanings stated above.

Yet an alternative process for the manufacture of an amide derivative of the invention wherein $R^2$ is hydrogen and X is a carbonyl comprises the reaction of a compound of the formula:—

$$Z^2—COCHR^{11}CHR^4CONR^5CR^6R^{16}QR^{12}$$

wherein $R^4$, $R^5$, $R^6$, $R^{11}$, $R^{12}$, $R^{16}$, and Q have the meanings stated above and $Z^2$ is a displaceable halogen atom, for example the chlorine or bromine atom, with a compound of the formula

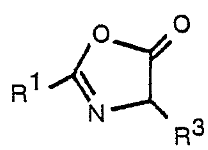

wherein $R^1$ and $R^3$ have the meanings stated above, followed by hydrolysis of the oxazolone ring.

The last-mentioned reaction may be carried out in the presence of a base, for example triethylamine and/or 4-dimethylaminopyridine, and it may be carried out in a diluent or solvent, for example tetrahydrofuran.

An amide derivative of the invention wherein $R^2$ is alkyl, arylalkyl or aryl (and $R^{10}$ is $R^{12}$) may be obtained by reacting the corresponding amide derivative of the invention wherein $R^2$ is hydrogen (and $R^{10}$ is $R^{12}$) with a compound of the formula $R^2Z$, wherein $R^2$ and Z have the meanings stated above.

An amide derivative of the invention wherein $R^3$ is unsubstituted or substituted alkyl as defined above may be obtained by the hydrogenation of the corresponding compound wherein $R^3$ is unsubstituted or substituted alkenyl as defined above.

An amide derivative of the invention wherein $R^{10}$ is other than hydroxy (that is, wherein Q—$R^{10}$ forms an ester or amide group) may be obtained from the corresponding acid wherein Q—$R^{10}$ is —COOH by conventional means of ester or amide formation.

An amide derivative of the invention wherein Q is carbonyl and $R^{10}$ is hydroxy may be obtained by the hydrolysis of the corresponding amide derivative of the invention wherein $R^{10}$ is alkoxy, or, when $R^{10}$ is t-butoxy, by the acid-catalysed cleavage of said compound.

An amide derivative of the invention wherein X is hydroxymethylene may be obtained by the reduction, for example with an alkali metal borohydride, of the corresponding amide derivative of the invention wherein X is carbonyl, and an amide derivative of the invention wherein X is oximinomethylene may be obtained by the reaction of the corresponding amide derivative of the invention wherein X is carbonyl with hydroxylamine or an *O*-substituted hydroxylamine derivative of the formula $H_2NOR^{20}$, wherein $R^{20}$ has the meaning stated above.

As stated above, an amide derivative of the invention possesses ACE-inhibiting properties. ACE is the enzyme which converts angiotensin I to angiotensin II. The ACE-inhibiting properties of an amide derivative of the invention may be demonstrated by its ability to prevent the cleavage of angiotensin I or of a synthetic peptide related to angiotensin I by ACE.

Angiotensin II is a potent constrictor of vascular smooth muscle, and is therefore involved in the control of blood pressure. A compound which prevents conversion of angiotensin I to angiotensin II will therefore lower circulating levels of angiotensin II and cause a fall in blood pressure. An amide derivative of the invention may therefore be used to lower blood pressure in a warm-blooded animal (including a human). At a dose of amide derivative of the invention which lowers blood pressure in an experimental animal, for example a rat, no symptoms of toxicity are apparent.

An amide derivative of the invention may be administered to a warm-blooded animal, including man, in the form of a pharmaceutical composition comprising as active ingredient at least one amide derivative of the invention, or a salt thereof, in association with a pharmaceutically-acceptable diluent or carrier therefor.

A suitable composition is, for example, a tablet, capsule, aqueous or oily solution or suspension, emulsion, injectable aqueous or oily solution or suspension, dispersible powder, spray or aerosol formulation.

The pharmaceutical composition may contain, in addition to the amide derivative of the invention, one or more drugs selected from diuretics, for example bendrofluazide, chlorothiazide and chlorthalidone; and other hypotensive agents, for example β-adrenergic blocking agents, for example atenolol and propanolol.

When used for the treatment of hypertension in man, it is expected that the amide derivative of the invention would be given to man at a total oral dose of between 1 mg. and 500 mg. daily, at doses spaced at 6—8 hourly intervals, or at an intravenous dose of between 0.1 mg. and 50 mg.

Preferred oral dosage forms are tablets or capsules containing between 1 mg. and 100 mg. of active ingredient. Preferred intravenous dosage forms are sterile aqueous solutions of active ingredient containing between 0.1% and 1% w/v of active ingredient.

The following is illustrated but not limited by the following Examples:—

Example 1

Dicyclohexylcarbodi-imide (3.0 g.) was added to a stirred, ice-cooled solution of (R,S)-5-acetamido-(R,S)-2-methyl-4-oxo-6-phenylhexanoic acid (2.77 g.), *N*-hydroxybenzotriazole (1.4 g.) and L-proline t-butyl ester (1.88 g.) in dry tetrahydrofuran (35 ml.), and the mixture was allowed to warm up to laboratory temperatures and was then stirred at that temperature for 24 hours. Acetic acid (5 ml.) was added and 15 minutes after that addition the mixture was partitioned between ethyl acetate (200 ml.) and aqueous 10% w/v sodium carbonate solution (75 ml.). The ethyl acetate layer was separated, washed successively twice with aqueous 10% w/v sodium carbonate solution, three times with aqueous N-hydrochloric acid and once with saturated anhydrous sodium chloride solution, filtered through anhydrous sodium sulphate and evaporated to dryness under reduced pressure. The residue was dissolved in diethyl ether, the solid dicyclohexylurea was filtered off and the ethereal solution was evaporated to dryness. The residue was dissolved in methylene chloride and chromatographed on a silica gel column (344 mm. x 42 mm., Merck K60) by elution with methylene chloride and then with a 1:1 v/v mixture of methylene chloride and ethyl acetate. There was thus obtained, as an oil, *N*-[(R,S)-5-acetamido-(R,S)-2-methyl-4-oxo-6-phenylhexanoyl]-L-proline t-butyl ester (Compound No. 1), the structure of which was confirmed by elemental analysis (C, H and N), by proton magnetic resonance spectroscopy and by mass spectroscopy.

*Elemental Analysis*
Found: C, 64.9%; H, 8.0%; N, 5.8%. $C_{24}H_{34}N_2O_5.H_2O$ requires C, 64.4%; H, 8.05%; N, 6.2%.

*Proton Magnetic Resonance Spectrum* (in $CDCl_3$)

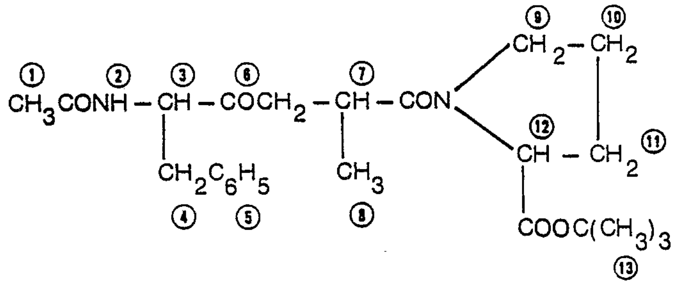

$C_{24}H_{34}N_2O_5$

| Shift (δ). | Type of Peak | No. of H Atoms | Specific H atoms |
|---|---|---|---|
| 1.15 | t | 3 | ⑧ |
| 1.45 | s | 9 | ⑬ |
| 1.95 | d | 3 | ① |
| 1.70—2.3 | m | 6 | ④, ⑩, ⑪ |
| 3.0 | m | 3 | ⑥, ⑦ |
| 3.60 | m | 2 | ⑨ |
| 4.31 | m | 1 | ③ |
| 4.70 | m | 1 | ⑫ |
| 6.25/6.55/6.68 | m | 1 | ② |
| 7.20 | s | 5 | ⑤ |

*Mass Spectrum* (70 ev)

| Mass No. | Ion |
|---|---|
| 431 | $M^+ + 1$ |
| 430 | $C_{24}H_{34}N_2O_5(M^+)$ |
| 374 | M—$C_4H_8$ |
| 357 | M—$C_4H_9O$ |
| 329 | M—$COOC(CH_3)_3$ |
| 268 | M—$CH_3CONHCH(CH_2C_6H_5)$ |
| 260 | M—Proline t-butyl ester |
| 260 | M—$CH_3CONH$ |

The (R,S)-5-acetamido-(R,S)-2-methyl-4-oxo-6-phenylhexanoic acid used as starting material was prepared as follows:—

A solution of 3-methoxycarbonylbutyric acid (29.2 g.; J.Org.Chem., 1972, *37*, 555) and oxalyl chloride (31.5 g.) in benzene (100 ml.) was stirred at laboratory temperature for 4 hours, evaporated to

dryness under reduced pressure and then twice re-evaporated to dryness from an excess of methylene chloride. A solution of the 3-methoxycarbonylbutyryl chloride thus obtained (20 g.) in dry tetrahydrofuran (40 ml.) was added dropwise during 15 minutes to a stirred solution of freshly distilled 4-benzyl-2-methyloxazol-5(4H)-one (17.7 g.; prepared from phenylalanine and acetic anhydride as described in Annalen, 1926, *449*, 277), 4-dimethylaminopyridine (0.7 g.) and triethylamine (27 ml.) in dry tetrahydrofuran (100 ml.) which had been cooled to —50°C., at such a rate that the temperature of the mixture did not exceed —30°C. The stirred mixture was allowed to warm up to laboratory temperature during 4 hours and was then stirred at laboratory temperature for 70 hours. Acetic acid (30 ml.) was added and the mixture was allowed to stand at laboratory temperature for 1 hour and then evaporated to dryness. The residue was partitioned between ethyl acetate (500 ml.) and aqueous 2N-hydrochloric acid (200 ml.), and the ethyl acetate layer was washed successively twice with aqueous 2N-hydrochloric acid, three times with 10% w/v aqueous sodium carbonate solution and once with saturated aqueous sodium chloride solution, filtered through anhydrous sodium sulphate and evaporated to dryness under reduced pressure. The residue was purified by chromatography on a 30 cm. x 5 cm. silica gel column using a 1:1 v/v mixture of ethyl acetate and methylene chloride as eluant, and there was thus obtained methyl (R,S)-5-acetamido-(R,S)-2-methyl-4-oxo-6-phenylhexanoate (24.75 g.).

A mixture of the above ester (12.6 g.) and aqueous 2N-sodium hydroxide solution (100 ml.) was kept at laboratory temperature for 4 hours, washed twice with diethyl ether and then acidified with aqueous 2N-hydrochloric acid and extracted with ethyl acetate. The extract was washed with saturated aqueous sodium chloride solution, filtered through anhydrous sodium sulphate and evaporated to dryness under reduced pressure. The residue was crystallised from a mixture of ethyl acetate and ether and there was thus obtained (R,S)-5-acetamido-(R,S)-2-methyl-4-oxo-6-phenylhexanoic acid, m.p. 115—127°C.

Example 2

The process described in Example 1 was repeated except that the appropriate 4-oxo-6-phenylhexanoic acid and the appropriate L-proline ester were used as starting materials. There were thus obtained the compounds described in the following table:—

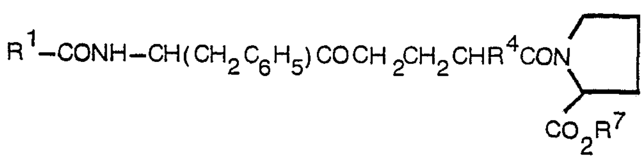

| $R^1$ | $R^4$ | $R^7$ | Compound No |
|---|---|---|---|
| methyl | methyl | methyl | 2 |
| methyl | H | methyl | 3 |
| methyl | benzyl | methyl (more polar isomer) | 4 |
| methyl | benzyl | methyl (less polar isomer) | 5 |
| phenyl | methyl | t-butyl | 6 |
| phenyl | H | t-butyl | 7 |
| benzyl | methyl | t-butyl (more polar isomer) | 8 |
| benzyl | methyl | t-butyl (less polar isomer) | 9 |
| benzyl | H | t-butyl | 10 |
| benzamidomethyl | H | t-butyl | 11 |
| cyclopentanecarbon-amidomethyl | methyl | t-butyl | 12 |
| cyclopentanecarbon-amidomethyl | H | t-butyl | 13 |
| n-tridecyl | methyl | t-butyl | 14 |

The isomeric Compounds Nos. 4 and 5, and Compounds Nos. 8 and 9, were separated from each other by the final chromatography step. The structures of all the compounds were confirmed by elemental analysis, proton magnetic resonance spectroscopy and also (in the case of Compounds Nos. 2, 3, 4, 5, 6, 10, 11 and 13) by mass spectroscopy.

Example 3

The process described in Example 1 was repeated except that L-phenylalanine ethyl ester was used in place of L-proline t-butyl ester. There was thus obtained, as an oil, *N*-[(R,S)-5-acetamido-(R,S)-2-methyl-4-oxo-6-phenylhexanoyl]-L-phenylalanine ethyl ester (Compound No. 15), the structure of which was confirmed by elemental analysis, proton magnetic resonance spectroscopy and mass spectroscopy.

Example 4

The process described in Example 1 was repeated except that (R,S)-5-acetamido-4-oxo-6-phenylhexanoic acid and *N*-benzylglycine ethyl ester were used as starting materials. There was thus obtained *N*-[(R,S)-5-acetamido-4-oxo-6-phenylhexanoyl]-*N*-benzylglycine ethyl ester (Compound No. 16), the structure of which was confirmed by elemental analysis, proton magnetic resonance spectroscopy and mass spectroscopy.

The starting materials (hexanoic acids) used to prepare Compounds Nos. 2 to 14 and 16 described in Examples 2 and 4 were prepared by similar methods to those used to prepare the hexanoic acid described in the second part of Example 1. Particular intermediates used were as follows:—

(a) 3-ethoxycarbonyl-4-phenylbutyric acid (for Compounds Nos. 4 and 5); monomethyl succinate (for Compounds Nos. 3, 7, 10, 11, 13 and 16).

(b) 2,4-dibenzyl-oxazol-5(4H)-one (for Compounds Nos. 8, 9 and 10); 4-benzyl-2-phenyloxazol-(4H)-one (for Compounds Nos. 6 and 7) (the preparation of the two last-mentioned compounds is described below).

(c) methyl (R,S)-5-benzamido-(R,S)-2-methyl-4-oxo-6-phenylhexanoate (for Compound No. 6); ethyl (R,S)-5-phenylacetamido- (R,S)-2-methyl-4-oxo-6-phenylhexanoate (for Compounds Nos. 8 and 9); ethyl (R,S)-5-acetamido-(R,S)-2-benzyl-4-oxo-6-phenylhexanoate (for Compounds Nos. 4 and 5); methyl (R,S)-5-benzamido-4-oxo-6-phenylhexanoate (for Compound No. 7); methyl (R,S)-5-acetamido-4-oxo-6-phenylhexanoate (for Compounds Nos. 3 and 16).

The 2,4-dibenzyl-oxazol-5(4H)-one used as an intermediate in the preparation of Compounds Nos. 8 and 9 was prepared by the reaction of (*N*-phenylacetyl)-phenylalanine with dicyclohexylcarbodi-imide, and the 4-benzyl-2-phenyloxazol-5(4H)-one was similarly prepared from (*N*-benzoyl)-phenylalanine.

The hexanoic acid starting materials used for the preparation of Compounds Nos. 10 to 14 (and also alternatively for Compounds Nos. 8 and 9) were prepared from the appropriate methyl (R,S)-5-acetamido-4-oxo-6-phenylhexanoate, by replacing the 5-acetamido group by the appropriate amido group, as exemplified by the following procedure:

A mixture of methyl (R,S)-5-acetamido-(R,S)-2-methyl-4-oxo-6-phenylhexanoate (10.11 g.) and aqueous 6N-hydrochloric acid (100 ml.) was heated at 100°C. for 6 hours, kept at laboratory temperature for 17 hours and then evaporated to dryness under reduced pressure. Thionyl chloride (5 ml.) was added during 15 minutes to a stirred, ice-cooled solution of the residue in methanol (100 ml.) and the mixture was stirred at laboratory temperature for 3 hours and then evaporated to dryness under reduced pressure. A mixture of the methyl (R,S)-5-amino-(R,S)-2-methyl-4-oxo-6-phenylhexanoate hydrochloride thus obtained (5.7 g.), (*N*-cyclopentanecarbonyl)glycine (2.87 g.) and tetrahydrofuran (50 ml.) was stirred and ice-cooled, and *N*-hydroxybenzotriazole (2.6 g.), triethylamine (10 ml.) and dicyclohexylcarbodi-imide (4.12 g.) were sequentially added. The mixture was kept at laboratory temperature for 17 hours and filtered, and the filtrate was evaporated to dryness under reduced pressure. The residue was partitioned between ethyl acetate and aqueous 2N-hydrochloric acid, and the ethyl acetate layer was separated, washed successively twice with aqueous 2N-hydrochloric acid, three times with aqueous 10% w/v sodium carbonate solution and once with saturated aqueous sodium chloride solution, filtered through anhydrous sodium sulphate and evaporated to dryness. The residue was stirred with diethyl ether, the solid dicyclohexylurea was filtered off and the ethereal solution was evaporated to dryness. The residue was dissolved in methylene chloride and chromatographed on a silica gel column using a 1:1 v/v mixture of methylene chloride and ethyl acetate as eluant. There was thus obtained, as an oil, methyl (R,S)-5-(*N*-cyclopentanecarbonyl)glycinamido-(R,S)-2-methyl-4-oxo-6-phenylhexanoate.

This ester was then hydrolysed to the hexanoic acid by a similar procedure to that described in the last paragraph of Example 1. There was thus obtained (R,S)-5-(*N*-cyclopentanecarbonyl)glycinamido-(R,S)-2-methyl-4-oxo-6-phenylhexanoic acid, the structure of which was confirmed by proton magnetic resonance spectroscopy and mass spectroscopy. This compound was used to prepare Compound No. 12. There were similarly obtained the compounds shown in the following table, all of which structures were similarly confirmed:—

$R^1$—CONH—$CH(CH_2C_6H_5)COCHR^4COOH$

| $R^1$ | $R^4$ | Intermediate for Compound No. |
|---|---|---|
| cyclopentanecarbon amidomethyl | H | 13 |
| benzamidomethyl- | H | 11 |
| benzyl | methyl | 8.9 |
| benzyl | H | 10 |
| tridecyl | methyl | 14 |

Example 5

A mixture of *N*-[(R,S)-5-acetamido-(R,S)-2-methyl-4-oxo-6-phenylhexanoyl]-L-proline t-butyl ester (Example 1, Compound No. 1, 3.04 g.) and a 9:1 v/v mixture of trifluoroacetic acid and water (20 ml.) was stirred at laboratory temperature for 2 hours, evaporated to dryness under reduced pressure and then re-evaporated to dryness from toluene and then from cyclohexane. The residue was dissolved in methylene chloride and chromatographed on silica gel using a 3% v/v solution of methanol in methylene chloride as eluant. There was thus obtained *N*-[(R,S)-5-acetamido-(R,S)-2-methyl-4-oxo-6-phenylhexanoyl]-L-proline (Compound No. 21), the structure of which was confirmed by proton magnetic resonance spectroscopy and by mass spectroscopy.

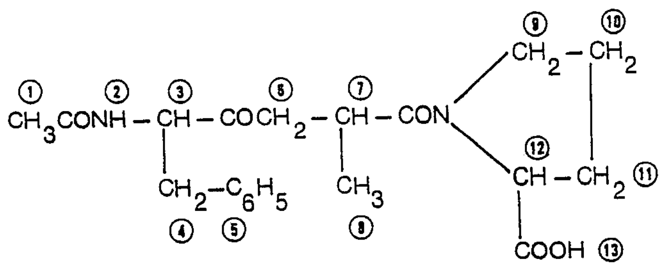

$C_{20}H_{26}N_2O_5$

| Shift (δ) | Type of Peak | No. of H Atoms | Specific H atoms |
|---|---|---|---|
| 1.08 | t | 3 | ⑧ |
| 1.91 | m | 3 | ① |
| 2.10 | m | 5 | ⑦, ⑩,, ⑪ |
| 3.00 | m | 4 | ④, ⑥ |
| 3.62 | m | 2 | ⑨ |
| 4.45 | m | 1 | ③ |
| 4.71 | t | 1 | ⑫ |
| 6.50 | t | 1 | ② |
| 7.20 | s | 5 | ⑤ |
| 8.24 | s | 1 | ⑬ |

*Mass Spectrum* (70 ev)

| Mass No. | Ion |
|---|---|
| 374 | $C_{20}H_{26}N_2O_5$ ($M^+$) |
| 356 | M—$H_2O$ |
| 330 | M—$CO_2$ |
| 283 | M—$CH_2C_6H_5$ |
| 260 | M—Proline |
| 212 | M—$CH_3CONHCH(CH_2C_6H_5)$ |
| 168 | (212—$CO_2$) |
| 162 | $CH_3CONH^{\oplus}{=}CHCH_2C_6H_5$ |

A sample of Compound 21 was dissolved in ethyl acetate and a solution of dicyclohexylamine in a mixture of ethyl acetate and ether was added. The mixture was filtered and the solid residue was crystallised from ethyl acetate. There was thus obtained the hemihydrate of the dicyclohexylamine salt of Compound 21, m.p. 191—194°C., the structure of which was confirmed by elemental analysis (Found: C, 68.5%; H, 9.1%; N, 7.2%. $C_{32}H_{49}N_3O_5.\frac{1}{2}H_2O$ requires: C, 68.1%; H, 8.9%; N, 7.4%).

Example 6

The process described in Example 5 was repeated using the appropriate ester as starting material. There were thus obtained the compounds described in the following table:

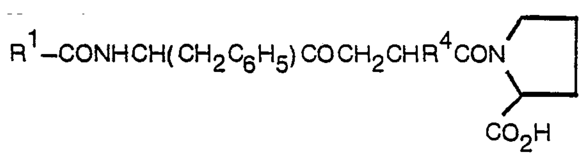

| Starting Material Compound No. | $R^1$ | $R^4$ | Acid Compound No. |
|---|---|---|---|
| 6 | phenyl | methyl | 26 |
| 7 | phenyl | H | 27 |
| 8 | benzyl | methyl | 28 |
| 9 | benzyl | methyl | 29 |
| 10 | benzyl | H | 30 |
| 11 | benzamidomethyl | H | 31 |
| 12 | cyclopentanecarbon-amidomethyl | methyl | 32 |
| 13 | cyclopentanecarbon-amidomethyl | H | 33 |

The structures of all these compounds were confirmed by proton magnetic resonance spectroscopy.

Example 7

A solution of potassium hydroxide (0.7 g.) in water (10 ml.) was added to a solution of *N*-[(R,S)-5-acetamido-4-oxo-6-phenylhexanoyl]-L-proline methyl ester (Example 2, Compound No. 3, 2.15 g.) in

ethanol (40 ml.) and the mixture was stirred at laboratory temperature for 1 hour, acidified to pH 4 with aqueous 2N-hydrochloric acid, diluted with saturated aqueous sodium chloride solution and extracted three times with chloroform (15 ml. each time). The combined extracts were washed with saturated aqueous sodium chloride solution and then evaporated to dryness. There was thus obtained *N*-[(R,S)-5-acetamido-4-oxo-6-phenylhexanoyl]-L-proline (Compound No. 23), m.p. 50—60°C., the structure of which was confirmed by elemental analysis and proton magnetic resonance spectroscopy.

The process described above was repeated using the appropriate methyl or ethyl ester as starting material and there were thus obtained the compounds shown in the following table:

$R^1CONHCH(CH_2C_6H_5)COCH_2CHR^4CONR^5CHR^6COOH$

| Starting Material Compound No. | $R^1$ | $R^4$ | $R^5$ | $R^6$ | Acid Compound No. |
|---|---|---|---|---|---|
| 4 | methyl | benzyl | $—(CH_2)_3—$ | | 24 |
| 5 | methyl | benzyl | $—(CH_2)_3—$ | | 25 |
| 15 | methyl | methyl | H | benzyl | 35 |
| 16 | methyl | H | benzyl | H | 36 |

The structures of all these compounds were confirmed by proton magnetic resonance spectroscopy.

Example 8

The process described in Example 1 was repeated except that the appropriate (R,S)-5-acylamino-4-oxo-6-phenylhexanoic acid (prepared by a similar process to that described in Example 4 for the preparation of intermediates for Compounds Nos. 10 to 14) and L-proline t-butyl ester were used as starting materials. There were thus obtained, as oils, the compounds described in the following table, the structures of which were all confirmed by elemental analysis, proton magnetic resonance spectroscopy and mass spectroscopy:—

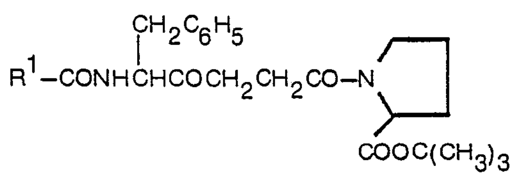

| $R^1$ | Compound No. |
|---|---|
| 4-acetamidophenyl | 17 |
| 4-methoxyphenyl | 18 |
| 2-naphthyl | 19 |
| β-phenylethyl | 20 |
| β-benzoylethyl | 41 |
| γ-phenoxypropyl | 42 |
| benzyloxy | 43 |
| 4-nitrophenyl | 278 |
| 4-chlorophenyl | 279 |

Example 9

N,N'-Dicyclohexylcarbodi-imide (38.3 g.) was added to a stirred solution of (R,S)-5-benzyloxycarbonylamino-4-oxo-6-phenylhexanoic acid (60 g.), L-proline t-butyl ester (30.5 g.) and *N*-hydroxybenzotriazole (24 g.) in dry tetrahydrofuran (750 ml.) which was cooled to 0°C., and the mixture was allowed to warm up to laboratory temperature and was then stirred for 18 hours. The mixture was cooled to 0°C and filtered, and the filtrate was evaporated to dryness under reduced pressure. The residue was dissolved in ethyl acetate and the solution was washed successively with aqueous 3% w/v citric acid solution, aqueous 5% w/v sodium bicarbonate solution and saturated aqueous sodium chloride solution, dried over sodium sulphate and evaporated to dryness under reduced pressure. The residual oil was purified by chromatography on a silica gel column using a 1:1 v/v mixture of ethyl acetate and methylene chloride as eluant, and there was thus obtained as an oil *N*-[(R,S)-5-benzyloxycarbonylamino-4-oxo-6-phenylhexanoyl]-L-proline t-butyl ester (Compound No. 43).

A solution of the above oil, which consists of two isomers (59 g.), in diethyl ether (300 ml.) was kept at laboratory temperature for 18 hours and then filtered. The solid product was crystallised from a mixture of ethyl acetate and petroleum ether (b.p. 40—60°C.) and there was thus obtained one isomer (hereinafter Isomer A) of *N*-(5-benzyloxycarbonylamino-4-oxo-6-phenylhexanoyl)-L-proline t-butyl ester, m.p. 126—128°C. (Compound No. 43A).

The diethyl ether mother liquors from the initial crystallisation were evaporated to dryness under reduced pressure. There was thus obtained as an oil a second isomer (hereinafter Isomer B) of *N*-(5-benzyloxycarbonylamino-4-oxo-6-phenylhexanoyl)-L-proline t-butyl ester (Compound No. 43B).

The structures of both isomers A and B were confirmed by elemental analysis, proton magnetic resonance spectroscopy and mass spectroscopy.

The (R,S)-5-benzyloxycarbonylamino-4-oxo-6-phenylhexanoic acid used as starting material was obtained as follows:—

A mixture of methyl (R,S)-5-acetamido-4-oxo-6-phenylhexanoate (9.5 g; the same intermediate as is described in Example 4 as an intermediate for Compounds Nos. 3 and 16) and aqueous 6N-hydrochloric acid (100 ml.) was heated at 100°C. for 6 hours, cooled and evaporated to dryness under reduced pressure. Toluene (100 ml.) was added and the mixture again evaporated to dryness under reduced pressure. There was thus obtained as oily residue (R,S)-5-amino-4-oxo-6-phenylhexanoic acid hydrochloride. A suspension of sodium bicarbonate (10 g.) in water (50 ml.) was cautiously added to a stirred solution of the above hydrochloride (9.25 g.) in water (100 ml.), and after evolution of carbon dioxide had ceased a solution of benzyl chloroformate (6.1 ml.) in acetone (20 ml.) was added and the mixture was stirred at 5°C. for 18 hours. The mixture was washed with diethyl ether (200 ml.) and then acidified to pH 2 with concentrated aqueous hydrochloric acid, and filtered. The solid residue was washed with water (200 ml.) and there was thus obtained (R,S)-5-benzyloxycarbonylamino-4-oxo-6-phenylhexanoic acid, m.p. 107—109°C.

## Example 10

Potassium t-butoxide (0.2 g.) was added to a solution of Isomer B of *N*-(5-benzyloxycarbonylamino-4-oxo-6-phenylhexanoyl)-L-proline t-butyl ester (25 g.) in diethyl ether (200 ml.) and the mixture was kept at laboratory temperature for 5 days and then filtered. The solid residue was crystallised from a mixture of ethyl acetate and petroleum ether (b.p. 40—60°C), and there was thus obtained Isomer A of *N*-(5-benzyloxycarbonylamino-4-oxo-6-phenylhexanoyl)-L-proline t-butyl ester, m.p. 126—128°C.

## Example 11

A mixture of Isomer A of *N*-(5-benzyloxycarbonylamino-4-oxo-6-phenylhexanoyl)-L-proline-t-butyl ester (1.0 g.), trifluoroacetic acid (18 ml.) and water (2 ml.) was kept at laboratory temperature for 40 minutes and then evaporated to dryness under reduced pressure. Toluene (30 ml.) was added and the mixture was again evaporated to dryness under reduced pressure, finally at 0.1 mm.Hg. There was thus obtained as oily residue Isomer A of *N*-(5-benzyloxycarbonylamino-4-oxo-6-phenylhexanoyl)-L-proline (Compound No. 143A), the structure of which was confirmed by elemental analysis, proton magnetic resonance spectroscopy and mass spectroscopy.

The process described above was repeated using the t-butyl ester Isomer B in place of Isomer A, and there was thus obtained Isomer B of *N*-(5-benzyloxycarbonylamino-4-oxo-6-phenylhexanoyl)-L-proline (Compound No. 143B).

## Example 12

A 30% palladium-on-charcoal catalyst (0.1 g.) was suspended in a solution of Isomer A of *N*-(5-benzyloxycarbonylamino-4-oxo-6-phenylhexanoyl)-L-proline (0.9 g.) in a mixture of ethanol (30 ml.) and aqueous N-hydrochloric acid (2.0 ml.), and hydrogen was bubbled through the mixture for 75 minutes. The mixture was filtered and the filtrate was evaporated to dryness under reduced pressure. Toluene (30 ml.) was added and the mixture was again evaporated to dryness under reduced pressure, finally at 0.1 mm/Hg. There was thus obtained, as a foam, Isomer A of *N*-(5-amino-4-oxo-6-phenylhexanoyl)-L-proline hydrochloride (Compound No. 144A), the structure of which was confirmed by elemental analysis, proton magnetic resonance spectroscopy and mass spectroscopy.

The process described above was repeated using the corresponding Isomer B as starting material in place of Isomer A, and there was thus obtained Isomer B of *N*-(5-amino-4-oxo-6-phenylhexanoyl)-L-proline hydrochloride (Compound No. 144B), also as a foam.

Example 13

Sodium bicarbonate (0.8 g.) was added to a stirred solution of Isomer A of *N*-(5-amino-4-oxo-6-phenylhexanoyl)-L-proline hydrochloride (0.71 g.) in water (30 ml.), and after evolution of carbon dioxide had ceased a solution of *p*-cyanobenzoyl chloride (0.4 g.) in acetone (5 ml.) was added and the mixture was stirred at laboratory temperature for 4 hours and then washed with diethyl ether (30 ml.). The aqueous solution was acidified to pH 3 with aqueous 2N-hydrochloric acid, and then extracted with ethyl acetate (60 ml.). The extract was dried over magnesium sulphate and evaporated to dryness. The solid residue was crystallised from a mixture of ethyl acetate and petroleum ether (b.p. 60—80°C.) and there was thus obtained Isomer A of *N*-(5-*p*-cyanobenzamido-4-oxo-6-phenylhexanoyl)-L-proline (Compound No. 145A), m.p. 163—165°C.

The process described above was repeated using the appropriate acid chloride and either Isomer A or Isomer B of *N*-(5-amino-4-oxo-6-phenylhexanoyl)-L-proline hydrochloride as starting materials, and where necessary purifying the product obtained by chromatography on a silica gel column using a 2.5% v/v solution of acetic acid in ethyl acetate as eluant. There were thus obtained the compounds described in the following table, all of which were non-crystalline and the structures of which were confirmed by elemental analysis, proton magnetic resonance spectroscopy and mass spectroscopy:—

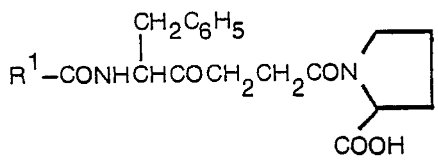

| $R^1$ | Isomer | Compound No. |
|---|---|---|
| phenyl | A | 27A |
| phenyl | B | 27B |
| 3,4-dichlorophenyl | A | 147A |
| 4-amino-3,5-dichlorophenyl | A | 148A |
| 2-trifluoromethylphenyl | A | 149A |
| 3,4,5-trimethoxyphenyl | A | 150A |
| 4-methylthiophenyl | B | 151B |
| 3,5-dinitrophenyl | B | 152B |
| 2-carboxyphenyl | B | 153B |
| 4-biphenylyl | B | 154B |
| cyclohexyl | A | 155A |
| cyclopropyl | B | 156B |
| phenyl-CH=CH— | B | 157B |
| phenyl-CH=CCl— | A | 158A |
| phenyl-CH=CCl— | B | 158B |
| $(phenyl)_2CH$— | B | 159B |
| 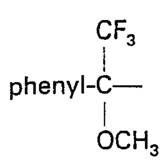 | A<br>B | 160A<br>160B } Note 1 |

*Note 1.* These two compounds were prepared from (+)-2-methoxy-2-phenyl-3,3,3-trifluoropropionic acid (Dale *et al.*, J.Organic Chemistry, 1969, *34*, 2543), were purified by chromatography on a silica gel column using a 2% v/v solution of acetic acid in ethyl acetate as eluant, and served to distinguish Isomers A and B.

The elemental analysis, $^{13}C$ and $^{19}F$ nuclear magnetic resonance spectra and mass spectra of the two isomers were identical, but Isomer A had a larger retention time than Isomer B on high pressure liquid chromatography, and the proton magnetic resonance spectra were different as shown below:

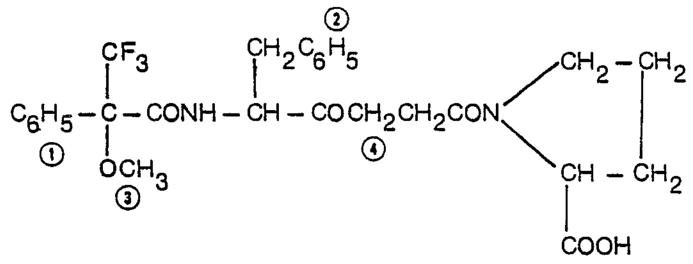

| δ-values (p.p.m.) | (No. of H atoms and type of peak) | Specific H Atoms |
|---|---|---|
| Isomer A | Isomer B | |
| 7.40 (5H, singlet) | 7.35—7.20 (5H, multiplet) | } ① + ② |
| 7.20 (5H, singlet) | 7.15 (5H, singlet) | |
| 3.19 (3H, multiplet) | 3.28 (3H, multiplet) | ③ |
| 2.63 (2H, multiplet) | 2.74 (2H, multiplet) | ④ |

The proton magnetic resonance spectra showed that Isomer A was uncontaminated by Isomer B, but that Isomer B was sometimes contaminated by a small amount of Isomer A.

The process described above was repeated using the appropriate acid chloride (or phenyl isocyanate*), and Isomer A of *N*-(5-amino-4-oxo-6-phenylhexanoyl)-L-proline t-butyl ester (prepared from Compound 43A of Example 9 by hydrogenolysis of the benzyloxycarbonyl group by a similar process to that described in Example 12) as starting materials. There were thus obtained the compounds described in the following table, all of which were non-crystalline and the structures of which were confirmed by elemental analysis, proton magnetic resonance spectroscopy and mass spectroscopy:

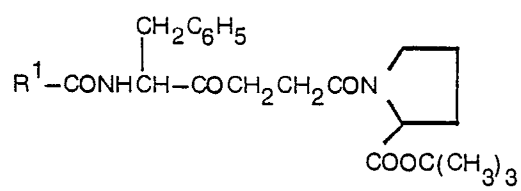

| $R^1$ | Compound No. |
|---|---|
| phenyl-$CH_2$—CH ($NHCOOCH_2C_6H_5$)— | 61A |
| $CH_3CH(NHCOOCH_2C_6H_5)$— | 62A |
| phenyl-NH—* | 63A |

Example 14

The process described in the first part of Example 9 was repeated except that (R,S)-5-benzyloxycarbonylamino-(R,S)-2-methyl-4-oxo-6-phenylhexanoic acid was used as starting material in place of the 2-unsubstituted compound. There was thus obtained *N*-[(R,S)-5-benzyloxycarbonylamino-(R,S)-2-methyl-4-oxo-6-phenylhexanoyl]-L-proline t-butyl ester monohydrate (Compound No. 65), the structure of which was confirmed by elemental analysis and infra-red spectroscopy.

The hexanoic acid used as starting material was obtained as an oil from methyl (R,S)-5-acetamido-(R,S)-2-methyl-4-oxo-6-phenylhexanoate (Example 1) and benzyl chloroformate by a similar procedure to that described in the last part of Example 9.

Example 15

The process described in Example 5 was repeated except that *N*-[(R,S)-5-benzyloxycarbonylamino-(R,S)-2-methyl-4-oxo-6-phenylhexanoyl]-L-proline t-butyl ester (Compound No. 65, Example 14) was used as starting material, and that the product was purified by chromatography on silica gel using a 9:9:1:1 v/v/v/v mixture of ethyl acetate: toluene: formic acid: methanol as eluant. There was thus obtained *N*-[(R,S)-5-benzyloxycarbonylamino-(R,S)-2-methyl-4-oxo-6-phenylhexanoyl]-L-proline (Compound No. 165) as an oil, the structure of which was confirmed by elemental analysis and infrared spectroscopy.

Example 16

The process described in Example 12 was repeated except that *N*-[(R,S)-5-benzyloxycarbonylamino-(R,S)-2-methyl-4-oxo-6-phenylhexanoyl]-L-proline (Compound No. 165, Example 15) was used as starting material. There was thus obtained *N*-[(R,S)-5-amino-(R,S)-2-methyl-4-oxo-6-phenylhexanoyl]-L-proline hydrochloride sesquihydrate (Compound No. 166) as a pale yellow solid, the structure of which was confirmed by elemental analysis.

Example 17

Thionyl chloride (15 ml.) was added to a stirred solution of *N*-[(R,S)-5-amino-(R,S)-2-methyl-4-oxo-6-phenylhexanoyl]-L-proline hydrochloride (5 g.) in methanol (50 ml.) which was maintained at —20°C., and the mixture was then stirred for 24 hours, evaporated to dryness and toluene was added and reevaporated until all thionyl chloride had been removed. There was thus obtained as oily residue *N*-[(R,S)-5-amino-(R,S)-2-methyl-4-oxo-6-phenylhexanoyl]-L-proline methyl ester (Compound No. 67), the structure of which was confirmed by elemental analysis.

Example 18

The process described in Example 13 was repeated except that *N*-[(R,S)-5-amino-(R,S)-2-methyl-4-oxo-6-phenylhexanoyl]-L-proline hydrochloride (Example 16) or the methyl ester thereof (Example 17) and the appropriate acyl chloride were used as starting materials. There were thus obtained, as oils, the compounds described in the following table, the structures of which were all confirmed by elemental analysis and spectroscopic procedures:—

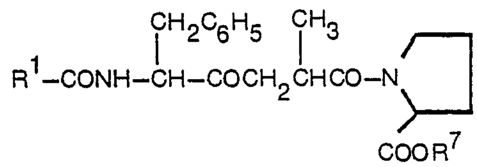

| $R^1$ | $R^7$ | Compound No. |
|---|---|---|
| 2-tolyl | H | 168 |
| 3-tolyl | H | 169 |
| 4-tolyl | H | 170 |
| 3,5-dimethylphenyl | H | 171 |
| 2-methoxyphenyl | H | 172 |
| 2-methoxyphenyl | methyl | 72 |
| 3-methoxyphenyl | H | 173 |
| 3-methoxyphenyl | methyl | 73 |
| 4-fluorophenyl | H | 174 |
| 2,6-difluorophenyl | H | 175 |
| 3-iodophenyl | H | 176 |
| 3-iodophenyl | methyl | 76 (Note 1) |
| 4-acetylphenyl | H | 177 |
| 4-methanesulphonylphenyl | H | 178 |
| phenoxymethyl | H | 179 |
| phenylthiomethyl | H | 180 |
| β-phenylethyl | H | 181 |
| phenoxy | H | 182 |
| p-hydroxyphenyl | H | 183 (Note 2) |

*Note 1.* The methyl ester was prepared by the reaction of the corresponding acid with thionyl chloride and methanol by a similar procedure to that described in Example 17.

*Note 2.* *p*-Acetoxybenzoylchloride was used as starting material, the acetyl group being removed by shaking the final ethyl acetate solution with dilute aqueous sodium hydroxide solution and then acidifying the aqueous layer and extracting with ethyl acetate.

Example 19

*N*-Methylmorpholine (1.1 g.) and isobutyl chloroformate (0.67 g.) were added to a stirred solution of *o*-ethoxycarbonylmethylbenzoic acid (1 g.) in tetrahydrofuran (40 ml.) which was cooled to −20°C., and the mixture was stirred at that temperature for 30 minutes. A solution of *N*-[(R,S)-5-amino-(R,S)-2-methyl-4-oxo-6-phenylhexanoyl]-L-proline methyl ester (0.9 g.) in tetrahydrofuran (6 ml.) was added and the mixture was stirred for 5 hours and then evaporated to dryness. The residue was dissolved in ethyl acetate and the solution was washed successively with aqueous 2N-hydrochloric acid, saturated aqueous sodium bicarbonate solution and water, dried over magnesium sulphate and evaporated to dryness under reduced pressure. The residue was purified by chromatography on a silica gel column using a 49.5:49.5:1 v/v/v mixture of ethyl acetate, petroleum ether (b.p. 60—80°C) and formic acid as

eluant. There was thus obtained *N*-[(R,S)-5-*o*-ethoxycarbonylmethylbenzamido-(R,S)-2-methyl-4-oxo-6-phenylhexanoyl]-L-proline methyl ester (Compound No. 84), the structure of which was confirmed by elemental analysis and spectroscopic procedures.

The process described above was repeated using the appropriate acid in place of *o*-ethoxycarbonylmethylbenzoic acid, and there were thus obtained the compounds described in the following table:—

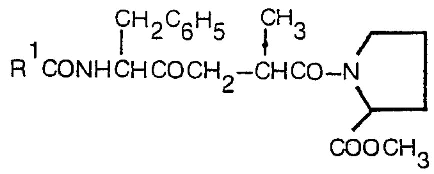

| $R^1$ | Compound No. |
|---|---|
| *p*-acetamidophenyl | 85 |
| 2-(benzyloxycarbonylamino)ethyl* | 86 |
| 2-aminoethyl* | 87 |
| ethoxy | 88 |

* Derived from L-alanine.

The process described above was repeated except that *p*-acetamidobenzoic acid and *N*-[(R,S)-5-amino-(R,S)-2-methyl-4-oxo-6-phenylhexanoyl]-L-proline isopropyl ester (prepared by a similar process to that described in Example 17 except that isopropanol was used in place of methanol) were used as starting materials. There was thus obtained *N*-[(R,S)-5-*p*-acetamidobenzamido-(R,S)-2-methyl-4-oxo-6-phenylhexanoyl]-L-proline isopropyl ester (Compound No. 280).

The process described above was repeated except that *p*-acetamidobenzoic acid and *N*-[(R,S)-5-amino-(R,S)-2-methyl-4-oxo-6-phenylhexanoyl]-L-proline (Compound No. 166; Example 16) were used as starting materials.

There was thus obtained *N*-[(R,S)-5-amino-(R,S)-2-methyl-4-oxo-6-phenylhexanoyl]-L-proline. (Compound No. 185).

Example 20

A solution of *N,N*-dicyclohexylcarbodi-imide in methylene chloride (2 ml.) was added to a stirred solution of (R,S)-5-benzamido-6-*p*-cyanophenyl-(R,S)-2-methyl-4-oxohexanoic acid (0.75 g.) and 1-hydroxybenzotriazole (0.3 g.) in methylene chloride (7.5 ml.) which was cooled to 0°C. under an atmosphere of argon, and the mixture was stirred at laboratory temperature for 1 hour and then recooled to 0°C. A solution of L-proline isopropyl ester (0.35 g.) in methylene chloride (2 ml.) was added and the mixture was stirred at laboratory temperature for 16 hours and filtered, and the filtrate was evaporated to dryness under reduced pressure. The residue was purified by chromatography on a silica gel column using a 1:1 v/v mixture of ethyl acetate and toluene as eluant, and there was thus obtained as an oil *N*-[(R,S)-5-benzamido-6-*p*-cyanophenyl-(R,S)-2-methyl-4-oxohexanoyl]-L-proline isopropyl ester (Compound No. 89), the structure of which was confirmed by elemental analysis and spectroscopic procedures.

The (R,S)-5-benzamido-6-*p*-cyanophenyl-(R,S)-2-methyl-4-oxohexanoic acid used as starting material was obtained as follows:—

A solution of 3-methoxycarbonylbutyryl chloride (prepared as described in Example 1 from 32.1 g. of 3-methoxycarbonylbutyric acid) in tetrahydrofuran (50 ml.) was added during 5 minutes to a stirred solution of 2-phenyloxazol-5 (4H)-one (32 g.) and 4-dimethylaminopyridine (0.5 g.) in tetrahydrofuran (1000 ml.) which was cooled to —30°C. under an atmosphere of argon, and the mixture was stirred at —30°C. for 30 minutes and then poured into vigorously stirred ice-water (200 ml.). The mixture was acidified to pH 3 with aqueous N-hydrochloric acid and filtered, and the solid product was washed with water, dried over phosphorus pentoxide and crystallised from acetone. There was thus obtained 4-(3-methoxycarbonylbutyryl)-2-phenyloxazol-5(4H)-one, m.p. 151—154°C.

Triethylamine (1 ml.) and *p*-cyanobenzyl bromide (1.35 g.) were added to a stirred solution of the above oxazolone (2 g.) in dimethylformamide (10 ml.) and the mixture was stirred at laboratory temperature for 16 hours and then poured into aqueous 5% hydrochloric acid (100 ml.). The mixture was extracted 5 times with diethyl ether (25 ml. each time) and the combined extracts were washed twice with saturated aqueous sodium bicarbonate solution (25 ml. each time) and twice with saturated

aqueous sodium chloride solution (25 ml. each time), dried over magnesium sulphate and evaporated to dryness. The residue was dissolved in acetic acid (15 ml.) containing 4-dimethylaminopiperidine (0.05 g.) and the mixture was heated under reflux for 4 hours and then evaporated to dryness under reduced pressure. The residue was purified by chromatography on a silica gel column using a 4:1 v/v mixture of toluene and ethyl acetate as eluant. There was thus obtained methyl (R,S)-5-benzamido-6-*p*-cyanophenyl-(R,S)-2-methyl-4-oxohexanoate as an oil.

A mixture of the above ester (1.85 g.), methanol (10 ml.) and aqueous 2N-sodium hydroxide solution (8 ml.) was stirred at laboratory temperature for 2 hours, acidified with aqueous 2N-hydrochloric acid and extracted 3 times with ethyl acetate (25 ml. each time). The combined extracts were washed with saturated aqueous sodium chloride solution, dried over magnesium sulphate and evaporated to dryness. There was thus obtained as residue (R,S)-5-benzamido-6-*p*-cyanophenyl-(R,S)-2-methyl-4-oxohexanoic acid.

The L-proline isopropyl ester used as starting material was obtained as follows:—

Thionyl chloride (30 ml.) was added to isopropanol (130 ml.) which was cooled to —50°C., the mixture was allowed to warm up to —10°C and L-proline (23 g.) was added. The mixture was stirred at laboratory temperature for 16 hours, heated under reflux for 30 minutes and the excess of isopropanol and thionyl chloride were removed by evaporation under reduced pressure followed by azeotropic distillation of toluene. The hydrochloride salt thus obtained (1.3 g.) was added to aqueous 75% w/v sodium carbonate solution (10 ml.) at 0°C. and the mixture was extracted 3 times with diethyl ether. The combined extracts were dried over magnesium sulphate and evaporated to dryness and there was thus obtained as residue L-proline isopropyl ester.

## Example 21

The process described in Example 20 was repeated using the appropriate (R,S)-5-benzamido- 6-substituted-phenyl-(R,S)-2-methyl-4-oxohexanoic acid (prepared by similar means to those described in Example 20) and the appropriate L-proline ester as starting materials, and there were thus obtained the compounds described in the following table, all of which were oils, the structures of which were confirmed by elemental analysis and spectroscopic procedures.

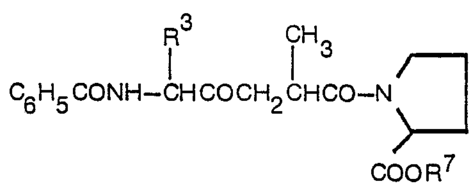

| $R^3$ | $R^7$ | Compound No. |
|---|---|---|
| 4-methylbenzyl | isopropyl | 94 |
| 4-methoxybenzyl | isopropyl | 90 |
| 4-cyanobenzyl | t-butyl | 91 |
| 4-methylbenzyl | t-butyl | 92 |
| 4-methoxybenzyl | t-butyl | 93 |
| 2-chlorobenzyl | isopropyl | 275 |
| 2-chlorobenzyl | t-butyl | 276 |
| 3-chlorobenzyl | isopropyl | 277 |

## Example 22

The process described in Example 20 was repeated except that (R,S)-5-benzamido-6-*p*-cyanophenyl-4-oxohexanoic acid and L-proline t-butyl ester were used as starting materials. There was thus obtained, as an oil, *N*-[(R,S)-5-benzamido-6-*p*-cyanophenyl-4-oxohexanoyl]-L-proline t-butyl ester (Compound No. 95) the structure of which was confirmed by elemental analysis and spectroscopic procedures.

The (R,S)-5-benzamido-6-*p*-cyanophenyl-4-oxohexanoic acid (m.p. 182—184°C.) used as starting material was obtained by a similar process to that described in the second part of Example 20 except that 3-methoxycarbonylpropionyl chloride was used as initial starting material.

Example 23

The process described in Example 20 was repeated using the appropriate (R,S)-5-benzamido-6-substituted-phenyl-4-oxohexanoic acid (prepared by a similar process to that described in the second part of Example 22) and the appropriate L-proline ester, as starting materials, and there were thus obtained the compounds described in the following table:—

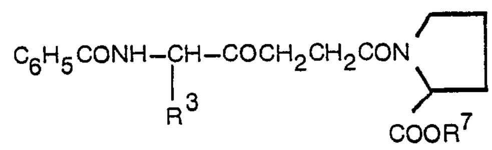

| $R^3$ | $R^7$ | Compound No. | |
|---|---|---|---|
| *p*-methylbenzyl | t-butyl | 207 | |
| *p*-methylbenzyl | methyl | 96 | |
| *m*-methylbenzyl | t-butyl | 97 | |
| *m*-methylbenzyl | methyl | 98 | |
| *o*-methylbenzyl | t-butyl | 99 | |
| *p*-chlorobenzyl | t-butyl | 100 | |
| *p*-fluorobenzyl | t-butyl | 201 | |
| *p*-fluorobenzyl | methyl | 202 | |
| *o*-bromobenzyl | t-butyl | 203 | |
| 3,4-dichlorobenzyl | t-butyl | 204 | |
| *p*-methoxybenzyl | t-butyl | 205 | |
| *p*-nitrobenzyl | t-butyl | 206 | |
| benzyl | benzyl | 281 | |
| *m*-cyanobenzyl | t-butyl | 208 | |
| *p*-trifluoromethylbenzyl | t-butyl | 209 | |
| 2-phenylethyl | t-butyl | 210 | Note 1 |
| 3-phenylpropyl | t-butyl | 211 | Note 2 |
| 3-phenylprop-2-enyl | t-butyl | 212 | |
| naphth-1-ylmethyl | t-butyl | 213 | |
| naphth-2-ylmethyl | t-butyl | 214 | |
| 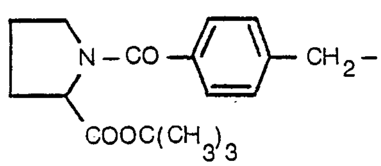 | t-butyl | 215 | Note 3 |

*Note 1.* The starting material was prepared from 3-methoxycarbonylpropionyl chloride and 4-(2-phenylethyl)-2-phenyloxazol-5(4H)-one by a similar method to that described in the second part of Example 1, but including the step of heating with 4-dimethylaminopyridine in acetic acid as described in the third part of Example 20. The 4-(β-phenylethyl)-2-phenyloxazol-5(4H)-one (m.p. 119—122°C.) was

itself obtained by the reaction of β-phenylethyl bromide with diethyl acetamidomalonate in the presence of sodium ethoxide, then hydrolysis of the acetamido group with concentrated aqueous hydrochloric acid to give 2-amino-4-phenylbutyric acid hydrochloride; acylation of the amino group with benzoyl chloride in aqueous acetone to give 2-benzamido-4-phenylbutyric acid (m.p. 139—141°C.); and finally treatment of this acid with *N*-(3-dimethylaminopropyl)-*N'*-ethylcarbodiimide hydrochloride in methylene chloride solution at 5°C.

*Note 2.* The starting material was prepared as described under Note 1 from 4-(3-phenylpropyl)-2-phenyloxazol-5(4H)-one, which itself was obtained by hydrogenation of 4-(3-phenyl prop-1-ylidene)-2-phenyloxazol-5(4H)-one (prepared by a similar process to that described in Chemistry and Industry, 1954, 191) with a 10% palladium-on-charcoal catalyst in dioxane solution. The intermediate methyl (R,S)-5-benzamido-4-oxo-8-phenyloctanoate had m.p. 110—112°C.

*Note 3.* The starting material was (R,S)-5-benzamido-6-p-carboxyphenyl-4-oxohexanoic acid, and a second molecule of L-proline t-butyl ester formed an amide with the *p*-carboxy group.

The process described above was repeated except that (R,S)-5-benzamido-6-*p*-methoxyphenyl-4-oxohexanoic acid and L-proline amide were used as starting materials. There were thus obtained two isomers of 1-(5-benzamido-6-*p*-methoxyphenyl-4-oxohexanoyl)pyrrolidine-(S)-2-carboxamide, a less polar isomer (Compound No. 282A) and a more polar isomer (Compound No. 282B), the isomers being separated by the chromatographic purification step.

Example 24

The process described in Example 20 was repeated except that (R,S)-5-*N*-methylbenzamido-4-oxo-6-phenylhexanoic acid and L-proline t-butyl ester were used as starting materials. There was thus obtained *N*-[(R,S)-5-*N*-methylbenzamido-4-oxo-6-phenylhexanoyl]-L-proline t-butyl ester (Compound No. 216), the structure of which was confirmed by elemental analysis and spectroscopic procedures.

The hexanoic acid used as starting material was obtained as follows:—

Methyl trifluoromethanesulphonate (0.8 ml.) was added to a solution of 4-benzyl-2-phenyl-4-(3-methoxycarbonylpropionyl)-oxazol-5(4H)-one (1.46 g., m.p. 93—94°C.; prepared from 3-methoxycarbonylpropionyl chloride, 2-phenyloxazol-5(4H)-one and benzyl bromide by the general procedure described in the second and third parts of Example 20) in methylene chloride (10 ml.) and the mixture was heated under reflux under an atmosphere of argon for 10 hours and then evaporated to dryness under reduced pressure. The residue was dissolved in a mixture of methylene chloride (21 ml.) and acetic acid (7 ml.), 4-dimethylaminopyridine (0.2 g.) was added and the mixture was heated under reflux for 30 minutes and then evaporated to dryness. The residue was dissolved in methylene chloride and the solution was washed successively with saturated aqueous sodium bicarbonate solution, 10% w/v aqueous hydrochloric acid, water and saturated aqueous sodium chloride solution, dried over sodium sulphate and evaporated to dryness. There was thus obtained (R,S)-5-*N*-methylbenzamido-4-oxo-6-phenylhexanoic acid as an oil which was used without further purification.

Example 25

A solution of *N*-[(R,S)-5-benzamido-6-*p*-nitrophenyl-4-oxohexanoyl]-L-proline t-butyl ester (Compound No. 206; Example 23; 9.1 g.) in ethanol (350 ml.) was shaken with hydrogen at laboratory temperature and atmospheric pressure for 5 hours in the presence of a 10% palladium-on-charcoal catalyst (0.5 g.). The mixture was filtered, the filtrate was evaporated to dryness and the residue was stirred with diethyl ether. The mixture was filtered and there was thus obtained as solid residue Isomer B of *N*-6-*p*-aminophenyl-5-benzamido-4-oxohexanoyl)-L-proline t-butyl ester (Compound No. 217B), $[\alpha]_D^{25}$ −3.9° (C=1 in methanol).

The ethereal filtrate was evaporated to dryness under high vacuum, and there was thus obtained as an oil Isomer A of N-[6-*p*-aminophenyl-5-benzamido-4-oxohexanoyl)-L-proline t-butyl ester (Compound No. 217A) $[\alpha]_D^{25}$ −82.6° (C=1 in methanol).

Example 26

Acetyl chloride (0.0785 ml.) was added dropwise to a stirred solution of Isomer A of *N*-(6-*p*-aminophenyl-5-benzamido-4-oxohexanoyl)-L-proline t-butyl ester (Compound No. 217A; Example 25; 0.493 g.) and triethylamine (0.1384 ml.) in tetrahydrofuran (7 ml.) which was cooled to 0°C. under an atmosphere of argon, and the mixture was stirred for 1 hour and then poured onto a silica gel column (10 g.). The column was eluted with a 19:1 v/v mixture of methylene chloride and methanol and the appropriate fractions collected and evaporated to dryness. There was thus obtained Isomer A of *N*-(6-*p*-acetamidophenyl-5-benzamido-4-oxohexanoyl)-L-proline t-butyl ester (Compound No. 218A), the structure of which was confirmed by elemental analysis and spectroscopic procedures.

The process described above was repeated using methanesulphonyl chloride in place of acetyl chloride. There was thus obtained *N*-(5-benzamido-6-*p*-methanesulphonamido-4-oxohexanoyl)-*L*-

proline t-butyl ester (Compound No. 219A), the structure of which was confirmed by elemental analysis and spectroscopic procedures.

By using trifluoromethanesulphonylchloride there was similarly obtained *N*-(5-benzamido-6-*p*-trifluoromethanesulphonamido-4-oxohexanoyl)-L-proline t-butyl ester (Compound No. 220A).

Example 27

The process described in Example 1 was repeated except that (R,S)-5-*p*-cyanobenzamido-(R,S)-2-methyl-4-oxo-6-*p*-tolylhexanoic acid and L-proline t-butyl ester were used as starting materials. There was thus obtained as an oil *N*-[(R,S)-5-*p*-cyanohenzamido-(R,S)-2-methyl-4-oxo-6-*p*-tolylhexanoyl]-L-proline t-butyl ester (Compound No. 221), the structure of which was confirmed by elemental analysis and spectroscopic procedures.

The (R,S)-5-*p*-cyanobenzamido-(R,S)-2-methyl-4-oxo-6-*p*-tolylhexanoic acid used as starting material was obtained as follows:—

Diethyl acetamidomalonate (108.5 g.) was slowly added to a stirred solution of sodium (12 g.) in ethanol (400 ml.) and the mixture was stirred at laboratory temperature for 30 minutes. Sodium iodide (5 g.) and *p*-methylbenzyl bromide (96 g.) were added and the mixture was stirred and heated under reflux for 16 hours, cooled and evaporated to dryness under reduced pressure. The residue was partitioned between water (250 ml.) and ethyl acetate (500 ml.) and the organic layer was washed with saturated aqueous sodium chloride solution, dried over magnesium sulphate and evaporated to dryness. The residue was stirred with a 4:1 v/v mixture of petroleum ether (b.p. 40—60°C.) and diethyl ether and the mixture was filtered. There was thus obtained ethyl 2-acetamido-2-ethoxycarbonyl-3-*p*-tolylpropionate, m.p. 111—113°C.

A stirred suspension of the above diester (127 g.) in 10% aqueous sodium hydroxide solution (600 ml.) was heated under reflux for 4 hours, cooled, acidified with aqueous 3N-hydrochloric acid, reheated under reflux for 1 hour, cooled to 0°C., kept at that temperature for 72 hours and then filtered. There was thus obtained as solid residue (±)-2-acetamido-3-*p*-tolylpropionic acid.

A mixture of the above acid (35 g.) and acetic anhydride (300 ml.) was stirred at 100°C for 40 minutes, cooled and the excess of acetic anhydride was removed by evaporation under reduced pressure. The residue was distilled and there was thus obtained 2-methyl-4-*p*-tolyloxazol-5(4H)-one, b.p. 116—120°C/0.2 mm.Hg.

This oxazolone was reacted with 3-methoxycarbonylbutyryl chloride by a similar process to that described in the second part of Example 1 to give methyl (R,S)-5-acetamido-(R,S)-2-methyl-4-oxo-6-*p*-tolylhexanoate, and this was hydrolysed with aqueous 6N hydrochloric acid and acylated with *p*-cyanobenzoyl chloride by a similar procedure to that described in the last part of Example 9. There was thus obtained (R,S)-5-*p*-cyanobenzamido-(R,S)-2-methyl-4-oxo-6-*p*-tolylhexanoic acid which was used without further purification.

Example 28

The process described in Example 27 was repeated except the corresponding 5-*p*-acetamido-benzamido-hexanoic acid was used as starting material in place of the 5-*p*-cyanobenzamido-hexanoic acid. There was thus obtained as an oil *N*-[(R,S)-5-*p*-acetamidobenzamido-(R,S)-methyl-4-oxo-6-*p*-tolylhexanoyl]-L-proline t-butyl ester (Compound No. 222), the structure of which was confirmed by elemental analysis and spectroscopic procedures.

The starting material was obtained by a similar procedure to that described in Example 27 except that the final acylation stage was carried out as follows:—

*N*-Methylmorpholine (0.77 ml.) and isobutyl chloroformate (0.91 ml.) were added to a stirred solution of *p*-acetamidobenzoic acid (1.25 g.) in tetrahydrofuran (40 ml.) which was cooled to −20°C. under an atmosphere of argon, and the stirred mixture was allowed to warm up to laboratory temperature during 1 hour and was then cooled to 0°C. (R,S)-5-Amino-(R,S)-2-methyl-4-oxo-6-*p*-tolyl-hexanoic acid (2 g.) and then triethylamine (1.95 ml.) were added and the mixture was stirred at laboratory temperature for 16 hours, and then filtered, and the filtrate was evaporated to dryness under reduced pressure. The residue was partitioned between ethyl acetate (50 ml.) and aqueous 0.1 N-hydrochloric acid (25 ml.) and the organic phase was dried over magnesium sulphate and evaporated to dryness. The residue was purified by chromatography on a silica gel column using a 10:10:1 v/v/v mixture of toluene, ethyl acetate and acetic acid as eluant, and there was thus obtained (R,S)-5-*p*-acetamidobenzamido-(R,S)-2-methyl-4-oxo-6-*p*-tolylhexanoic acid.

Example 29

Oxalyl chloride (1.0 ml.) and dimethylformamide (0.25 ml.) were added to a stirred solution of *N*-[3-carboxy-(R,S)-2-methylpropionyl]-L-proline t-butyl ester (2.86 g.) in dry methylene chloride (50 ml.) which was cooled to −70°C. under an atmosphere of argon, and the mixture was stirred at −70°C. for 2 hours, then at 0—5°C. for 2 hours and finally at laboratory temperature for 1 hour, and then evaporated to dryness under reduced pressure. Toluene was added and the mixture was evaporated to dryness under reduced pressure. A solution of the acid chloride thus obtained in dry tetrahydrofuran (2 ml.) was added to a stirred solution of 2-phenyloxazol-5(4H)-one (1.47 g.), 4-dimethylaminopyridine

(0.08 g.) and triethylamine (2.54 ml.) in dry tetrahydrofuran (20 ml.) which was cooled to −40°C. under an atmosphere of argon, at such a rate that the temperature of the mixture did not exceed −35°C. The mixture was stirred at −35°C. for 1 hour, then at laboratory temperature for 16 hours, benzyl bromide (1.19 ml.) was added and the mixture was stirred at laboratory temperature for 48 hours. Saturated aqueous sodium carbonate solution (4 ml.) was added and the mixture was stirred at laboratory temperature for 2 hours and then neutralised to pH 7 with aqueous 2N-hydrochloric acid. The organic solvents were removed by evaporation under reduced pressure and the aqueous residue was extracted 4 times with ethyl acetate (50 ml. each time). The combined extracts were washed successively twice with saturated aqueous oxalic acid solution (50 ml. each time), twice with saturated aqueous sodium bicarbonate solution (50 ml. each time) and once with saturated aqueous sodium chloride solution, dried over magnesium sulphate and evaporated to dryness under reduced pressure. The residue was purified by chromatography on a silica gel column using a 3:1 v/v mixture of methylene chloride and ethyl acetate as eluant. There was thus obtained, as a foam, *N*-[(R,S)-5-benzamido-(R,S)-2-methyl-4-oxo-6-phenylhexanoyl]-L-proline t-butylester (Compound No. 6), identical with that hereinbefore described in Example 2.

The *N*-[3-carboxy-(R,S)-2-methylpropionyl]-L-proline t-butyl ester used as starting material was obtained as follows:—

A solution of dicyclohexylcarbodi-imide (4.8 g.) in methylene chloride (100 ml.) was added during 30 minutes to a stirred suspension of *N*-hydroxybenzotriazole (40.5 g.) and 3-methoxycarbonyl-(R,S)-2-methylpropionic acid (43.8 g.) in methylene chloride (1000 ml.) which was cooled to 0°C. under an atmosphere of argon, and the mixture was then stirred at laboratory temperature for 1 hour and recooled to 0°C. A solution of L-proline t-butyl ester (51.3 g.) in methylene chloride (100 ml.) was added and the mixture was stirred at laboratory temperature for 16 hours and then filtered. The filtrate was evaporated to dryness, the residue was dissolved in diethyl ether (500 ml.) and the mixture was filtered. The filtrate was washed successively five times with aqueous 2N-hydrochloric acid (50 ml. each time) four times with saturated aqueous sodium carbonate solution (100 ml. each time) and twice with saturated aqueous sodium chloride solution, dried over magnesium sulphate and evaporated to dryness under reduced pressure. Aqueous 2N-sodium hydroxide solution (113 ml.) was added to a solution of the methyl ester thus obtained (61.8 g.) in methanol (325 ml.) and the mixture was stirred at laboratory temperature for 3.5 hours, neutralised with aqueous N-hydrochloric acid and the methanol was removed by evaporation under reduced pressure. Saturated aqueous sodium carbonate solution was added to the aqueous residue and the mixture was washed 3 times with diethyl ether (50 ml. each time). The aqueous solution was then acidified to pH 2 with aqueous N-hydrochloric acid and extracted 6 times with ethyl acetate (50 ml. each time). The combined extracts were washed twice with saturated aqueous sodium chloride solution (50 ml. each time), dried over magnesium sulphate and evaporated to dryness under reduced pressure. There was thus obtained *N*-[3-carboxy-(R,S)-2-methylpropionyl]-L-proline t-butyl ester as an oil which was used without further purification.

## Example 30

The process described in Example 29 was repeated except that the appropriate bromide was used in place of benzyl bromide. There were thus obtained as foams, the compounds described in the following table, the structures of which were all confirmed by elemental analysis and proton magnetic resonance spectroscopy:—

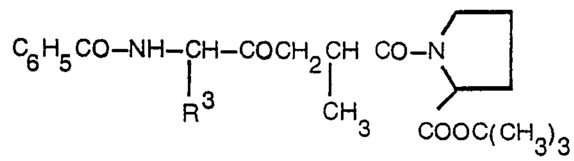

| $R^3$ | Compound No. |
|---|---|
| *p*-methylbenzyl | 92 |
| *m*-methylbenzyl | 223 |
| *p*-chlorobenzyl | 224 |
| *m*-chlorobenzyl | 225 |
| *p*-methoxybenzyl | 93 |
| *p*-nitrobenzyl | 226 |
| *p*-trifluoromethylbenzyl | 227 |
| *p*-cyanobenzyl | 91 |
| 4-biphenylmethyl | 228 |
| *p*-t-butylbenzyl | 229 |
| 3-phenylprop-2-enyl | 230 |
| prop-2-enyl | 231 |
| 2-methylprop-2-enyl | 232 |

Example 31

A suspension of a 10% palladium-on-charcoal catalyst (0.03 g.) in a solution of *N*-[(R,S)-5-benzamido-(R,S)-2-methyl-4-oxo-8-phenylocta-7-enoyl]-L-proline t-butyl ester (Compound No. 230; 0.94 g.) in ethyl acetate (10 ml.) was stirred in an atmosphere of hydrogen for 1 hour and then filtered. The filtrate was evaporated to dryness under reduced pressure and there was thus obtained *N*-[(R,S)-5-benzamido-(R,S)-2-methyl-4-oxo-8-phenyloctanoyl]-L-proline t-butyl ester (Compound No. 233).

The process described above was repeated using *N*-[(R,S)-5-benzamido-(R,S)-2-methyl-4-oxo-octa-7-enoyl]-L-proline t-butyl ester (Compound No. 231) or *N*-[(R,S)-5-benzamido-(R,S)-2,7-dimethyl-4-oxo-octa-7-enoyl]-L-proline t-butyl ester (Compound No. 232) as starting material, and there was thus obtained, respectively, *N*-[(R,S)-5-benzamido-(R,S)-2-methyl-4-oxo-octanoyl]-L-proline t-butyl ester (Compound No. 234) or *N*-[(R,S)-5-benzamido-(R,S)-2,7-dimethyl-4-oxo-octanoyl]-L-proline t-butyl ester (Compound No. 235).

All three compounds described above are non-crystalline foams the structures of which were confirmed by elemental analysis and spectroscopic procedures.

Example 32

The process described in Example 5 was repeated using the t-butyl esters described in Examples 8, 21, 22, 23, 24, 25, 26, 27, 28, 30 and 31 as starting materials, and there were thus obtained the acids described in the following table, all of which were oils the structures of which were confirmed by elemental analysis and spectroscopic procedures:—

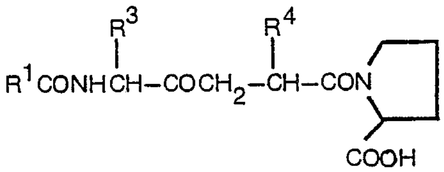

| Starting Material Compound No. | $R^1$ | $R^3$ | $R^4$ | Acid Compound No. |
|---|---|---|---|---|
| 17 | 4-acetamidophenyl | benzyl | H | 37 |
| 18 | 4-methoxyphenyl | benzyl | H | 38 |
| 19 | 2-naphthyl | benzyl | H | 39 |
| 20 | β-phenylethyl | benzyl | H | 40 |
| 41 | β-benzoylethyl | benzyl | H | 141 |
| 43 | benzyloxy | benzyl | H | 143 |
| 91 | phenyl | *p*-cyanobenzyl | methyl | 191 |
| 92 | phenyl | *p*-methylbenzyl | methyl | 192 |
| 93 | phenyl | *p*-methoxybenzyl | methyl | 193 |
| 95 | phenyl | *p*-cyanobenzyl | H | 195 |
| 97 | phenyl | *m*-methylbenzyl | H | 197 |
| 99 | phenyl | *o*-methylbenzyl | H | 199 |
| 100 | phenyl | *p*-chlorobenzyl | H | 200 |
| 201 | phenyl | *p*-fluorobenzyl | H | 301 |
| 203 | phenyl | *o*-bromobenzyl | H | 303 |
| 204 | phenyl | 3,4-dichlorobenzyl | H | 304 |
| 205 | phenyl | *p*-methoxybenzyl | H | 305 |
| 206 | phenyl | *p*-nitrobenzyl | H | 306 |
| 207 | phenyl | *p*-methylbenzyl | H | 307 |
| 208 | phenyl | *m*-cyanobenzyl | H | 308 |
| 209 | phenyl | *p*-trifluoromethyl-benzyl | H | 309 |
| 210 | phenyl | β-phenylethyl | H | 310 |
| 211 | phenyl | 3-phenylpropyl | H | 311 |
| 212 | phenyl | 3-phenylprop-2-enyl | H | 312 |
| 213 | phenyl | naphth-1-ylmethyl | H | 313 |

| Starting Material Compound No. | $R^1$ | $R^3$ | $R^4$ | Acid Compound No. |
|---|---|---|---|---|
| 214 | phenyl | naphth-2-ylmethyl | H | 314 |
| 215 | phenyl | 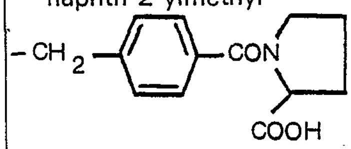 | H | 315 |
| 216 | phenyl (*N*-methyl) | benzyl | H | 316 |
| 217 | phenyl | *p*-aminobenzyl | H | 317 |
| 217A (Isomer A) | phenyl | p-aminobenzyl | H | 317A |
| 218A (Isomer A) | phenyl | p-acetamidobenzyl | H | 318A |
| 219A (Isomer A) | phenyl | *p*-methanesulphon amidobenzyl | H | 319A |
| 220A | phenyl | p-trifluoromethane-sulphonamidobenzyl | H | 320A |
| 221 | *p*-cyanophenyl | *p*-methylbenzyl | methyl | 321 |
| 222 | *p*-acetamidophenyl | *p*-methylbenzyl | methyl | 322 |
| 223 | phenyl | *m*-methylbenzyl | methyl | 323 |
| 224 | phenyl | p-chlorobenzyl | methyl | 324 |
| 225 | phenyl | *m*-chlorobenzyl | methyl | 325 |
| 226 | phenyl | *p*-nitrobenzyl | methyl | 326 |
| 227 | phenyl | *p*-trifluoromethylbenzyl | methyl | 327 |
| 228 | phenyl | 4-biphenylmethyl | methyl | 328 |
| 229 | phenyl | *p*-t-butylbenzyl | methyl | 329 |
| 230 | phenyl | 3-phenylprop-2-enyl | methyl | 330 |
| 231 | phenyl | prop-2-enyl | methyl | 331 |
| 232 | phenyl | 2-methylprop-2-enyl | methyl | 332 |
| 233 | phenyl | 3-phenylpropyl | methyl | 333 |
| 234 | phenyl | n-propyl | methyl | 334 |
| 235 | phenyl | isobutyl | methyl | 335 |
| 276 | phenyl | *o*-chlorobenzyl | methyl | 376 |
| 61A | phenyl-$CH_2$— $CH(NHCOOCH_2C_6H_5)$— | benzyl | H | 161A |

| Starting Material Compound No. | $R^1$ | $R^3$ | $R^4$ | Acid Compound No. |
|---|---|---|---|---|
| 62A | $CH_2CH(NHCO—OCH_2C_6H_5)—$ | benzyl | H | 162A |
| 63A | phenyl-NH— | benzyl | H | 163A |
| (161A) | phenyl-$CH_2CH(NH_2)—$ | benzyl | H | 164A* |
| 278 | *p*-nitrophenyl | benzyl | H | 378 |
| 279 | *p*-chlorophenyl | benzyl | H | 379 |

* Prepared from Compound No. 161A by hydrogenolysis of the benzyloxycarbonyl group by a similar process to that described in Example 12.

Example 33

*N,N'*-Dicyclohexylcarbodiimide (0.93 g.) was added to a stirred solution of *N*-[(R,S)-5-benzamido-(R,S)-2-methyl-4-oxo-6-phenylhexanoyl]-L-proline (Compound No. 26; 2.09 g.), 4-dimethylaminopyridine (0.06 g.) and methanol (2.0 ml.) in tetrahydrofuran (25 ml.) which was cooled to 0°C. and the mixture was stirred at laboratory temperature for 18 hours, recooled to 0°C. and filtered. The filtrate was evaporated to dryness under reduced pressure, the residue was dissolved in methylene chloride and the solution was washed successively with 1% w/v aqueous citric acid solution, water, 1% w/v aqueous sodium bicarbonate solution and water, dried over magnesium sulphate and evaporated to dryness under reduced pressure. The residue was purified by chromatography on a silica gel column using a 1:1 v/v mixture of methylene chloride and ethyl acetate as eluant. There was thus obtained, as a foam, *N*-[(R,S)-5-benzamido-(R,S)-2-methyl-4-oxo-6-phenylhexanoyl]-L-proline methyl ester (Compound No. 236), the structure of which was confirmed by elemental analysis and spectroscopic procedures.

The process described above was repeated except that the appropriate *N*-acyl-L-proline (Compound No. 26, 27 or 27A), and the appropriate alcohol were used as starting materials. There were thus obtained the compounds described in the following table, the structures of which were all confirmed by elemental analysis and spectroscopic procedures:—

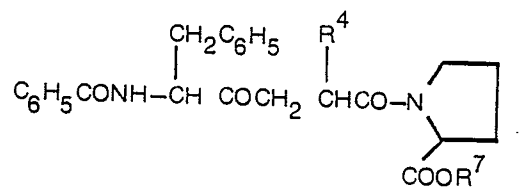

| $R^4$ | $R^7$ | Compound No. |
|---|---|---|
| methyl | isopropyl | 237 |
| methyl | n-pentyl | 238 |
| methyl | *β*-morpholinoethyl | 239 |
| methyl | *β*-dimethylaminoethyl | 240 |
| H (Isomer A) | isopropyl | 241A |
| H (Mixed Isomers) | *p*-chlorophenyl | 242 |
| H (Isomer A) | methyl | 283A |

There was also similarly prepared, from *N*-[(R,S)-5-benzamido-4-oxo-6-phenylhexanoyl]-L-proline (Compound No. 27) and thiophenol, *N*-[(R,S)-5-benzamido-4-oxo-6-phenylhexanoyl]-L-proline thiophenyl ester (Compound No. 243).

Example 34

Chloromethyl pivaloate (0.66 ml.) was added to a solution of *N*-[(R,S)-5-benzamido-4-oxo-6-phenylhexanoyl]-L-proline (Compound No. 27; 0.71 g.) and triethylamine (0.45 ml.) in ethyl acetate (20 ml.) which had been stirred at laboratory temperature for 30 minutes, and the mixture was then heated under reflux for 18 hours and evaporated to dryness under reduced pressure. The residue was purified by chromatography on a silica gel column using a 3:1 v/v mixture of methylene chloride and ethyl acetate as eluant. There was thus obtained *N*-[(R,S)-5-benzamido-4-oxo-6-phenylhexanoyl]-L-proline pivaloyloxymethyl ester, (Compound No. 244) the structure of which was confirmed by elemental analysis and spectroscopic procedures.

Example 35

The process described in Example 1 was repeated using (R,S)-5-benzamido-(R,S)-2-methyl-4-oxo-6-phenylhexanoic acid and the appropriate L-proline derivative as starting materials, and there were thus obtained as foams the compounds described in the following table, the structures of which were all confirmed by elemental analysis and spectroscopic procedures:—

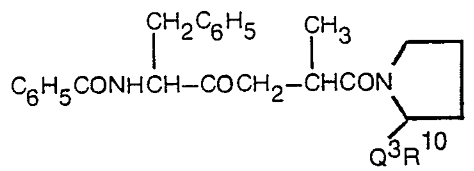

| —$Q^3R^{10}$ | Compound No. |
|---|---|
| —$COOCH_2C_6H_5$ | 245 |
| —$CONH_2$ | 246 |
| —$CH_2OH$ | 247 |

Example 36

*N*-[(R,S)-5-Benzamido-(R,S)-2-methyl-4-oxo-6-phenylhexanoyl]-L-proline methyl ester (Compound No. 236, Example 33, 0.5 g.) was dissolved in saturated methanolic ammonia solution (20 ml.) and the mixture was kept at laboratory temperature for 10 days and then evaporated to dryness under reduced pressure. The residue was purified by chromatography on a silica gel column using a 19:1 v/v mixture of methylene chloride and methanol as eluant. There was thus obtained as a foam 1-[(R,S)-5-benzamido-(R,S)-2-methyl-4-oxo-6-phenylhexanoyl]-pyrrolidine-(S)-2-carboxamide (Compound No. 246), the structure of which was confirmed by elemental analysis and spectroscopic procedures. The compound was identical to that obtained in Example 35.

Example 37

*N,N'*-Dicyclohexylcarbodi-imide (0.47 g.) was added to a stirred solution of *N*-[(R,S)-benzamido-(R,S)-2-methyl-4-oxo-6-phenylhexanoyl]-L-proline (Compound No. 26; 1.0 g.) 1-hydroxybenzotriazole (0.3 g.) and β-dimethylaminoethylamine (1.0 ml.) which was cooled to 0°C, and the mixture was stirred at laboratory temperature for 16 hours, recooled to 0°C. and filtered. The filtrate was evaporated to dryness under reduced pressure, the residue was dissolved in methylene chloride (100 ml.) and the solution was washed with 1% aqueous sodium bicarbonate solution and then with water, dried over magnesium sulphate and evaporated to dryness under reduced pressure. The residue was purified by chromatography on a silica gel column using a 9:1 v/v mixture of methylene chloride and methanol as eluant, and there was thus obtained, as a foam, 1-[(R,S)-benzamido-(R,S)-2-methyl-4-oxo-6-phenylhexanoyl]pyrrolidine-(S)-2-(*N*-β-dimethylaminoethyl)carboxamide (Compound No. 248), the structure of which was confirmed by elemental analysis and spectroscopic procedures.

The process described above was repeated except that a 33% w/v solution of ethylamine in ethanol was used in place of β-dimethylaminoethylamine. There was thus obtained 1-[(R,S)-5-benzamido-(R,S)-2-methyl-4-oxo-6-phenylhexanoyl]pyrrolidine-(S)-2-N-ethylcarboxamide (Compound No. 249).

Example 38

A solution of diazomethane in diethyl ether was added to a solution of *N*-[(R,S)-5-benzamido-(R,S)-2-methyl-4-oxo-6-phenylhexanoyl]-L-proline (Compound No. 26; 0.11 g.) in diethyl ether (2 ml.) until the yellow colour persisted. The mixture was then evaporated to dryness by blowing a stream of argon through it, and there was thus obtained as an oil *N*-[(R,S)-5-benzamido-(R,S)-2-methyl-4-oxo-6-phenylhexanoyl]-L-proline methyl ester (Compound No. 236), identical to that prepared according to Example 33.

The process described above was repeated using the appropriate acid prepared according to Example 32, and there were thus obtained the esters described in the following table:—

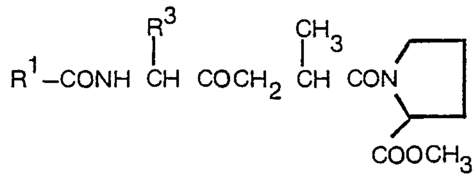

| $R^1$ | $R^3$ | Compound No. |
|---|---|---|
| phenyl | 3-phenylprop-2-enyl | 250 |
| phenyl | 3-phenylpropyl | 251 |
| *p*-cyanophenyl | *p*-methylbenzyl | 252 |
| p-acetamidophenyl | *p*-methylbenzyl | 253 |
| phenyl | *p*-methylbenzyl | 254 |
| phenyl | *m*-methylbenzyl | 255 |
| phenyl | *p*-chlorobenzyl | 256 |
| phenyl | *m*-chlorobenzyl | 257 |
| phenyl | *o*-chlorobenzyl | 258 |
| phenyl | *p*-cyanobenzyl | 259 |
| phenyl | *p*-methoxybenzyl | 260 |
| phenyl | *p*-nitrobenzyl | 261 |
| phenyl | *p*-trifluoromethylphenyl | 262 |

Example 39

Silica gel (Merck Kieselgel 60, 70—230 mesh ASTM: 450 g.) was deactivated with water (50 ml.) ethyl acetate (25 ml.) and toluene (25 ml.) and the mixture was allowed to stand for 16 hours and then packed into a tube of 5 mm. diameter. *N*-[(R,S)-5-Benzamido-(R,S)-2-methyl-4-oxo-6-phenylhexanoyl]-L-proline t-butyl ester (Compound No. 6; 3 g.) was absorbed onto silica gel (6 g.) and the mixture was placed on top of the column which was then developed with a 1:1 v/v mixture of ethyl acetate and toluene. The column was dried and cut up into bands from which were separately isolated two isomers of the ester (isomeric at the 2-carbon atom of the hexanoyl chain). The less polar isomer (Compound No. 6A) was 10 times more active as an inhibitor of angiotensin converting enzyme than the more polar isomer (Compound No. 6B).

The less polar isomer (1.5 g.) was reapplied to a similar column to that described above which consisted of 1500 g. of silica gel and again separate bands of the dried and cut up column yielded two isomers (isomeric at the 5-carbon atom of the hexanoyl chain). The more polar isomer (Compound No. 6C) was 100 times more active as an inhibitor or angiotensin converting enzyme than the less polar isomer (Compound No. 6D).

A mixture of a single isomer at the 5-carbon atom (0.2 g.) dissolved in dimethoxyethane (15 ml.) and aqueous 2N-sodium hydroxide solution was kept at laboratory temperature for 36 hours and neutralised with aqueous N-hydrochloric acid. The organic material was isolated by conventional

procedures and found to be a mixture of isomers at the 5-carbon atom. These could be separated as described above and thus it is possible to convert a less active isomer (for example Compound No. 6D) into a more active isomer (for example Compound No. 6C).

The t-butyl ester Compound No. 6C was converted into the free acid by the process described in Example 5, and there was thus obtained one isomer of *N*-(5-benzamido-2-methyl-4-oxo-6-phenylhexanoyl)-L-proline (Compound No. 26C).

Example 40

The process described in Example 1 was repeated except that (R,S)-5-benzamido-(R,S)-2-methyl-4-oxo-6-phenylhexanoic acid and the appropriate amino acid methyl ester were used as starting materials. There were thus obtained the compounds described in the following table, most of which were oils or low-melting solids, all of which were purified by chromatography on silica gel, and the structures of all of which were confirmed by elemental analysis and spectroscopic procedures:

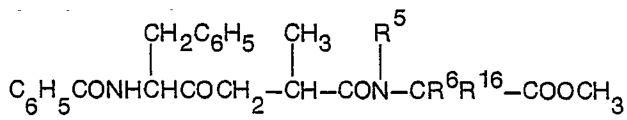

| $R^5$ | $R^6$ | $R^{16}$ | Compound No. |
|---|---|---|---|
| $-(CH_2)_2-$ | | H | 263 (Note 1) |
| $-(CH_2)_4-$ | | H | 264 |
| $-(CH_2)_5-$ | | H | 265 |
| $-CH_2CHOHCH_2-$ | | | 266 (more polar isomer), 267 (less polar isomer) (Note 2) |
| H | $CH_3$ | $CH_3$ | 268 |
| H | $-(CH_2)_4-$ | | 269 (m.p. 121–124°C) |
| H | Indol-3-yl-methyl | H | 294 (Note 3) |

*Note 1.* The amino acid ester used was methyl azetidine-(S)-2-carboxylate.
*Note 2.* The amino acid ester used was (R)-4-hydroxy-L-proline methyl ester.
*Note 3.* The amino acid ester used was L-tryptophan methyl ester.

Example 41

The process described in Example 7 was repeated using the appropriate methyl ester described in Example 40. There were thus obtained the acids shown in the following table:—

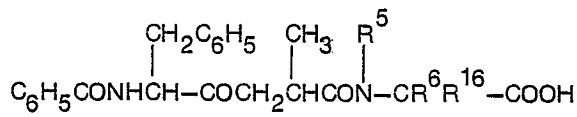

| $R^5$ | $R^6$ | $R^{16}$ | Compound No. |
|---|---|---|---|
| $-(CH_2)_5-$ | | H | 365 |
| H | $CH_3$ | $CH_3$ | 368 |
| H | $-(CH_2)_4-$ | | 369 (m.p. 157–158°C) |
| H | Indol-3-yl-methyl | H | 394 |

The structures of all these compounds were confirmed by elemental analysis and spectroscopic procedures.

Example 42

A solution of methyl thiazolidine-(S)-4-carboxylate (0.294 g.) and succinic anhydride (0.2 g.) in methylene chloride (2 ml.) was stirred at laboratory temperature for 2 hours, cooled to −30°C. and oxalyl chloride (0.27 g.) and dimethylformamide (1 drop) were added. The mixture was stirred at −15°C for 2 hours and then evaporated to dryness, the residue was dissolved in tetrahydrofuran (1.5 ml.) and the mixture was added during 5 minutes to a stirred solution of 4-benzyl-2-phenyloxazol-5(4H)-one (0.5 g.), 4-dimethylaminopropyridine (0.03 g.) and triethylamine (0.56 ml.) in tetrahydrofuran (4 ml.) which was cooled to −50°C. The mixture was stirred for 2 hours at −40°C, then 40 hours at laboratory temperature, acetic acid (3 ml.) was added and the mixture was heated under reflux for 3 hours and then evaporated to dryness under reduced pressure. The residue was dissolved in ethyl acetate and the solution was washed successively with water, aqueous 2N-hydrochloric acid, saturated aqueous sodium chloride solution, saturated aqueous sodium bicarbonate solution and saturated aqueous sodium chloride solution, dried over magnesium sulphate and evaporated to dryness. The residue was purified by chromatography on a silica gel column using a 1:1 v/v mixture of ethyl acetate and toluene as eluant, and there was thus obtained as a foam methyl *N*-[(R,S)-5-benzamido-4-oxo-6-phenylhexanoyl]-thiazolidine-(S)-4-carboxylate, (Compound No. 270) the structure of which was confirmed by elemental analysis and spectroscopic procedures.

Example 43

The process described in Example 7 was repeated using Compound No. 270 (Example 42) as starting material. There was thus obtained *N*-[(R,S)-5-benzamido-4-oxo-6-phenylhexanoyl]-thiazolidine-(S)-4-carboxylic acid (Compound No. 370).

Example 44

*N*-Methylmorpholine (0.31 g.) was added to a stirred solution of (R,S)-5-benzamido-(R,S)-2-methyl-4-oxo-6-phenylhexanoic acid (1.02 g.), L-homoserine lactone hydrochloride (0.42 g.), *N*-hydroxybenzotriazole (0.45 g.) and dicyclohexylcarbodi-imide (0.65 g.) in dimethylformamide (8 ml.) which was cooled to 0°C., and the mixture was stirred at 10°C. for 3 hours and then at laboratory temperature for 16 hours, and then filtered. The filtrate was evaporated to dryness under reduced pressure, the residue was partitioned between ethyl acetate and aqueous 2N-hydrochloric acid and the organic layer was separated, washed successively with aqueous 2N-hydrochloric acid, saturated aqueous sodium carbonate solution (twice) and saturated aqueous sodium chloride solution, dried and evaporated to dryness. The residue was purified by chromatography on a silica gel column using a 19:1 v/v mixture of chloroform and methanol as eluant, and there was thus obtained *N*-[(R,S)-5-benzamido-(R,S)-2-methyl-4-oxo-6-phenylhexanoyl]-L-homoserine lactone (Compound No. 271), m.p. 104—109°C.

Example 45

Hydroxylamine hydrochloride (1.0 g.) and pyridine (1 ml.) were added to a solution of *N*-[(R,S)-5-benzamido-4-oxo-6-phenylhexanoyl)-L-proline t-butyl ester (Compound No. 7; 1.0 g.) in ethanol (20 ml.) and the mixture was heated under reflux for 40 minutes and then evaporated to dryness under reduced pressure. The residue was dissolved in ethyl acetate (75 ml.) and the solution was washed successively with 30 ml. each of aqueous 5% hydrochloric acid, water, saturated aqueous sodium bicarbonate solution and saturated sodium chloride solution, dried over sodium sulphate and evaporated to dryness. There was thus obtained as oily residue *N*-[(R,S)-5-benzamido-4-hydroxy-imino-6-phenylhexanoyl]-L-proline t-butyl ester (Compound No. 272), the structure of which was confirmed by elemental analysis and spectroscopic procedures.

Example 46

The process described in Example 5 was repeated using Compound No. 272 (Example 45) as starting material. There was thus obtained *N*-[(R,S)-5-benzamido-4-hydroxyimino-6-phenylhexanoyl]-L-proline (Compound No. 372).

Example 47

The process described in Example 14 was repeated except that L-proline methyl ester was used in place of L-proline t-butyl ester. There was thus obtained as an oil *N*-[(R,S)-5-benzyloxycarbonylamino-(R,S)-2-methyl-4-oxo-6-phenylhexanoyl]-L-proline methyl ester (Compound No. 273), the structure of which was confirmed by elemental analysis and spectroscopic procedures.

Example 48

S-Thiobenzoylthioglycollic acid (0.33 g.) was added to a stirred solution of *N*-[(R,S)-5-amino-(R,S)-2-methyl-4-oxo-6-phenylhexanoyl]-L-proline methyl ester (Compound No. 67; Example 17; 0.54 g.) and triethylamine (0.2 ml.) in pyridine (9 ml.) and the mixture was stirred at laboratory temperature for 40 hours and then poured into aqueous 2N-sulphuric acid. The mixture was extracted with ethyl acetate and the extract was washed with dilute aqueous sodium bicarbonate solution, dried over magnesium sulphate and evaporated to dryness. The residue was purified by chromatography on a silica gel column (18 g.) using an 11:8:1 v/v/v mixture of toluene, ethyl acetate and methanol as eluant. There was thus obtained as an oil *N*-[(R,S)-2-methyl-4-oxo-6-phenyl-(R,S)-5-thiobenzamidohexanoyl]-L-proline methyl ester (Compound No. 274), the structure of which was confirmed by elemental analysis and spectroscopic procedures.

Example 49

The process described in Example 33 was repreated using Isomer A of *N*-(5-*p*-cyanobenzamido-4-oxo-6-phenylhexanoyl)-L-proline (Compound No. 145A, Example 13) and methanol as starting materials, and there was thus obtained as an oil Isomer A of *N*-(5-*p*-cyanobenzamido-4-oxo-6-phenyl-hexanoyl)-L-proline methyl ester (Compound No. 284A), the structure of which was confirmed by elemental analysis and spectroscopic procedures.

Example 50

The process described in Example 1 was repeated except that (R,S)-5-benzamido-(R,S)-2-methyl-4-oxo-6-phenylhexanoic acid and the appropriate amino acid ethyl ester were used as starting materials. There were thus obtained the compounds described in the following table, all of which were oils, all of which were purified by chromatography on silica gel, and the structures of all of which were confirmed by elemental analysis and spectroscopic procedures:—

$$C_6H_5CONH\underset{\phantom{x}}{\overset{CH_2C_6H_5}{CH}}-COCH_2-\overset{CH_3}{CH}-CO\overset{R^5}{N}-CHR^6-COOC_2H_5$$

| $R^5$ | $R^6$ | Compound No. |
|---|---|---|
| $-CH_2CH_2SCH_2-$ | | 285 |
| $-CH_2CH_2OCH_2-$ | | 286 |
| $-CH_2CH_2\underset{CH_3}{N}CH_2-$ | | 287 |
| $-CH_2CH=CH-$ | | 288 (Note 1) |
| $-CH_2SCH_2-$ | | 289 (Notes 1, 2) |
| $CH_3$ | H | 290 (Note 2) |

*Note 1.* The L-amino acid ethyl ester was used as starting material.
*Note 2.* A mixed anhydride procedure using isobutyl chloroformate (similar to that described in Example 19) was used instead of dicyclohexylcarbodiimide to couple the acid and the aminoacid ester.

Example 51

The process described in Example 45 was repeated except that methoxyamine or benzyloxyamine were used in place of hydroxylamine. There were thus obtained *N*-[(R,S)-5-benzamido-4-methoxyimino-6-phenylhexanoyl]-L-proline t-butyl ester (Compound No. 291) and *N*-[(R,S)-5-benzamido-4-benzyloxyimino-6-phenylhexanoyl]-L-proline t-butyl ester (Compound No. 292), the structures of which were confirmed by elemental analysis and spectroscopic procedures.

Example 52

The process described in Example 46 was repeated using Compounds Nos. 291 and 292 (Example 51) as starting materials. There was thus obtained *N*-[(R,S)-5-benzamido-4-methoxyimino-6-phenylhexanoyl]-L-proline (Compound No. 391) and *N*-[(R,S)-5-benzamido-4-benzyloxyimino-6-phenylhexanoyl]-L-proline (Compound No. 392).

Example 53

The process described in Example 1 was repeated except that (R,S)-5-benzamido-(R,S)-3-methyl-4-oxo-6-phenylhexanoic acid and L-proline t-butyl ester were used as starting materials. There was thus obtained *N*-[(R,S)-5-benzamido-(R,S)-3-methyl-4-oxo-6-phenylhexanoyl]-L-proline t-butyl ester (Compound No. 293).

This compound was converted to the free acid by the process described in Example 5, and there was thus obtained *N*-[(R,S)-5-benzamido-(R,S)-3-methyl-4-oxo-6-phenylhexanoyl]-L-proline (Compound No. 393).

The structures of both ester and acid were confirmed by elemental analysis and spectroscopic procedures.

The (R,S)-5-benzamido-(R,S)-3-methyl-4-oxo-6-phenylhexanoic acid used as starting material was obtained by a similar process to that described in the second part of Example 1, using 3-methoxycarbonyl-2-methylpropionyl chloride and 4-benzyl-2-phenyloxazol-5(4H)-one as starting materials.

Example 54

The process described in Example 1 was repeated using 4-oxo-6-phenyl-(R,S)-5-*p*-toluenesulphonamidohexanoic acid and L-proline t-butyl ester as starting materials. There are thus obtained *N*-[4-oxo-6-phenyl-(R,S)-5-*p*-toluenesulphonamidohexanoyl]-L-proline t-butyl ester (Compound No. 295).

This compound was converted to the free acid by the process described in Example 5, and there was thus obtained *N*-[4-oxo-6-phenyl-(R,S)-5-*p*-toluenesulphonamidohexanoyl]-L-proline (Compound No. 395).

The structures of both ester and acid were confirmed by elemental analysis and spectroscopic procedures.

The 4-oxo-6-phenyl-(R,S)-5-*p*-toluenesulphonamidohexanoic acid used as starting material was obtained by a similar process to that described in the last two parts of Example 4, from *p*-toluenesulphonyl chloride and methyl (R,S)-5-amino-4-oxo-6-phenylhexanoate.

Example 55

Sodium borohydride (0.14 g.) was added to a solution of *N*-[(R,S)-5-benzamido-(R,S)-2-methyl-4-oxo-6-phenylhexanoyl]-L-proline (Compound No. 26; 0.412 g.) in methanol (10 ml.), and after 15 minutes the mixture was poured into water (50 ml.). The methanol was removed by evaporation under reduced pressure, the aqueous residue was acidified to pH 3 with aqueous 2N-hydrochloric acid and the mixture was extracted with ethyl acetate. The extract was washed with water, dried and evaporated to dryness and the residue was purified by chromatography on a silica gel column using an 11:7:1:1 v/v/v/v mixture of toluene: ethyl acetate: formic acid: methanol as eluant. There was thus obtained, as an oil, *N*-[(R,S)-5-benzamido-(R,S)-4-hydroxy-(R,S)-2-methyl-6-phenylhexanoyl]-L-proline (Compound No. 396), the structure of which was confirmed by elemental analysis and spectroscopic procedures.

The process described above was repeated using *N*-[(R,S)-5-benzamido-4-oxo-6-phenylhexanoyl]-L-proline (Compound No. 27) as starting material. There was thus obtained *N*-[(R,S)-5-benzamido-(R,S)-4-hydroxy-6-phenylhexanoyl]-L-proline (Compound No. 397).

**Claims for the Contracting States: BE CH DE FR GB IT LI LU NL SE**

1. An amide derivative of the formula:—

$$R^1—Y—NR^2—CHR^3—X—CHR^{11}—CHR^4—CO—NR^5—CR^6R^{16}—Q—R^{10}$$

wherein either $R^1$ is hydrogen, or alkyl of up to 15 carbon atoms which is unsubstituted, or which bears one substituent selected from amino, benzyloxycarbonylamino, hydroxy, alkoxy, alkylthio and alkoxycarbonyl each of up to 5 carbon atoms, and aryl, aryloxy and arylthio substituents; or which bears two or three substituents one of which is aryl, another of which is aryl, hydroxy, amino, benzyloxycarbonylamino, trifluoromethyl, aryloxy or alkoxy of up to 5 carbon atoms and the third of which, if present, is aryl or trifluoromethyl;

or $R^1$ is aryl or, when Y is carbonyl or thiocarbonyl, is aryloxy, alkoxy or arylalkoxy wherein the alkoxy part has up to 5 carbon atoms;

or $R^1$ is halogenoalkyl of up to 6 carbon atoms or is alkenyl, halogenoalkyl or cycloalkyl each of up to 6 carbon atoms which is unsubstituted or which bears an aryl substituent;

or $R^1$ is a substituent of the formula $R^8CONHCHR^9$— or $R^8COCH_2CHR^9$— wherein $R^8$ is alkyl or cycloalkyl each of up to 10 carbon atoms, or aryl and $R^9$ is hydrogen, or alkyl of up to 5 carbon atoms which is unsubstituted or which bears an aryl substituent, or $R^9$ is a group other than those stated above such that the compound $H_2NCHR^9COOH$ would be a naturally-occurring amino acid;

wherein $R^2$ is hydrogen, alkyl of up to 5 carbon atoms which is unsubstituted or which bears an aryl substituent, or aryl;

wherein $R^3$ is alkyl or alkenyl each of up to 5 carbon atoms which is unsubstituted or which bears an aryl substituent, or $R^3$ is aryl or indolylmethyl;

wherein $R^4$ is hydrogen or alkyl of up to 5 carbon atoms which is unsubstituted or which bears an aryl substituent;

wherein either $R^5$ is hydrogen or aryl, or alkyl of up to 5 carbon atoms which is unsubstituted or which bears an aryl substituent, or $R^5$ is joined together with $R^6$ as defined below;

wherein either $R^6$ is hydrogen, aryl or heterocyclic, or alkyl of up to 5 carbon atoms which is unsubstituted or which bears a hydroxy, aryl or heterocyclic substituent;

or $R^6$ and $R^5$ are joined together to form alkylene or alkenylene of 2 to 5 carbon atoms; or an oxa, thia or aza-derivative of said alkylene or alkylene; or a hydroxy- or oxo-substituted derivative of said alkylene or alkenylene; or $R^6$ and $R^{16}$, or $R^6$, $R^{16}$ and $R^5$, $R^6$ and $R^{10}$ are joined together as defined below;

wherein $R^{16}$ is hydrogen or alkyl of up to 5 carbon atoms;

or $R^6$ and $R^{16}$ are joined together to form alkylene of 2 to 5 carbon atoms (that is, to form a spirocycloalkyl group);

or $R^{16}$ together with the first carbon atom of $R^6$ form a double bond wherein $R^6$ is otherwise alkyl, or wherein $R^6$ is otherwise substituted alkyl as defined above, or wherein $R^6$ and $R^5$ are otherwise joined together as defined above (that is, so that $R^5$, $R^6$ and $R^{16}$ together form alkylidene);

wherein Q is carbonyl (—CO—) or methylene (—$CH_2$—);

and wherein either $R^{10}$ is hydroxy, amino, aryloxy, arylthio, alkoxy, cycloalkoxy, alkylamino, dialkylamino, cyclic amino, hydroxyalkoxy, acyloxyalkoxy, aminoalkoxy, alkylaminoalkoxy, dialkylaminoalkoxy, cyclic aminoalkoxy, aminoalkylamino, alkylaminoalkylamino, dialkylaminoalkylamino, cyclic aminoalkylamino or aryl alkoxy wherein each alkyl or alkoxy has up to 5 carbon atoms and wherein cyclic amino has up to 6 carbon atoms;

or wherein $R^{10}$ and $R^6$ are joined together such that $R^{10}$ is oxygen (—O—) joined to the terminal carbon atom of $R^6$ when it is alkyl;

wherein $R^{11}$ is hydrogen or alkyl of up to 3 carbon atoms;

wherein X is carbonyl (—CO—), hydroxymethylene (—CHOH—), thiocarbonyl (—CS—) or oximinomethylene (—C=N—$OR^{20}$ wherein $R^{20}$ is hydrogen or alkyl of up to 5 carbon atoms which is unsubstituted or which bears an aryl substituent);

and wherein Y is carbonyl, thiocarbonyl, sulphonyl (—$SO_2$—) or amido (—NHCO—);

or a salt thereof where appropriate.

2. An amide derivative as claimed in claim 1 wherein X, Y and Q are all carbonyl and $R^2$ and $R^{11}$ are both hydrogen, or a salt thereof.

3. An amide derivative having the formula:—

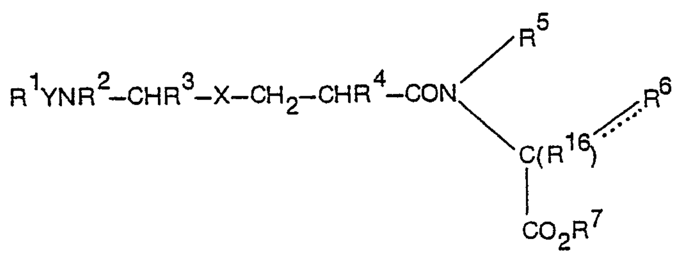

wherein $R^1$ is alkyl of up to 15 carbon atoms which is unsubstituted or which bear an aryl, aryloxy or arylthio substituent, or $R^1$ is aryl or aryloxy, or $R^1$ is a substituent of the formula $R^8CONHCHR^9$— or $R^8COCH_2CHR^9$— wherein $R^8$ is alkyl or cycloalkyl each of up to 10 carbon atoms, or aryl, and $R^9$ is hydrogen, or alkyl of up to 5 carbon atoms which is unsubstituted or which bears an aryl substituent, or $R^9$ is a group other than those stated above such that the compound $N_2NCHR^9COOH$ would be a naturally-occurring amino acid;

wherein Y and $R^2$ have the meanings stated in claim 1;

wherein $R^3$ is alkyl or alkenyl each of up to 5 carbon atoms which is unsubstituted or which bears an aryl substituent, or $R^3$ is aryl or indolylmethyl;

wherein X is carbonyl, hydroxymethylene, thiocarbonyl or oximinomethylene (>C=N—OH);

wherein $R^4$ is hydrogen or alkyl of up to 5 carbon atoms which is unsubstituted or which bears an aryl substituent;

wherein either $R^5$ is hydrogen, aryl or alkyl of up to 5 carbon atoms which is unsubstituted or which bears an aryl substituent, $R^6$ is hydrogen, alkyl of up to 5 carbon atoms which is unsubstituted or which bears an aryl or heterocyclic substituent, or $R^6$ is heterocyclic or aryl, and $R^{16}$ is hydrogen;

or $R^5$ and $R^6$ are joined together to form alkylene or alkenylene of 2 to 4 carbon atoms; or an oxa, thia or aza-derivative of said alkylene or alkenylene;

or $R^6$, $R^{16}$ and $R^5$ are joined together such that $R^{16}$ together with the first carbon atom of $R^6$ form a double bond wherein $R^6$ and $R^5$ are otherwise joined together as defined above;

and wherein $R^7$ is hydrogen or alkyl of up to 5 carbon atoms;

or a salt thereof.

4. An amide derivative having the formula:—

$$R^1CONH—CHR^3—COCH_2—CHR^4—CONR^5—CHR^6—COR^{10}$$

wherein $R^1$ is methyl, benzyl, β-phenylethyl, benzyloxy, phenoxymethyl, phenylthiomethyl, phenyl, tolyl, methoxyphenyl, methylthiophenyl, monochlorophenyl, dichlorophenyl, fluorophenyl, iodophenyl, nitrophenyl, cyanophenyl, trifluoromethylphenyl, acetamidophenyl, acetylphenyl, methanesulphonylphenyl, biphenylyl, naphthyl, benzamidomethyl, cyclopentanecarbonamidomethyl or β-benzoylethyl;

$R^3$ is benzyl which is unsubstituted or bears a methyl, t-butyl, methoxy, fluoro, chloro, bromo, nitro, cyano, trifluoromethyl, amino, acetamido, methanesulphonamido, trifluoromethanesulphonamido or phenyl substituent, or

$R^3$ is 3-phenylpropyl or 3-phenylprop-2-enyl;

$R^4$ is hydrogen or methyl;

$R^5$ is hydrogen or methyl and $R^6$ is hydrogen, benzyl or indol-3-ylmethyl, or $R^5$ and $R^6$ together form trimethylene, tetramethylene, pentamethylene, 2-hydroxytrimethylene, 2-thiatrimethylene, 3-thiatetramethylene, 3-oxatetramethylene, 3-methyl-3-azatetramethylene; and

$R^{10}$ is hydroxy, amino, alkoxy of up to 5 carbon atoms, or

β-dimethylaminoethoxy or β-morpholinoethoxy;

or a salt thereof.

5. An amide derivative having the formula:—

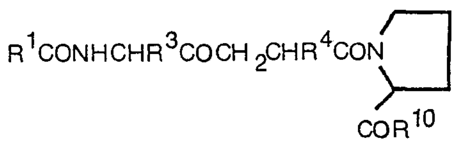

wherein $R^1$ is phenyl, *p*-acetamidophenyl or *p*-cyanophenyl, $R^3$ is benzyl, *p*-methylbenzyl or *p*-methoxybenzyl, $R^4$ is hydrogen or methyl and $R^{10}$ is hydroxy, amino, methoxy, ethoxy, isopropoxy or t-butoxy, or a salt thereof.

6. The compound *N*-(5-acetamido-2-methyl-4-oxo-6-phenylhexanoyl-L-proline, *N*-(5-benzamido-2-methyl-4-oxo-6-phenylhexanoyl)-L-proline or *N*-(5-phenylacetamido-2-methyl-4-oxo-6-phenylhexanoyl)-L-proline, or a salt thereof, and or an ester thereof with an alcohol of up to 5 carbon atoms, or an amide thereof.

7. The compound *N*-(5-benzamido-2-methyl-4-oxo-6-phenylhexanoyl)-L-proline, or its methyl ester, or its amide, or a salt thereof.

8. A salt of an amide derivative, claimed in any of claims 1 to 7 wherein $R^{10}$ is hydroxy which is an alkali metal or alkaline earth metal salt, or an ammonium or dicyclohexylamine salt.

9. A process for the manufacture of an amide derivative or a salt thereof, claimed in any of claims 1 to 8, which comprises (a) the reaction of an acid of the formula:—

$$R^1—Y—NR^2—CHR^3—X—CHR^{11}—CHR^4—COOH$$

wherein $R^1$, $R^2$, $R^3$, $R^4$, $R^{11}$, X and Y have the meanings stated in any of claims 1 to 5, or of a reactive derivative thereof, with an amine of the formula:—

$$HNR^5—CR^6R^{16}—Q—R^{12}$$

wherein $R^5$, $R^6$, $R^{16}$ and Q have the meanings stated in any of claims 1 to 5 and wherein $R^{12}$ has any of the meanings stated in claims 1 to 5 for $R^{10}$ except those containing a free hydroxy or amino group; or

(b) for the manufacture of an amide derivative wherein $R^2$ is hydrogen and X is carbonyl or thiocarbonyl, the reaction of an amine of the formula:

$$H_2NCHR^3—X^1—CHR^{11}CHR^4CONR^5—CR^6R^{16}—Q—R^{12}$$

wherein $R^3$, $R^4$, $R^5$, $R^6$, $R^{11}$, $R^{12}$, $R^{16}$ and Q have the meanings stated above and wherein $X^4$ is carbonyl or thiocarbonyl, with a compound of the formula $R^1—Y—Z^1$, wherein $R^1$ and Y have the meanings stated above and wherein $Z^1$ is a displaceable halogen, alkanesulphonyl or arensulphonyl group, or $Z^1$ is hydroxy (in which case the reaction is carried out in the presence of a carbodiimide or an alkyl chloroformate), or, when Y is amido, with a compound of the formula $R^1NCO$, or, when Y is thiocarbonyl, with a compound of the formula $R^1CS—SCH_2COOH$; or

(c) for the manufacture of an amide derivative wherein $R^2$ is hydrogen and X is a carbonyl, the reaction of a compound of the formula:—

$$Z^2COCHR^{11}CHR^4CONR^5CR^6R^{16}QR^{12}$$

wherein $R^4$, $R^5$, $R^6$, $R^{11}$, $R^{12}$, $R^{16}$, and Q have the meanings stated above and $Z^2$ is a displaceable halogen atom, with a compound of the formula

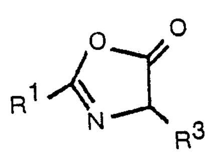

wherein $R^1$ and $R^3$ have the meanings stated above, followed by hydrolysis of the oxazolone ring; whereafter an amide derivative wherein $R^2$ is alkyl, arylalkyl or aryl (and $R^{10}$ is $R^{12}$) may be obtained by reacting the corresponding amide derivative wherein $R^2$ is hydrogen (and $R^{10}$ is $R^{12}$) with a compound of the compound of the formula $R^2Z$, wherein $R^2$ has the meaning stated above and Z is a displaceable halogen, alkanesulphonyl or arensulphonyl group; or whereafter an amide derivative wherein $R^3$ is unsubstituted or substituted alkyl as defined above may be obtained by the hydrogenation of the corresponding compound wherein $R^3$ is unsubstituted or substituted alkenyl as defined above; or whereafter an amide derivative wherein $R^{10}$ is other than hydroxy (that is, wherein —Q—$R^{10}$ forms an ester or amide group) may be obtained from the corresponding acid wherein —Q—$R^{10}$ is —COOH by conventional means of ester or amide formation; or whereafter an amide derivative wherein Q is carbonyl and $R^{10}$ is hydroxy may be obtained by the hydrolysis of the corresponding amide derivative

wherein $R^{10}$ is alkoxy, or, when $R^{10}$ is t-butoxy, by the acid-catalysed cleavage of said compound; or whereafter an amide derivative wherein X is hydroxymethylene may be obtained by the reduction of the corresponding amide derivative wherein X is carbonyl; or whereafter an amide derivative where X is oximinomethylene may be obtained by the reaction of the corresponding amide derivative wherein X is carbonyl with hydroxylamine or an *O*-substituted hydroxylamine derivative of the formula $H_2NOR^{20}$, wherein $R_{20}$ has the meaning stated in claim 1.

10. A pharmaceutical composition comprising as active ingredient at least one amide derivative, or a salt thereof, claimed in any of claims 1 to 8, in association with a pharmaceutically-acceptable diluent or carrier therefor.

**Claims for the Contracting State: AT**

1. A process for the manufacture of an amide derivative of the formula:—

$$R^1—Y—NR^2—CHR^3—X—CHR^{11}—CHR^4—CO—NR^5—CR^6R^{16}—Q—R^{10}$$

wherein either $R^1$ is hydrogen, or alkyl of up to 15 carbon atoms which is unsubstituted, or which bears one substituent selected from amino, benzyloxycarbonylamino, hydroxy, alkoxy, alkylthio and alkoxycarbonyl each of up to 5 carbon atoms, and aryl, aryloxy and arylthio substituents; or which bears two or three substituents one of which is aryl, another of which is aryl, hydroxy, amino, benzyloxycarbonylamino, trifluoromethyl, aryloxy or alkoxy of up to 5 carbon atoms and the third of which, if present, is aryl or trifluoromethyl;

or $R^1$ is aryl or, when Y is carbonyl or thiocarbonyl, is aryloxy, alkoxy or arylalkoxy wherein the alkoxy part has up to 5 carbon atoms;

or $R^1$ is halogenalkyl of up to 6 carbon atoms or is alkenyl, halogenoalkenyl or cycloalkyl each of up to 6 carbon atoms which is unsubstituted or which bears an aryl substituent;

or $R^1$ is a substituent of the formula $R^8CONHCHR^9$— or $R^8COCH_2CHR^9$— wherein $R^8$ is alkyl or cycloalkyl each of up to 10 carbon atoms, or aryl and $R^9$ is hydrogen, or alkyl of up to 5 carbon atoms which is unsubstituted or which bears an aryl substituent, or $R^9$ is a group other than those stated above such that the compound $H_2NCHR^9COOH$ would be a naturally occurring amino acid; wherein $R^2$ is hydrogen, alkyl of up to 5 carbon atoms which is unsubstituted or which bears an aryl substituent, or aryl;

wherein $R^3$ is alkyl or alkenyl each of up to 5 carbon atoms which is unsubstituted or which bears an aryl substituent, or $R^3$ is aryl or indolylmethyl;

wherein $R^4$ is hydrogen or alkyl of up to 5 carbon atoms which is unsubstituted or which bears an aryl substituent; wherein either $R^5$ is hydrogen or aryl, or alkyl of up to 5 carbon atoms which is unsubstituted or which bears an aryl substituent, or $R^5$ is joined together with $R^6$ as defined below;

wherein either $R^6$ is hydrogen, aryl or heterocyclic, or alkyl of up to 5 carbon atoms which is unsubstituted or which bears a hydroxy, aryl or heterocyclic substituent;

or $R^6$ and $R^5$ are joined together to form alkylene or alkenylene of 2 to 5 carbon atoms; or an oxa, thia or aza-derivative of said alkylene or alkenylene; or a hydroxy- or oxo-substituted derivative of said alkylene or alkenylene; or $R^6$ and $R^{16}$, or $R^6$, $R^{16}$ and $R^5$, or $R^6$ and $R^{10}$ are joined together as defined below;

wherein $R^{16}$ is hydrogen or alkyl of up to 5 carbon atoms;

or $R^6$ and $R^{16}$ are joined together to form alkylene of 2 to 5 carbon atoms (that is, to form a spiro-cycloalkyl group);

or $R^{16}$ together with the first carbon atom of $R^6$ form a double bond wherein $R^6$ is otherwise alkyl, or wherein $R^6$ is otherwise substituted alkyl as defined above, or wherein $R^6$ and $R^5$ are otherwise joined together as defined above (that is, so that $R^5$, $R^6$ and $R^{16}$ together from alkylidene);

wherein Q is carbonyl (—CO—) or methylene (—$CH_2$—);

and wherein either $R^{10}$ is hydroxy, amino, aryloxy, arylthio, alkoxy, cycloalkoxy, alkylamino, dialkylamino, cyclic amino, hydroxyalkoxy, acyloxyalkoxy, aminoalkoxy, alkylaminoalkoxy, dialkylaminoalkoxy, cyclic aminoalkoxy, aminoalkylamino, alkylaminoalkylamino, dialkylaminoalkylamino, cyclic aminoalkylamino or arylalkoxy wherein each alkyl or alkoxy has up to 5 carbon atoms and wherein cyclic amino has up to 6 carbon atoms;

or wherein $R^{10}$ and $R^6$ are joined together such that $R^{10}$ is oxygen (—O—) joined to the terminal carbon atom of $R^6$ when it is alkyl;

wherein $R^{11}$ is hydrogen of alkyl of up to 3 carbon atoms;

wherein X is carbonyl (—CO—), hydroxymethylene (—CHOH—), thiocarbonyl (—CS—) or oximinomethylene (—C=N—$OR^{20}$ wherein $R^{20}$ is hydrogen or alkyl of up to 5 carbon atoms which is unsubstituted or which bears an aryl substituent);

and wherein Y is carbonyl, thiocarbonyl, sulphonyl (—$SO_2$—) or amido (—NHCO—);

or a salt thereof where appropriate, characterised by either:

(a) the reaction of an acid of the formula:—

$R^1—Y—NR^2—CHR^3—X—CHR^{11}—CHR^4—COOH$

wherein $R^1$, $R^2$, $R^3$, $R^4$, $R^{11}$, X and Y have the meanings stated above, or of a reactive derivative thereof, with an amine of the formula:—

$HNR^5—CR^6R^{16}—Q—R^{12}$

wherein $R^5$, $R^6$, $R^{16}$ and Q have the meanings stated above and wherein $R^{12}$ has any of the meanings stated above for $R^{10}$ except those containing a free hydroxy or amino grop; or

(b) for the manufacture of an amide derivative wherein $R^2$ is hydrogen and X is carbonyl or thiocarbonyl, the reaction of an amine of the formula:

$H_2NCHR^3—X^1—CHR^{11}—CHR^4CONR^5—CR^6R^{16}—Q—R^{12}$

wherein $R^3$, $R^4$, $R^5$, $R^6$, $R^{11}$, $R^{12}$, $R^{16}$ and Q have the meanings stated above and $X^1$ is carbonyl or thiocarbonyl, with a compound of the formula $R^1—Y—Z^1$, wherein $R^1$ and Y have the meanings stated above and wherein $Z^1$ is a displaceable halogen, alkanesulphonyl or arensulphonyl group, or $Z^1$ is hydroxy (in which case the reaction is carried out in the presence of a carbodiimide or an alkyl chloroformate), or, when Y is amido, with a compound of the formula $R^1NCO$, or, when Y is thiocarbonyl, with a compound of the formula $R^1CS—SCH_2COOH$; or

(c) for the manufacture of an amide derivative wherein $R^2$ is hydrogen and X is a carbonyl, the reaction of a compound of the formula:—

$Z^2COCHR^{11}CHR^4CONR^5CR^6R^{16}QR^{12}$

wherein $R^4$, $R^5$, $R^6$, $R^{11}$, $R^{12}$, $R^{16}$, and Q have the meanings stated above and $Z^2$ is a displaceable halogen atom, with a compound of the formula

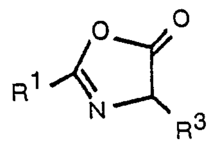

wherein $R^1$ and $R^3$ have the meanings stated above, followed by hydrolysis of the oxazolone ring; whereafter an amide derivative wherein $R^2$ is alkyl, arylalkyl or aryl (and $R^{10}$ is $R^{12}$) may be obtained by reacting the corresponding amide derivative wherein $R^2$ is hydrogen (and $R^{10}$ is $R^{12}$) with a compound of the formula $R^2Z$, wherein $R^2$ has the meaning stated above and Z is a displaceable halogen, alkanesulphonyl or arenesulphonyl group; or whereafter an amide derivative wherein $R^3$ is unsubstituted or substituted alkyl as defined above may be obtained by the hydrogenation of the corresponding compound wherein $R^3$ is unsubstituted or substituted alkenyl as defined above; or whereafter an amide derivative wherein $R^{10}$ is other than hydroxy (that is, wherein $—Q—R^{10}$ forms an ester or amide group) may be obtained from the corresponding acid wherein $—Q—R^{10}$ is $—COOH$ by conventional means of ester or amide formation; or whereafter an amide derivative wherein Q is carbonyl and $R^{10}$ is hydroxy may be obtained by the hydrolysis of the corresponding amide derivative wherein $R^{10}$ is alkoxy, or, when $R^{10}$ is t-butoxy, by the acid-catalysed cleavage of said compound; or whereafter an amide derivative wherein X is hydroxymethylene may be obtained by the reduction of the corresponding amide derivative wherein X is carbonyl; or whereafter an amide derivative wherein X is oximinomethylene may be obtained by the reaction of the corresponding amide derivative wherein X is carbonyl with hydroxylamine or an *O*-substituted hydroxylamine derivative of the formula $H_2NOR^{20}$, wherein $R^{20}$ has the meaning stated above.

2. A process for the manufacture of an amide derivative having the formula:—

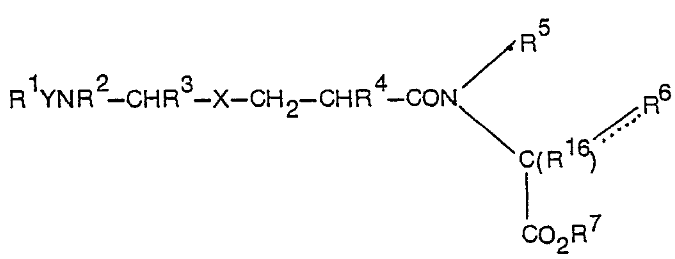

wherein $R^1$ is alkyl of up to 15 carbon atoms which is, unsubstituted or which bears an aryl, aryloxy or arylthio substituent, or $R^1$ is aryl or aryloxy, or $R^1$ is a substituent of the formula $R^8CONHCHR^9—$ or $R^8COCH_2CHR^9—$ wherein $R^8$ is alkyl or cycloalkyl each of up to 10 carbon atoms, or aryl, and $R^9$ is

hydrogen, or alkyl of up to 5 carbon atoms which is unsubstituted or which bears an aryl substituent, or $R^9$ is a group other than those stated above such that the compound $H_2NCHR^9COOH$ would be a naturally occurring amino acid;
wherein Y and $R^2$ have the meanings stated in claim 1;
wherein $R^3$ is alkyl or alkenyl each of up to 5 carbon atoms which is unsubstituted or which bears an aryl substituent; or $R^3$ is aryl or indolylmethyl;
wherein X is carbonyl, hydroxymethylene, thiocarbonyl or oximinomethylene (C=N—OH);
wherein $R^4$ is hydrogen or alkyl of up to 5 carbon atoms which is unsubstituted or which bears an aryl substituent;
wherein either $R^5$ is hydrogen, aryl or alkyl of up to 5 carbon atoms which is unsubstituted or which bears an aryl substituent, $R^6$ is hydrogen, alkyl of up to 5 carbon atoms which is unsubstituted or which bears an aryl or heterocyclic substituent, or $R^6$ is heterocyclic or aryl, and $R^{16}$ is hydrogen;

or $R^5$ and $R^6$ are joined together to form alkylene or alkenylene of 2 to 4 carbon atoms; or an oxa, thia or aza-derivative of said alkylene or alkenylene;

or $R^6$, $R^{16}$ and $R^5$ are joined together such that $R^{16}$ together with the first carbon atom of $R^6$ form a double bond wherein $R^6$ and R are otherwise joined together as defined above;
and wherein $R^7$ is hydrogen or alkyl of up to 5 carbon atoms; or a salt thereof, characterised by the reaction of an acid of the formula:—

$$R^1—Y—NR^2—CHR^3—X—CH_2—CHR^4—COOH$$

wherein $R^1$, $R^2$, $R^3$, $R^4$, X and Y have the meanings stated above, or of a reactive derivative thereof, with an amine of the formula:—

$$\begin{array}{c} \quad R^6 \\ \quad \vdots \\ HNR^5—C(R^{16})—CO_2R^{12} \end{array}$$

wherein $R^5$, $R^6$ and $R^{16}$ have the meanings stated above and wherein $R^{12}$ is alkyl of up to 5 carbon atoms; whereafter an amide derivative wherein $R^2$ is alkyl, arylalkyl or aryl (and $R^7$ is alkyl) may be obtained by reacting the corresponding amide derivative wherein $R^2$ is hydrogen (and $R^7$ is alkyl) with a compound of the formula $R^2Z$, wherein $R^2$ has the meaning stated above and Z is a displaceable halogen, alkanesulphonyl or arenesulphonyl group; or whereafter an amide derivative wherein $R^7$ is hydrogen may be obtained by the hydrolysis of the corresponding amide derivative wherein $R^7$ is alkyl, or, when $R^7$ is t-butyl, by the acid-catalysed cleavage of said compound; or whereafter an amide derivative wherein X is hydroxymethylene may be obtained by the reduction of the corresponding amide derivative wherein X is carbonyl; or whereafter an amide derivative wherein X is oximinomethylene may be obtained by the reaction of the corresponding amide derivative wherein X is carbonyl with hydroxylamine.

3. A process for the manufacture of an amide derivative having the formula:—

$$R^1CONH—CHR^3—COCH_2—CHR^4—CONR^5—CHR^6—COR^{10}$$

wherein $R^1$ is methyl, benzyl, β-phenylethyl, benzyloxy, phenoxymethyl, phenylthiomethyl, phenyl, tolyl, methoxyphenyl, methylthiophenyl, monochlorophenyl, dichlorophenyl, trifluoromethylphenyl, acetamidophenyl, acetylphenyl, methanesulphonylphenyl, biphenylyl, naphthyl, benzamidomethyl, cyclopentanecarbonamidomethyl or β-benzoylethyl;

$R^3$ is benzyl which is unsubstituted or bears a methyl, t-butyl, methoxy, fluoro, chloro, bromo, nitro, cyano, trifluoromethyl, amino, acetamido, methanesulphonamido, trifluoromethanesulphonamido or phenyl substituent, or

$R^3$ is 3-phenylpropyl or 3-phenylprop-2-enyl;

$R^4$ is hydrogen or methyl;

$R^5$ is hydrogen or methyl and $R^6$ is hydrogen, benzyl or indol-3-ylmethyl, or $R^5$ and $R^6$ together form trimethylene, tetramethylene, pentamethylene, 2-hydroxytrimethylene, 2-thiatrimethylene, 3-thiatetramethylene, 3-oxatetramethylene, 3-methyl-3-azatetramethylene; and

$R^{10}$ is hydroxy, amino, alkoxy of up to 5 carbon atoms, or β-dimethylaminoethoxy or β-morpholinoethoxy;
or a salt thereof, characterised by

(a) the reaction of an acid of the formula:—

$$R^1CONH—CHR^3—COCH_2—CHR^4—COOH$$

wherein $R^1$, $R^3$ and $R^4$ have the meanings stated above, or of a reactive derivative thereof, with an amine of the formula:—

$$HNR^5—CHR^6—COR^{12}$$

wherein $R^5$ and $R^6$ have the meanings stated above and wherein $R^{12}$ has any of the meanings stated above for $R^{10}$ except hydroxy and amino; or

(b) the reaction of an amine of the formula:—

$$H_2NCHR^3—COCH_2—CHR^4—CONR^5—CHR^6—COR^{12}$$

wherein $R^3$, $R^4$, $R^5$, $R^6$ and $R^{12}$ have the meanings stated above, with a compound of the formula $R^1—COZ^1$,
wherein $R^1$ has the meaning stated above and wherein $Z^1$ is a displaceable halogen, alkanesulphonyl or arensulphonyl group, or $Z^1$ is hydroxy (in which case the reaction is carried out in the presence of a carbodiimide or an alkyl chloroformate); or

(c) the reaction of a compound of the formula:—

$$Z^2COCH_2CHR^4CONR^5CHR^6COR^{12}$$

wherein $R^4$, $R^5$, $R^6$ and $R^{12}$ have the meanings stated above and $Z^2$ is a displaceable halogen atom, with a compound of the formula:

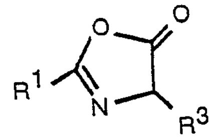

wherein $R^1$ and $R^3$ have the meanings stated above, followed by hydrolysis of the oxazolone ring; whereafter an amide derivative wherein $R^3$ is 3-phenylpropyl may be obtained by the hydrogenation of the corresponding compound wherein $R^3$ is 3-phenylprop-2-enyl; whereafter an amide derivative where $R^{10}$ is hydroxy may be obtained by the hydrolysis of the corresponding amide derivative wherein $R^{10}$ is alkoxy, or, when $R^{10}$ is t-butoxy, by the acid-catalysed cleavage of said compound; and whereafter an amide derivative wherein $R^{10}$ is other than hydroxy (that is, wherein $COR^{10}$ forms an ester or amide group) may be obtained from the corresponding acid wherein $—CO—R^{10}$ is $—COOH$ by conventional means of ester or amide formation.

4. A process as claimed in claim 3 wherein in the starting materials $R^1$ is phenyl, *p*-acetamidophenyl or *p*-cyanophenyl, $R^3$ is benzyl, *p*-methylbenzyl or *p*-methoxybenzyl, $R^4$ is hydrogen or methyl, $R^5$ and $R^6$ are joined together to form trimethylene and $R^{10}$ is hydroxy, amino, methoxy, ethoxy, isopropyl or t-butoxy.

**Patentansprüche für die Vertragsstaaten: BE CH DE FR GB IT LI LU NL SE**

1. Amidderivat der Formel

$$R^1—Y—NR^2—CHR^3—X—CHR^{11}—CHR^4—CO—NR^5—CR^6R^{16}—Q—R^{10}$$

worin $R^1$ entweder für Wasserstoff oder Alkyl mit bis zu 15 Kohlenstoffatomen steht, welches unsubstituiert ist oder welches einen Substituenten trägt, der ausgewählt ist aus Amino-, Benzyloxycarbonylamino-, Hydroxy-, Alkoxy-, Alkylthio- und Alkoxycarbonyl- mit jeweils bis zu 5 Kohlenstoffatomen sowie Aryl-, Aryloxy- und Arylthio-Substituenten, oder welches zwei oder drei Substituenten trägt, von denen einer Aryl ist, von denen ein weiterer Aryl, Hydroxy, Amino, Benzyloxycarbonylamino, Trifluoromethyl, Aryloxy oder Alkoxy mit bis zu 5 Kohlenstoffatomen ist und von denen der dritte, sofern anwesend, Aryl oder Trifluoromethyl ist;

oder $R^1$ für Aryl oder, sofern Y Carbonyl oder Thiocarbonyl bedeutet, für Aryloxy, Alkoxy oder Arylalkoxy steht, wobei der Alkoxyteil bis zu 5 Kohlenstoffatome aufweist;

oder $R^1$ für Halogenoalkyl mit bis zu 6 Kohlenstoffatomen oder Alkenyl, Halogenoalkenyl oder Cycloalkyl mit jeweils bis zu 6 Kohlenstoffatomen steht, welches unsubstituiert ist oder welches einen Arylsubstituenten trägt;

oder $R^1$ für einen Substituenten der Formel $R^8CONHCHR^9—$ oder $R^8COCH_2CHR^9—$ steht, worin $R^8$ Alkyl oder Cycloalkyl mit jeweils bis zu 10 Kohlenstoffatomen oder Aryl bedeutet und $R^9$ Wasserstoff oder Alkyl mit bis zu 5 Kohlenstoffatomen bedeutet, welches unsubstituiert ist oder welches einen Arylsubstituenten trägt, oder $R^9$ eine andere Gruppe als die oben angegebenen bedeutet, so daß die Verbindung $H_2NCHR^9COOH$ eine natürlich vorkommende Aminosäure ist;

worin $R^2$ für Wasserstoff, Alkyl mit bis zu 5 Kohlenstoffatomen, welches unsubstituiert ist oder welches einen Arylsubstituenten trägt, oder Aryl steht;

worin $R^3$ für Alkyl oder Alkenyl mit jeweils bis zu 5 Kohlenstoffatomen steht, welches unsubstituiert ist oder welches einen Arylsubstituenten trägt, oder $R^3$ für Aryl oder Indolylmethyl steht;

worin $R^4$ für Wasserstoff oder Alkyl mit bis zu 5 Kohlenstoffatomen steht, welches unsubstituiert ist oder welches einen Arylsubstituenten trägt;

worin $R^5$ entweder für Wasserstoff oder Aryl oder Alkyl mit bis zu 5 Kohlenstoffatomen, welches unsubstituiert ist oder welches einen Arylsubstituenten trägt, steht oder $R^5$ mit $R^6$ verbunden ist, wie es unten definiert ist;

worin $R^6$ entweder für Wasserstoff, Aryl oder einen Heterozyklus oder Alkyl mit bis zu 5 Kohlenstoffatomen, welches unsubstituiert ist oder welches einen Hydroxy-, Aryl- oder heterocyclischen Substituenten trägt, steht;

oder $R^6$ und $R^5$ miteinander verbunden sind, so daß Alkylen oder Alkenylen mit 2 bis 5 Kohlenstoffatomen oder ein Oxa-, Thia- oder Azaderivat des Alkylens oder Alkenylens oder ein hydroxy- oder oxo-substituiertes Derivat des Alkylens oder Alkenylens gebildet wird;

oder $R^6$ und $R^{16}$ oder $R^6$, $R^{16}$ und $R^5$ und $R^{10}$ miteinander verbunden sind, wie es unten definiert ist;

worin $R^{16}$ für Wasserstoff oder Alkyl mit bis zu 5 Kohlenstoffatomen steht;

oder $R^6$ und $R^{16}$ miteinander verbunden sind, so daß Alkylen mit 2 bis 5 Kohlenstoffatomen gebildet wird (d.h., daß eine Spirocycloalkylgruppe gebildet wird);

oder $R^{16}$ zusammen mit dem ersten Kohlenstoffatom von $R^6$ eine Doppelbindung bildet, wobei $R^6$ ansonsten für Alkyl steht oder wobei $R^6$ ansonsten für substituiertes Alkyl steht, wie es oben definiert ist, oder wobei $R^5$ und $R^6$ ansonsten miteinander verbunden sind, wie es oben definiert ist (d.h., so daß $R^5$, $R^6$ und $R^{16}$ gemeinsam Alkyliden bilden);

worin Q für Carbonyl (—CO—) oder Methylen (—$CH_2$—) steht;

und worin $R^{10}$ entweder für Hydroxy, Amino, Aryloxy, Arylthio, Alkoxy, Cycloalkoxy, Alkylamino, Dialkylamino, cyclisches Amino, Hydroxyalkoxy, Acyloxyalkoxy, Aminoalkoxy, Alkylaminoalkoxy, Dialkylaminoalkoxy, cyclisches Aminoalkoxy, Aminoalkylamino, Alkylaminoalkylamino, Dialkylaminoalkylamino, cyclisches Aminoalkylamino oder Arylalkoxy steht, wobei jedes Alkyl oder Alkoxy bis zu 5 Kohlenstoffatome aufweist und wobei cyclisches Amino bis zu 6 Kohlenstoffatome aufweist;

oder worin $R^{10}$ und $R^6$ miteinander verbunden sind, so daß $R^{10}$ Sauerstoff (—O—) ist, der an das endständige Kohlenstoffatom von $R^6$ gebunden ist, wenn es Alkyl ist;

worin $R^{11}$ für Wasserstoff oder Alkyl mit bis zu 3 Kohlenstoffatomen steht;

worin X für Carbonyl (—CO—), Hydroxymethylen (—CHOH—), Thiocarbonyl (—CS—) oder Oximinomethylen (>C=N—$OR^{20}$, worin $R^{20}$ Wasserstoff oder Alkyl mit bis zu 5 Kohlenstoffatomen ist, welches unsubstituiert ist oder welches einen Arylsubstituenten trägt) steht;

und worin Y für Carbonyl, Thiocarbonyl, Sulfonyl (—$SO_2$—) oder Amido (—NHCO—) steht;

oder ein Salz davon, sofern möglich.

2. Amidderivat nach Anspruch 1, worin X, Y und Q alle für Carbonyl stehen und $R^2$ und $R^{11}$ beide für Wasserstoff stehen, oder ein Salz davon.

3. Amidderivat der Formel

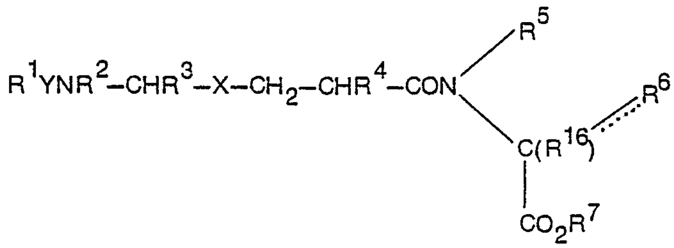

worin $R^1$ für Alkyl mit bis zu 15 Kohlenstoffatomen steht, das unsubstituiert ist oder das einen Aryl-, Aryloxy- oder Arylthiosubstituenten trägt, oder $R^1$ für Aryl oder Aryloxy steht oder $R^1$ für einen Substituenten der Formel $R^8CONHCHR^9$— oder $R^8COCH_2CHR^9$— steht, wobei $R^8$ Alkyl oder Cycloalkyl mit jeweils bis zu 10 Kohlenstoffatomen oder Aryl bedeutet und $R^9$ Wasserstoff oder Alkyl mit bis zu 5 Kohlenstoffatomen bedeutet, welches unsubstituiert ist oder welches einen Arylsubstituenten trägt, oder $R^9$ eine andere Gruppe als die oben angegebenen bedeutet, so daß die Verbindung $H_2NCHR^9COOH$ eine natürlich vorkommende Aminosäure ist;

worin Y und $R^2$ die in Anspruch 1 angegebenen Bedeutungen besitzen;

worin $R^3$ für Alkyl oder Alkenyl mit jeweils bis zu 5 Kohlenstoffatomen steht, welches unsubstituiert ist oder welches einen Arylsubstituenten trägt, oder $R^3$ für Aryl oder Indolylmethyl steht;

worin X für Carbonyl, Hydroxymethylen, Thiocarbonyl oder Oximinomethylen (>C=N—OH) steht;

worin $R^4$ für Wasserstoff oder Alkyl mit bis zu 5 Kohlenstoffatomen steht, welches unsubstituiert ist oder welches einen Arylsubstituenten trägt;

worin $R^5$ entweder für Wasserstoff, Aryl oder Alkyl mit bis zu 5 Kohlenstoffatomen steht, welches unsubstituiert ist oder welches einen Arylsubstituenten trägt, $R^6$ für Wasserstoff, Alkyl mit bis zu 5 Kohlenstoffatomen steht, welches unsubstituiert ist oder welches einen Aryl- oder heterocylischen Substituenten trägt, oder $R^6$ für einen Heterozyklus oder Aryl steht und $R^{16}$ für Wasserstoff steht;

oder $R^5$ und $R^6$ miteinander verbunden sind, so daß Alkylen oder Alkenylen mit 2 bis 4 Kohlenstoffatomen oder ein Oxa-, Thia- oder Azaderivat dieses Alkylens oder Alkenylens gebildet wird;

oder $R^6$, $R^{16}$ und $R^5$ miteinander verbunden sind, so daß $R^{16}$ zusammen mit dem ersten Kohlenstoffatom von $R^6$ eine Doppelbindung bildet, wobei $R^6$ und $R^5$ im übrigen miteinander verbunden sind, wie es oben definiert ist;

und worin $R^7$ für Wasserstoff oder Alkyl mit bis zu 5 Kohlenstoffatomen steht;

oder ein Salz davon.

4. Amidderivat der Formel

$$R^1CONH—CHR^3—COCH_2—CHR^4—CONR^5—CHR^6—COR^{10}$$

worin $R^1$ für Methyl, Benzyl, $\beta$-Phenylethyl, Benzyloxy, Phenoxymethyl, Phenylthiomethyl, Phenyl, Tolyl, Methoxyphenyl, Methylthiophenyl, Monochlorophenyl, Dichlorophenyl, Fluorophenyl, Jodophenyl, Nitrophenyl, Cyanophenyl, Trifluoromethylphenyl, Acetamidophenyl, Acetylphenyl, Methansulfonylphenyl, Biphenylyl, Naphthyl, Benzamidomethyl, Cyclopentancarbonamidomethyl oder $\beta$-Benzoylethyl steht;

$R^3$ für Benzyl steht, welches unsubstituiert ist oder welches einen Methyl-, t-Butyl-, Methoxy-, Fluoro-, Chloro-, Bromo-, Nitro-, Cyano-, Trifluoromethyl-, Amino-, Acetamido-, Methansulfonamido-, Trifluoromethansulfonamido- oder Phenylsubstituenten trägt, oder $R^3$ für 3-Phenylpropyl oder 3-Phenylprop-2-enyl steht;

$R^4$ für Wasserstoff oder Methyl steht;

$R^5$ für Wasserstoff oder Methyl steht und $R^6$ für Wasserstoff, Benzyl oder Indol-3-ylmethyl steht oder $R^5$ und $R^6$ gemeinsam Trimethylen, Tetramethylen, Pentamethylen, 2-Hydroxytrimethylen, 2-Thiatrimethylen, 3-Thiatetramethylen, 3-Oxatetramethylen, 3-Methyl-3-azatetramethylen bilden; und

$R^{10}$ für Hydroxy, Amino, Alkoxy mit bis zu 5 Kohlenstoffatomen oder $\beta$-Dimethylaminoethoxy oder $\beta$-Morpholinoethoxy steht;

oder ein Salz davon.

5. Amidderivat der Formel

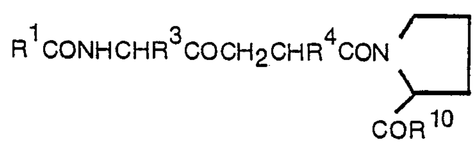

worin $R^1$ für Phenyl, p-Acetamidophenyl oder p-Cyanophenyl steht, $R^3$ für Benzyl, p-Methylbenzyl oder p-Methoxybenzyl steht, $R^4$ für Wasserstoff oder Methyl steht und $R^{10}$ für Hydroxy, Amino, Methoxy, Ethoxy, Isopropoxy oder t-Butoxy steht, oder ein Salz davon.

6. Die Verbindung N-(5-Acetamido-2-methyl-4-oxo-6-phenylhexanoyl-L-prolin, N-(5-Benzamido-2-methyl-4-oxo-6-phenylhexanoyl)-L-prolin oder N-(5-Phenylacetamido-2-methyl-4-oxo-6-phenylhexanoyl)-L-prolin, oder ein Salz davon oder ein Ester davon mit einem Alkohol mit bis zu 5 Kohlenstoffatomen oder ein Amid davon.

7. Die Verbindung N-(5-Benzamido-2-methyl-4-oxo-6-phenylhexanoyl)-L-prolin oder deren Methylester oder deren Amid oder ein Salz davon.

8. Ein Salz eines Amidderivats nach einem der Ansprüche 1 bis 7, worin $R^{10}$ für Hydroxy steht, wobei es sich um ein Alkalimetall- oder Erdalkalimetallsalz oder um ein Ammonium- oder Dicyclohexylaminsalz handelt.

9. Verfahren zur herstellung eines Amidderivats oder eines Salzes davon nach einem der Ansprüche 1 bis 8, bei welchem

(a) eine Säure der Formel

$$R^1—Y—NR^2—CHR^3—X—CHR^{11}—CHR^4—COOH$$

worin $R^1$, $R^2$, $R^3$, $R^4$, $R^{11}$, X und Y die in einem der Ansprüche 1 bis 5 angegebenen Bedeutungen besitzen, oder ein reaktives Derivat davon mit einem Amin der Formel

$$HNR^5—CR^6R^{16}—Q—R^{12}$$

worin $R^5$, $R^6$, $R^{16}$ und Q die in einem der Ansprüche 1 bis 5 angegebenen Bedeutungen besitzen und worin $R^{12}$ eine der in den Ansprüchen 1 bis 5 zu $R^{10}$ angegebene Bedeutung besitzt, mit Ausnahme solcher, die eine freie Hydroxy- oder Aminogruppe aufweisen, umgesetzt wird; oder

(b) zur Herstellung eines Amidderivats, worin $R^2$ für Wasserstoff steht und X für Carbonyl oder Thiocarbonyl steht, ein Amin der Formel

$$H_2NCHR^3—X^1—CHR^{11}CHR^4CONR^5—CR^6R^{16}—Q—R^{12}$$

worin $R^3$, $R^4$, $R^5$, $R^6$, $R^{11}$, $R^{12}$, $R^{16}$ und Q die oben angegebenen Bedeutungen besitzen und worin $X^1$ für

Carbonyl oder Thiocarbonyl steht, mit einer Verbindung der Formel $R^1—Y—Z^1$, worin $R^1$ und Y die oben angegebenen Bedeutungen besitzen und worin $Z^1$ für eine ersetzbare Halogen-, Alkansulfonyl- oder Arensulfonylgruppe steht oder $Z^1$ für Hydroxy steht (in welchem Fall die Reaktion in Gegenwart eines Carbodiimids oder eines Alkylchloroformiats ausgeführt wird), oder, wenn Y Amido bedeutet, mit einer Verbindung der Formel $R^1NCO$ oder, wenn Y Thiocarbonyl bedeutet, mit einer Verbindung der Formel $R^1CS—SCH_2COOH$ umgesetzt wird; oder

(c) zur herstellung eines Amidderivats, worin $R^2$ für Wasserstoff steht und X für Carbonyl steht, eine Verbindung der Formel

$$Z^2COCHR^{11}CHR^4CONR^5CR^6R^{16}QR^{12}$$

worin $R^4$, $R^5$, $R^6$, $R^{11}$, $R^{12}$, $R^{16}$ und Q die oben angegebenen Bedeutungen besitzen und $Z^2$ für ein ersetzbares Halogenatom steht, mit einer Verbindung der Formel

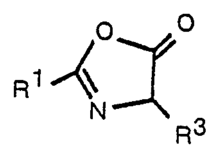

worin $R^1$ und $R^3$ die oben angegebenen Bedeutungen besitzen, umgesetzt wird, worauf der Oxazolonring hydrolysiert wird;

worauf ein Amidderivat, worin $R^2$ für Alkyl, Arylalkyl oder Aryl steht (und $R^{10}$ für $R^{12}$ steht) durch Umsetzung des entsprechenden Amidderivats, worin $R^2$ für Wasserstoff steht (unt $R^{10}$ für $R^{12}$ steht) mit einer Verbindung der Formel $R^2Z$, worin $R^2$ die oben angegebene Bedeutung besitzt und Z für eine ersetzbare Halogen-, Alkansulfonyl- oder Arensulfonylgruppe steht, erhalten werden kann;

oder worauf ein Amidderivat, worin $R^3$ für unsubstituiertes oder substituiertes Alkyl steht, wie es oben definiert ist, durch Hydrierung der entsprechenden Verbindung, worin $R^3$ für unsubstituiertes oder substituiertes Alkenyl steht, wie es oben definiert ist, erhalten werden kann;

oder worauf ein Amidderivat, worin $R^{10}$ für etwas anderes als Hydroxy steht (d.h. worin $—Q—R^{10}$ eine Ester- oder Amidgruppe bildet) aus der entsprechenden Säure, worin $—Q—R^{10}$ für $—COOH$ steht, durch herkömmliche Maßnahmen der ester- oder Amidbildung erhalten werden kann;

oder worauf ein Amidderivat, worin Q für Carbonyl steht und $R^{10}$ für Hydroxy steht, durch Hydrolyse des entsprechenden Amidderivats, worin $R^{10}$ für Alkoxy steht, oder, wenn $R^{10}$ für t-Butoxy steht, durch die säurekatalysierte Spaltung dieser Verbindung erhalten werden kann;

oder worauf ein Amidderivat, worin X für Hydroxymethylen steht, durch Reduktion des entsprechenden Amidderivats, worin X für Carbonyl steht, erhalten werden kann;

oder worauf ein Amidderivat, worin X für Oximinomethylen steht, durch Umsetzung des entsprechenden Amidderivats, worin X für Carbonyl steht, mit Hydroxylamin oder einem O-substituierten Hydroxylaminderivat der Formel $H_2NOR^{20}$, worin $R^{20}$ die in Anspruch 1 angegebene Bedeutung besitzt, erhalten werden kann.

10. Pharmazeutische Zusammensetzung, welche als aktiven Bestandteil mindestens ein Amidderivat oder ein Salz davon nach einem der Ansprüche 1 bis 8 gemeinsam mit einem pharmazeutisch zulässigen Verdünnungsmittel oder Trägermittel hierfür enthält.

**Patentansprüche für den Vertragsstaat: AT**

1. Verfahren zur Herstellung eines Amidderivats der Formel

$$R^1—Y—NR^2—CHR^3—X—CHR^{11}—CHR^4—CO—NR^5—CR^6R^{16}—Q—R^{10}$$

worin $R^1$ entweder für Wasserstoff oder Alkyl mit bis zu 15 Kohlenstoffatomen steht, welches unsubstituiert ist oder welches einen Substituenten trägt, der ausgewählt ist aus Amino-, Benzyloxycarbonylamino-, Hydroxy-, Alkoxy-, Alkylthio- und Alkoxycarbonyl- mit jeweils bis zu 5 Kohlenstoffatomen sowie Aryl-, Aryloxy- und Arylthio-Substituenten, oder welches zwei oder drei Substituenten trägt, von denen einer Aryl ist, von denen ein weiterer Aryl, Hydroxy, Amino, Benzyloxycarbonylamino, Trifluoromethyl, Aryloxy oder Alkoxy mit bis zu 5 Kohlenstoffatomen ist und von denen der dritte, sofern anwesend, Aryl oder Trifluoromethyl ist;

oder $R^1$ für Aryl oder, sofern Y Carbonyl oder Thiocarbonyl bedeutet, für Aryloxy, Alkoxy oder Arylalkoxy steht, wobei der Alkoxyteil bis zu 5 Kohlenstoffatome aufweist;

oder $R^1$ für Halogenoalkyl mit bis zu 6 Kohlenstoffatomen oder Alkenyl, Halogenoalkenyl oder Cycloalkyl mit jeweils bis zu 6 Kohlenstoffatomen steht, welches unsubstituiert ist oder welches einen Arylsubstituenten trägt;

oder $R^1$ für einen Substituenten der Formel $R^8CONHCHR^9—$ oder $R^8COCH_2CHR^9—$ steht, worin $R^8$

Alkyl oder Cycloalkyl mit jeweils bis zu 10 Kohlenstoffatomen oder Aryl bedeutet und $R^9$ Wasserstoff oder Alkyl mit bis zu 5 Kohlenstoffatomen bedeutet, welches unsubstituiert ist oder welches einen Arylsubstituenten trägt, oder $R^9$ eine andere Gruppe als die oben angegebenen bedeutet, so daß die Verbindung $H_2NCHR^9COOH$ eine natürlich vorkommende Aminosäure ist;

worin $R^2$ für Wasserstoff, Alkyl mit bis zu 5 Kohlenstoffatomen, welches unsubstituiert ist oder welches einen Arylsubstituenten trägt, oder Aryl steht;

worin $R^3$ für Alkyl oder Alkenyl mit jeweils bis zu 5 Kohlenstoffatomen steht, welches unsubstituiert ist oder welches einen Arylsubstituenten trägt, oder $R^3$ für Aryl oder Indolylmethyl steht;

worin $R^4$ für Wasserstoff oder Alkyl mit bis zu 5 Kohlenstoffatomen steht, welches unsubstituiert ist oder welches einen Arylsubstituenten trägt;

worin $R^5$ entweder für Wasserstoff oder Aryl oder Alkyl mit bis zu 5 Kohlenstoffatomen, welches unsubstituiert ist oder welches einen Arylsubstituenten trägt, steht oder $R^5$ mit $R^6$ verbunden ist, wie es unten definiert ist;

worin $R^6$ entweder für Wasserstoff, Aryl oder einen Heterozyklus oder Alkyl mit bis zu 5 Kohlenstoffatomen, welches unsubstituiert ist oder welches einen Hydroxy-, Aryl- oder heterocyclischen Substituenten trägt, steht;

oder $R^6$ und $R^5$ miteinander verbunden sind, so daß Alkylen oder Alkenylen mit 2 bis 5 Kohlenstoffatomen oder ein Oxa-, Thia- oder Azaderivat des Alkylens oder Alkenylens oder ein hydroxy- oder oxo-substituiertes Derivat des Alkylens oder Alkenylens gebildet wird;

oder $R^6$ und $R^{16}$ oder $R^6$, $R^{16}$ und $R^5$ und $R^{10}$ miteinander verbunden sind, wie es unten definiert ist;

worin $R^{16}$ für Wasserstoff oder Alkyl mit bis zu 5 Kohlenstoffatomen steht;

oder $R^6$ und $R^{16}$ miteinander verbunden sind, so daß Alkylen mit 2 bis 5 Kohlenstoffatomen gebildet wird (d.h., daß eine Spirocycloalkylgruppe gebildet wird);

oder $R^{16}$ zusammen mit dem ersten Kohlenstoffatom von $R^6$ eine Doppelbindung bildet, wobei $R^6$ ansonsten für Alkyl steht oder wobei $R^6$ ansonsten für substituiertes Alkyl steht, wie es oben definiert ist, oder wobei $R^5$ und $R^6$ ansonsten miteinander verbunden sind, wie es oben definiert ist (d.h., so daß $R^5$, $R^6$ und $R^{16}$ gemeinsam Alkyliden bilden);

worin Q für Carbonyl (—CO—) oder Methylen (—$CH_2$—) steht;

und worin $R^{10}$ entweder für Hydroxy, Amino, Aryloxy, Arylthio, Alkoxy, Cycloalkoxy, Alkylamino, Dialkylamino, cyclisches Amino, Hydroxyalkoxy, Acyloxyalkoxy, Aminoalkoxy, Alkylaminoalkoxy, Dialkylaminoalkoxy, cyclisches Aminoalkoxy, Aminoalkylamino, Alkylaminoalkylamino, Dialkylaminoalkylamino, cyclisches Aminoalkylamino oder Arylalkoxy steht, wobei jedes Alkyl oder Alkoxy bis zu 5 Kohlenstoffatome aufweist und wobei cyclisches Amino bis zu 6 Kohlenstoffatome aufweist;

oder worin $R^{10}$ und $R^6$ miteinander verbunden sind, so daß $R^{10}$ Sauerstoff (—O—) ist, der an das endständige Kohlenstoffatom von $R^6$ gebunden ist, wenn es Alkyl ist;

worin $R^{11}$ für Wasserstoff oder Alkyl mit bis zu 3 Kohlenstoffatomen steht;

worin X für Carbonyl (—CO—), Hydroxymethylen (—CHOH—), Thiocarbonyl (—CS—) oder Oximinomethylen ($>C{=}N{-}OR^{20}$, worin $R^{20}$ Wasserstoff oder Alkyl mit bis zu 5 Kohlenstoffatomen ist, welches unsubstituiert ist oder welches einen Arylsubstituenten trägt) steht;

und worin Y für Carbonyl, Thiocarbonyl, Sulfonyl (—$SO_2$—) oder Amido (—NHCO—) steht;

oder eines Salzes davon, sofern möglich,

dadurch gekennzeichnet, daß entweder

(a) eine Säure der Formel

$$R^1{-}Y{-}NR^2{-}CHR^3{-}X{-}CHR^{11}{-}CHR^4{-}COOH$$

worin $R^1$, $R^2$, $R^3$, $R^4$, $R^{11}$, X und Y die oben angegebenen Bedeutungen besitzen, oder ein reaktives Derivat davon mit einem Amin der Formel

$$HNR^5{-}CR^6R^{16}{-}Q{-}R^{12}$$

worin $R^5$, $R^6$, $R^{16}$ und Q die oben angegebenen Bedeutungen besitzen und worin $R^{12}$ eine der oben zu $R^{10}$ angegebenen Bedeutung besitzt, mit Ausnahme solcher, die eine freie Hydroxy- oder Aminogruppe aufweisen, umgesetzt wird; oder

(b) zur Herstellung eines Amidderivats, worin $R^2$ für Wasserstoff steht und X für Carbonyl oder Thiocarbonyl steht, ein Amin der Formel

$$H_2NCHR^3{-}X^1{-}CHR^{11}CHR^4CONR^5{-}CR^6R^{16}{-}Q{-}R^{12}$$

worin $R^3$, $R^4$, $R^5$, $R^6$, $R^{11}$, $R^{12}$, $R^{16}$ und Q die oben angegebenen Bedeutungen besitzen und worin $X^1$ für Carbonyl oder Thiocarbonyl steht, mit einer Verbindung der Formel $R^1{-}Y{-}Z^1$, worin $R^1$ und Y die oben angegebenen Bedeutungen bestizen und worin $Z^1$ für eine ersetzbare Halogen-, Alkansulfonyl- oder Arensulfonylgruppe steht oder $Z^1$ für Hydroxy steht (in welchem Fall die Reaktion in Gegenwart eines Carbodiimids oder eines Alkylchloroformiats ausgeführt wird), oder, wenn Y Amido bedeutet, mit einer

Verbindung der Formel $R^1NCO$ oder, wenn Y Thiocarbonyl bedeutet, mit einer Verbindung der Formel $R^1CS—SCH_2COOH$ umgesetzt wird; oder

(c) zur herstellung eines Amidderivats, worin $R^2$ für Wasserstoff steht und X für Carbonyl steht, eine Verbindung der Formel

$$Z^2COCHR^{11}CHR^4CONR^5CR^6R^{16}QR^{12}$$

worin $R^4$, $R^5$, $R^6$, $R^{11}$, $R^{12}$, $R^{16}$ und Q die oben angegebenen Bedeutungen besitzen und $Z^2$ für ein ersetzbares Halogenatom steht, mit einer Verbindung der Formel

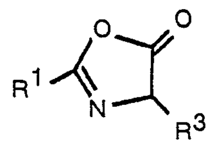

worin $R^1$ und $R^3$ die oben angegebenen Bedeutungen besitzen, umgesetzt wird, worauf der Oxazolonring hydrolysiert wird;

worauf ein Amidderivat, worin $R^2$ für Alkyl, Arylalkyl oder Aryl steht (und $R^{10}$ für $R^{12}$ steht) durch Umsetzung des entsprechenden Amidderivats, worin $R^2$ für Wasserstoff steht (und $R^{10}$ für $R^{12}$ steht) mit einer Verbindung der Formel $R^2Z$, worin $R^2$ die oben angegebene Bedeutung besitzt und Z für eine ersetzbare Halogen-, Alkansulfonyl- oder Arensulfonylgruppe steht, erhalten werden kann;

oder worauf ein Amidderivat, worin $R^3$ für unsubstituiertes oder substituiertes Alkyl steht, wie es oben definiert ist, durch Hydrierung der entsprechenden Verbindung, worin $R^3$ für unsubstituiertes oder substituiertes Alkenyl steht, wie es oben definiert ist, erhalten werden kann;

oder worauf ein Amidderivat, worin $R^{10}$ für etwas anderes als Hydroxy steht (d.h. worin $—Q—R^{10}$ eine Ester- oder Amidgruppe bildet) aus der entsprechenden Säure, worin $—Q—R^{10}$ für $—COOH$ steht, durch herkömmliche Maßnahmen der ester- oder Amidbildung erhalten werden kann;

oder worauf ein Amidderivat, worin Q für Carbonyl steht und $R^{10}$ für Hydroxy steht, durch Hydrolyse des entsprechenden Amidderivats, worin $R^{10}$ für Alkoxy steht, oder, wenn $R^{10}$ für t-Butoxy steht, durch die säurekatalysierte Spaltung dieser Verbindung erhalten werden kann;

oder worauf ein Amidderivat, worin X für Hydroxymethylen steht, durch Reduktion des entsprechenden Amidderivats, worin X für Carbonyl steht, erhalten werden kann;

oder worauf ein Amidderivat, worin X für Oximinomethylen steht, durch Umsetzung des entsprechenden Amidderivats, worin X für Carbonyl steht, mit Hydroxylamin oder einem O-substituierten Hydroxylaminderivat der Formel $H_2NOR^{20}$, worin $R^{20}$ die oben angegebene Bedeutung besitzt, erhalten werden kann.

2. Verfahren zur Herstellung eines Amidderivats der Formel

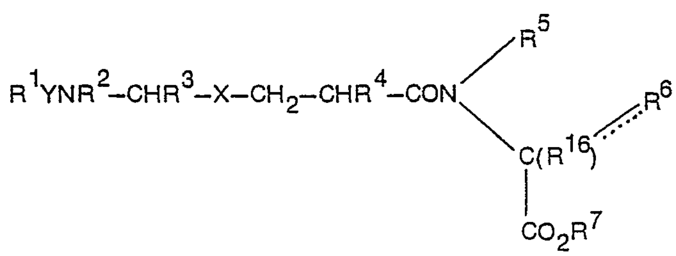

worin $R^1$ für Alkyl mit bis zu 15 Kohlenstoffatomen steht, das unsubstituiert ist oder das einen Aryl-, Aryloxy- oder Arylthiosubstituenten trägt, oder $R^1$ für Aryl oder Aryloxy steht oder $R^1$ für einen Substituenten der Formel $R^8CONHCHR^9—$ oder $R^8COCH_2CHR^9—$ steht, wobei $R^8$ Alkyl oder Cycloalkyl mit jeweils bis zu 10 Kohlenstoffatomen oder Aryl bedeutet und $R^9$ Wasserstoff oder Alkyl mit bis zu 5 Kohlenstoffatomen bedeutet, welches unsubstituiert ist oder welches einen Arylsubstituenten trägt, oder $R^9$ eine andere Gruppe als die oben angegebenen bedeutet, so daß die Verbindung $H_2NCHR^9COOH$ eine natürlich vorkommende Aminosäure ist;

worin Y und $R^2$ die in Anspruch 1 angegebenen Bedeutungen besitzen;

worin $R^3$ für Alkyl oder Alkenyl mit jeweils bis zu 5 Kohlenstoffatomen steht, welches unsubstituiert ist oder welches einen Arylsubstituenten trägt, oder $R^3$ für Aryl oder Indolylmethyl steht;

worin X für Carbonyl, Hydroxymethylen, Thiocarbonyl oder Oximinomethylen ($>C=C—OH$) steht;

worin $R^4$ für Wasserstoff oder Alkyl mit bis zu 5 Kohlenstoffatomen steht, welches unsubstituiert ist oder welches einen Arylsubstituenten trägt;

worin $R^5$ entweder für Wasserstoff, Aryl oder Alkyl mit bis zu 5 Kohlenstoffatomen steht, welches unsubstituiert ist oder welches einen Arylsubstituenten trägt, $R^6$ für Wasserstoff, Alkyl mit bis zu 5

Kohlenstoffatomen steht, welches unsubstituiert ist oder welches einen Aryl- oder heterocylischen Substituenten trägt, oder $R^6$ für einen Heterozyklus oder Aryl steht und $R^{16}$ für Wasserstoff steht;

oder $R^5$ und $R^6$ miteinander verbunden sind, so daß Alkylen oder Alkenylen mit 2 bis 4 Kohlenstoffatomen oder ein Oxa-, Thia- oder Azaderivat dieses Alkylens oder Alkenylens gebildet wird;

oder $R^6$, $R^{16}$ und $R^5$ miteinander verbunden sind, so daß $R^{16}$ zusammen mit dem ersten Kohlenstoffatom von $R^6$ eine Doppelbindung bildet, wobei $R^6$ und $R^5$ im übrigen miteinander verbunden sind, wie es oben definiert ist;

und worin $R^7$ für Wasserstoff oder Alkyl mit bis zu 5 Kohlenstoffatomen steht;

oder eines Salzes davon,

dadurch gekennzeichnet, daß eine Säure der Formel

$$R^1—Y—NR^2—CHR^3—X—CH_2—CHR^4—COOH$$

worin $R^1$, $R^2$, $R^3$, $R^4$, X und Y die oben angegebenen Bedeutungen besitzen, oder ein reaktives Derivat davon mit einem Amin der Formel

$$HNR^5—\overset{R^6}{\overset{\vdots}{C}}(R^{16})—CO_2R^{12}$$

worin $R^5$, $R^6$ und $R^{16}$ die oben angegebenen Bedeutungen besitzen und worin $R^{12}$ für Alkyl mit bis zu 5 Kohlenstoffatomen steht, umgesetzt wird;

worauf ein Amidderivat, worin $R^2$ für Alkyl, Arylalkyl oder Aryl steht (und $R^7$ für Alkyl steht) durch Umsetzung des entsprechenden Amidderivats, worin $R^2$ für Wasserstoff steht (und $R^7$ für Alkyl steht) mit einer Verbindung der Formel $R^2Z$, worin $R^2$ die oben angegebene Bedeutung besitzt und Z für eine ersetzbare Halogen-, Alkansulfonyl- oder Arensulfonylgruppe steht, erhalten werden kann;

oder worauf ein Amidderivat, worin $R^7$ für Wasserstoff steht, durch Hydrolyse des entsprechenden Amidderivates, worin $R^7$ für Alkyl steht, oder, wenn $R^7$ für t-Butyl steht, durch die säurekatalysierte Spaltung dieser Verbindung erhalten werden kann;

oder worauf ein Amidderivat, worin X für Hydroxymethylen steht, durch Reduktion des entsprechenden Amidderivats, worin X für Carbonyl steht, erhalten werden kann;

oder worauf ein Amidderivat, worin X für Oximinomethylen steht, durch Umsetzung des entsprechenden Amidderivats, worin X für Carbonyl steht, mit Hydroxylamin erhalten werden kann.

3. Verfahren zur Herstellung eines Amidderivats der Formel

$$R^1CONH—CHR^3—COCH_2—CHR^4—CONR^5—CHR^6—COR^{10}$$

worin $R^1$ für Methyl, Benzyl, $\beta$-Phenylethyl, Benzyloxy, Phenoxymethyl, Phenylthiomethyl, Phenyl, Tolyl, Methoxyphenyl, Methylthiophenyl, Monochlorophenyl, Dichlorophenyl, Fluorophenyl, Jodophenyl, Nitrophenyl, Cyanophenyl, Trifluoromethylphenyl, Acetamidophenyl, Acetylphenyl, Methansulfonylphenyl, Biphenylyl, Naphthyl, Benzamidomethyl, Cyclopentancarbonamidomethyl oder $\beta$-Benzoylethyl steht;

$R^3$ für Benzyl steht, welches unsubstituiert ist oder welches einen Methyl-, t-Butyl-, Methoxy-, Fluoro-, Chloro-, Bromo-, Nitro-, Cyano-, Trifluoromethyl-, Amino-, Acetamido-, Methansulfonamido-, Trifluoromethansulfonamido- oder Phenylsubstituenten trägt, oder $R^3$ für 3-Phenylpropyl oder 3-Phenylprop-2-enyl steht;

$R^4$ für Wasserstoff oder Methyl steht;

$R^5$ für Wasserstoff oder Methyl steht und $R^6$ für Wasserstoff, Benzyl oder Indol-3-ylmethyl steht oder $R^5$ und $R^6$ gemeinsam Trimethylen, Tetramethylen, Pentamethylen, 2-Hydroxytrimethylen, 2-Thiatrimethylen, 3-Thiatetramethylen, 3-Oxatetramethylen, 3-Methyl-3-axatetramethylen bilden; und

$R^{10}$ für Hydroxy, Amino, Alkoxy mit bis zu 5 Kohlenstoffatomen oder $\beta$-Dimethylaminoethoxy oder $\beta$-Morpholinoethoxy steht;

oder eines Salzes davon,

dadurch gekennzeichnet, daß

(a) eine Säure der Formel

$$R^1CONH—CHR^3—COCH_2—CHR^4—COOH$$

worin $R^1$, $R^3$ und $R^4$ die oben angegebenen Bedeutungen besitzen, oder ein reaktives Derivat davon mit einem Amin der Formel

$$HNR^5—CHR^6—COR^{12}$$

worin $R^5$ und $R^6$ die oben angegebenen Bedeutungen besitzen und worin $R^{12}$ eine der oben für $R^{10}$ angegebene Bedeutung besitzt, außer Hydroxy und Amino, umgesetzt wird oder

(b) ein Amin der Formel

$$H_2NCHR^3—COCH_2—CHR^4—CONR^5—CHR^6—COR^{12}$$

worin $R^3$, $R^4$, $R^5$, $R^6$ und $R^{12}$ die oben angegebenen Bedeutungen besitzen, mit einer Verbindung der Formel $R^1—COZ^1$, worin $R^1$ die oben angegebene Bedeutung besitzt und worin $Z^1$ für eine ersetzbare Halogen-, Alkansulfonyl- oder Arensulfonylgruppe steht oder $Z^1$ für Hydroxy steht (in welchem Fall die Umsetzung in Gegenwart eines Carbodiimids oder eines Alkylchloroformiats ausgeführt wird) umgesetzt wird; oder

(c) eine Verbindung der Formel

$$Z^2COCH_2CHR^4CONR^5CHR^6COR^{12}$$

worin $R^4$, $R^5$, $R^6$ und $R^{12}$ die oben angegebenen Bedeutungen besitzen und $Z^2$ für ein ersetzbares Halogenatom steht, mit einer Verbindung der Formel

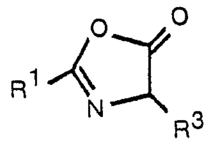

worin $R^1$ und $R^3$ die oben angegebenen Bedeutungen besitzen, umgesetzt wird, worauf der Oxazolonring hydrolysiert wird;

worauf ein Amidderivat, worin $R^3$ für 3-Phenylpropyl steht, durch die Hydrierung der entsprechenden Verbindung, worin $R^3$ für 3-Phenylprop-2-enyl steht, erhalten werden kann;

worauf ein Amidderivat, worin $R^{10}$ für Hydroxy steht, durch die Hydrolyse des entsprechenden Amidderivats, worin $R^{10}$ für Alkoxy steht, oder, wenn $R^{10}$ für t-Butoxy steht, durch die säurekatalysierte Spaltung dieser Verbindung erhalten werden kann;

und worauf ein Amidderivat, worin $R^{10}$ für etwas anderes als Hydroxy steht (d.h., worin $COR^{10}$ eine Ester- oder Amidgruppe bildet) aus der entsprechenden Säure, worin —CO—$R^{10}$ für —COOH steht, durch herkömmliche Maßnahmen der Ester- oder Amidbildung erhalten werden kann.

4. Verfahren nach Anspruch 3, worin in den Ausgangsmaterialien $R^1$ für Phenyl, p-Acetamidophenyl oder p-Cyanophenyl steht, $R^3$ für Benzyl, p-Methylbenzyl oder p-Methoxybenzyl steht, $R^4$ für Wasserstoff oder Methyl steht, $R^5$ und $R^6$ unter Bildung von Trimethylen miteinander verbunden sind und $R^{10}$ für Hydroxy, Amino, Methoxy, Ethoxy, Isopropoxy oder t-Butoxy steht.

**Revendications pour les Etats Contractants: BE CH DE FR GB IT LI LU NL SE**

1. Dérivé amidique de formule:

$$R^1—Y—NR^2—CHR^3—X—CHR^{11}—CHR^4—CO—NR^5—CR^6R^{16}—Q—R^{10}$$

dans laquelle $R^1$ est l'hydrogène ou un groupe alkyle ayant jusqu'à 15 atomes de carbone, qui n'est pas substitué ou qui porte un substituant choisi entre des substituants amino, benzyloxycarbonylamino, hydroxy, alkoxy, alkylthio et alkoxycarbonyle ayant chacun jusqu'à 5 atomes de carbone, et des substituants aryle, aryloxy et arylthio; ou qui porte deux ou trois substituants dont l'un est un substituant aryle, un autre est un substituant aryle, hydroxy, amino, benzyloxycarbonylamino, trifluorométhyle, aryloxy ou alkoxy ayant jusqu'à 5 atomes de carbone et dont le troisième, s'il est présent, est un substituant aryle ou trifluorométhyle;

ou bien $R^1$ est un groupe aryle ou, lorsque Y est un groupe carbonyle ou thiocarbonyle, $R^1$ est un groupe aryloxy, alkoxy ou arylalkoxy dont la portion alkoxy a jusqu'à 5 atomes de carbone;

ou bien $R^1$ est un groupe halogénalkyle ayant jusqu'à 6 atomes de carbone ou un groupe alcényle, halogénalcényle ou cycloalkyle, chacun ayant jusqu'à 6 atomes de carbone et n'étant pas substitué ou portant un substituant aryle;

ou bien $R^1$ est un substituant de formule $R^8CONHCHR^9$— ou $R^8COCH_2CHR^9$— où $R^8$ est un groupe alkyle ou cycloalkyle, chacun ayant jusqu'à 10 atomes de carbone, ou un groupe aryle et $R^9$ est l'hydrogène, ou un groupe alkyle ayant jusqu'à 5 atomes de carbone, qui n'est pas substitué ou qui porte un substituant aryle, ou bien $R^9$ est un groupe autre que ceux qui sont mentionnés ci-dessus, de manière que le composé $H_2NCHR^9COOH$ soit un amino-acide naturel; $R^2$ est l'hydrogène, un groupe alkyle ayant jusqu'à 5 atomes de carbone qui n'est pas substitué ou qui porte un substituant aryle, ou un groupe aryle;

$R^3$ est un groupe alkyle ou un groupe alcényle, chacun ayant jusqu'à 5 atomes de carbone, qui n'est pas substitué ou qui porte un substituant aryle, ou bien $R^3$ est un groupe aryle ou indolylméthyle;

$R^4$ est l'hydrogène ou un groupe alkyle ayant jusqu'à 5 atomes de carbone, qui n'est pas substitué ou qui porte un substituant aryle; $R^5$ est l'hydrogène ou un groupe aryle, ou un groupe alkyle ayant

jusqu'à 5 atomes de carbone qui n'est pas substitué ou qui porte un substituant aryle, ou bien $R^5$ s'associe avec $R^6$ comme défini ci-dessous;

$R^6$ est l'hydrogène, un groupe aryle ou un groupe hétérocyclique, ou un groupe alkyle ayant jusqu'à 5 atomes de carbone qui n'est pas substitué ou qui porte un substituant hydroxy, aryle ou hétérocyclique;

ou bien $R^6$ et $R^5$ forment conjointement un groupe alkylène ou alcénylène de 2 à 5 atomes de carbone; ou un dérivé oxa, thia ou aza dudit alkylène ou alcénylène; ou un dérivé à substituant hydroxy ou oxo dudit alkylène ou alcénylène; ou bien $R^6$ et $R^{16}$, ou $R^6$, $R^{16}$ et $R^5$, ou $R^6$ et $R^{10}$ s'associent de la manière définie ci-dessous;

$R^{16}$ est l'hydrogène ou un groupe alkyle ayant jusqu'à 5 atomes de carbone;

ou bien $R^6$ et $R^{16}$ s'associent pour former un groupe alkylène ayant 2 à 5 atomes de carbone (c'est-à-dire pour former un groupe spirocycloalkyle);

ou bien $R^{16}$ forme conjointement avec le premier atome de carbone de $R^6$ une double liaison, $R^6$ désignant autre chose qu'un groupe alkyle, ou bien $R^6$ est un groupe alkyle autrement substitué comme défini ci-dessus, ou bien $R^6$ et $R^5$ s'associent d'une autre façon comme défini ci-dessus (c'est-à-dire de manière que $R^5$, $R^6$ et $R^{16}$ forment conjointement un groupe alkylidène);

Q est un groupe carbonyle (—CO—) ou méthylène ($—CH_2—$);

et $R^{10}$ est un groupe hydroxy, amino, aryloxy, arylthio, alkoxy, cycloalkoxy, alkylamino, dialkylamino, amino cyclique, hydroxyalkoxy, acyloxyalkoxy, aminoalkoxy, alkylaminoalkoxy, dialkylaminoalkoxy, aminoalkoxy cyclique, aminoalkylamino, alkylaminoalkylamino, dialkylaminoalkylamino, aminoalkylamino cyclique ou arylalkoxy, chaque radical alkyle ou alkoxy ayant jusqu'à 5 atomes de carbone et le groupe amino cyclique ayant jusqu'à 6 atomes de carbone;

ou bien $R^{10}$ et $R^6$ s'associent de façon telle que $R^{10}$ est un atome d'oxygène (—O—) lié à l'atome terminal de carbone de $R^6$ lorsque c'est un groupe alkyle;

$R^{11}$ est l'hydrogène ou un groupe alkyle ayant jusqu'à 3 atomes de carbone;

X est un groupe carbonyle (—CO—), hydroxyméthylène (—CHOH—), thiocarbonyle (—CS—) ou oximinométhylène ($—C{=}N—OR^{20}$) où $R^{20}$ est l'hydrogène ou un radical alkyle ayant jusqu'à 5 atomes de carbone, qui n'est pas substitué ou qui porte un substituant aryle);

et Y est un groupe carbonyle, thiocarbonyle, sulfonyle ($—SO_2—$) ou amido (—NHCO—);

ou un sel de ce dérivé dans les cas appropriés.

2. Dérivé amidique suivant la revendication 1, dans lequel X, Y et Q sont tous des groupes carbonyle et $R^2$ et $R^{11}$ sont tous deux de l'hydrogène, ou un sel de ce dérivé.

3. Dérivé amidique de formule:

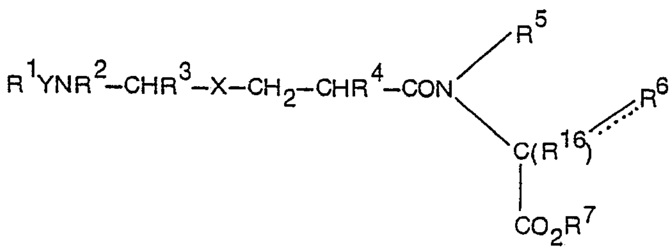

dans laquelle $R^1$ est un groupe alkyle ayant jusqu'à 15 atomes de carbone, qui n'est pas substitué ou qui porte un substituant aryle, aryloxy ou arylthio, ou bien $R^1$ est un groupe aryle ou aryloxy, ou bien $R^1$ est un substituant de formule $R^8CONHCHR^9—$ ou $R^8COCH_2CHR^9—$ dans laquelle $R^8$ est un radical alkyle ou cycloalkyle, chacun ayant jusqu'à 10 atomes de carbone, ou un substituant aryle, et $R^9$ est l'hydrogène ou un groupe alkyle ayant jusqu'à 5 atomes de carbone, qui n'est pas substitué ou qui porte un substituant aryle, ou bien $R^9$ est un groupe autre que les groupes indiqués ci-dessus, de telle sorte que le composé $H_2NCHR^9COOH$ soit un amino-acide naturel;

Y et $R^2$ ont les définitions indiquées dans la revendication 1; $R^3$ est un groupe alkyle ou alcényle, chacun ayant jusqu'à 5 atomes de carbone, qui n'est pas substitué ou qui porte un substituant aryle, ou bien $R^3$ est un groupe aryle ou indolylméthyle; X est un groupe carbonyle, hydroxyméthylène, thiocarbonyle ou oximinométhylène ($>C{=}N—OH$);

$R^4$ est l'hydrogène ou un groupe alkyle ayant jusqu'à 5 atomes de carbone, qui n'est pas substitué ou qui porte un substituant aryle; $R^5$ est l'hydrogène, un groupe aryle ou un groupe alkyle ayant jusqu'à 5 atomes de carbone, qui n'est pas substitué ou qui porte un substituant aryle, $R^6$ est l'hydrogène, un groupe alkyle ayant jusqu'à 5 atomes de carbone qui n'est pas substitué ou qui porte un substituant aryle ou hétérocyclique, ou bien $R^6$ est un substituant hétérocyclique ou aryle et $R^{16}$ est l'hydrogène;

ou bien $R^5$ et $R^6$ forment conjointement un groupe alkylène ou alcénylène ayant 2 à 4 atomes de carbone; ou un dérivé oxa, thia ou aza dudit alkylène ou alcénylène;

ou bien $R^6$, $R^{16}$ et $R^5$ s'associent de manière que $R^{16}$ forme avec le premier atome de carbone de $R^6$ une double liaison, $R^6$ et $R^5$ étant liés ensemble autrement que de la manière définie ci-dessus;

et $R^7$ est l'hydrogène ou un groupe alkyle ayant jusqu'à 5 atomes de carbone;

ou un sel de ce dérivé.

4. Dérivé amidique de formule:

$$R^1CONH—CHR^3—COCH_2—CHR^4—CONR^5—CHR^6—COR^{10}$$

dans laquelle $R^1$ est un groupe méthyle, benzyle, $\beta$-phényléthyle, benzyloxy, phénoxyméthyle, phénylthiométhyle, phényle, tolyle, méthoxyphényle, méthylthiophényle, monochlorophényle, dichlorophényle, fluorophényle, iodophényle, nitrophényle, cyanophényle, trifluorométhylphényle, acétamidophényle, acétylphényle, méthanesulfonylphényle, biphénylyle, naphtyle, benzamidométhyle, cyclopentanecarboxamidométhyle ou $\beta$-benzoyléthyle;

$R^3$ est un groupe benzyle qui n'est pas substitué ou qui porte un substituant méthyle, tertio-butyle, méthoxy, fluoro, chloro, bromo, nitro, cyano, trifluorométhyle, amino, acétamido, méthanesulfamido, trifluorométhanesulfamido ou phényle, ou bien $R^3$ est un groupe 3-phénylpropyle ou 3-phénylprop-2-ényle;

$R^4$ est l'hydrogène ou un groupe méthyle;

$R^5$ est l'hydrogène ou un groupe méthyle et $R^6$ est l'hydrogène, un groupe benzyle ou un groupe indole-3-ylméthyle, ou bien $R^5$ et $R^6$ forment conjointement un groupe triméthylène, tétraméthylène, pentaméthylène, 2-hydroxytriméthylène, 2-thiatriméthylène, 3-thiatétraméthylène, 3-oxatétraméthylène, 3-méthyl-3-azatétraméthylène; et

$R^{10}$ est un groupe hydroxy, amino, alkoxy ayant jusqu'à 5 atomes de carbone, ou un groupe $\beta$-diméthylaminoéthoxy ou $\beta$-morpholino-éthoxy;

ou un sel de ce dérivé.

5. Dérivé amidique de formule:

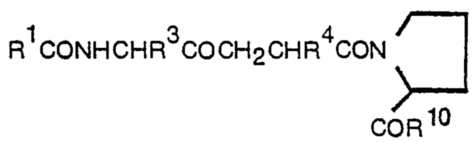

dans laquelle $R^1$ est un groupe phényle, *p*-acétamidophényle ou *p*-cyanophényle, $R^3$ est un groupe benzyle, *p*-méthylbenzyle ou *p*-méthoxybenzyle, $R^4$ est l'hydrogène ou le groupe méthyle et $R^{10}$ est un groupe hydroxy, amino, méthoxy, éthoxy, isopropoxy ou tertio-butoxy, ou un sel de ce dérivé.

6. La *N*-(5-acétamido-2-méthyl-4-oxo-6-phénylhexanoyl)-L-proline, la *N*-(5-benzamido-2-méthyl-4-oxo-6-phénylhexanoyl)-L-proline ou la *N*-(5-phénylacétamido-2-méthyl-4-oxo-6-phénylhexanoyl)-L-proline ou un sel de ce composé, et/ou un ester de ce composé avec un alcool ayant jusqu'à 5 atomes de carbone, ou un amide de ce composé.

7. La *N*-(5-benzamido-2-méthyl-4-oxo-6-phénylhexanoyl)-L-proline ou son ester de méthyle ou son amide ou un sel de ce composé.

8. Sel de dérivé amidique suivant l'une quelconque des revendications 1 à 7, dans lequel $R^{10}$ est un groupe hydroxy, qui est un sel de métal alcalin ou de métal alcalino-terreux ou un sel d'ammonium ou de dicyclohexylamine.

9. Procédé de production d'un dérivé amidique ou d'un sel de ce dérivé suivant l'une quelconque des revendications 1 à 8, qui comprend (a) la réaction d'un acide de formule:

$$R^1—Y—NR^2—CHR^3—X—CHR^{11}—CHR^4—COOH$$

dans laquelle $R^1$, $R^2$, $R^3$, $R^4$, $R^{11}$, X et Y ont les définitions indiquées dans l'une quelconque des revendications 1 à 5, ou d'un dérivé réactif de cet acide, avec une amine de formule:

$$HNR^5—CR^6R^{16}—Q—R^{12}$$

dans laquelle $R^5$, $R^6$, $R^{16}$ et Q ont les définitions indiquées dans l'une quelconque des revendications 1 à 5 et $R^{12}$ a l'une quelconque des définitions indiquées dans les revendications 1 à 5 pour $R^{10}$, excepté celles qui comprennent un groupe hydroxy ou amino libre; ou

(b) pour la préparation d'un dérivé amidique dans lequel $R^2$ est l'hydrogène et X est le groupe carbonyle ou thiocarbonyle, la réaction d'une amine de formule:

$$H_2NCHR^3—X^1—CHR^{11}CHR^4CONR^5—CR^6R^{16}—Q—R^{12}$$

dans laquelle $R^3$, $R^4$, $R^5$, $R^6$, $R^{11}$, $R^{12}$, $R^{16}$ et Q ont les définitions données ci-dessus et X' est le groupe carbonyle ou thiocarbonyle, avec un composé de formule $R^1—Y—Z^1$, dans lequel $R^1$ et Y ont les définitions données ci-dessus et $Z^1$ est un halogène, un groupe alcanesulfonyle ou un groupe arènesulfonyle pouvant être déplacé, ou bien $Z^1$ est un groupe hydroxy (auquel cas la réaction est conduite en présence d'un carbodiimide ou d'un chloroformiate d'alkyle), ou bien, lorsque Y est un groupe amido, avec un composé de formule $R^1$ NCO, ou bien, lorsque Y est le groupe thiocarbonyle, avec un composé de formule $R^1CS—SCH_2COOH$; ou

(c) pour la préparation d'un dérivé amidique dans lequel $R^2$ est l'hydrogène et X est le groupe carbonyle, la réaction d'un composé de formule:

$$Z^2COCHR^{11}CHR^4CONR^5CR^6R^{16}QR^{12}$$

dans laquelle $R^4$, $R^5$, $R^6$, $R^{11}$, $R^{12}$, $R^{16}$ et Q ont les définitions indiquées ci-dessus et $Z^2$ est un atome d'halogène déplaçable, avec un composé de formule:

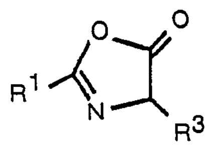

dans laquelle $R^1$ et $R^3$ ont les définitions indiquées ci-dessus, suivie de l'hydrolyse du noyau d'oxazolone; après quoi un dérivé amidique dans lequel $R^2$ est un groupe alkyle, arylalkyle ou aryle (et $R^{10}$ est un groupe $R^{12}$) peut être obtenu par réaction du dérivé amidique correspondant dans lequel $R^2$ est l'hydrogène (et $R^{10}$ est un groupe $R^{12}$) avec un composé de formule $R^2Z$, dans laquelle $R^2$ a la définition indiquée ci-dessus et Z est un halogène, un groupe alcanesulfonyle ou un groupe arènesulfonyle déplaçable;

ou bien un dérivé amidique dans lequel $R^3$ est un groupe alkyle non substitué ou substitué tel que défini ci-dessus peut être obtenu ensuite par hydrogénation du composé correspondant dans lequel $R^3$ est un groupe alcényle non substitué ou substitué tel que défini ci-dessus; ou bien un dérivé amidique dans lequel $R^{10}$ représente autre chose qu'un groupe hydroxy (c'est-à-dire dans lequel —Q—$R^{10}$ forme un groupe ester ou amide) peut ensuite être obtenu à partir de l'acide correspondant dans lequel —Q—$R^{10}$ est un groupe —COOH par des moyens classiques de formation d'un ester ou d'un amide;

ou bien un dérivé amidique dans lequel Q est le groupe carbonyle et $R^{10}$ est le groupe hydroxy peut être obtenu ensuite par hydrolyse du dérivé amidique correspondant dans lequel $R^{10}$ est un groupe alkoxy, ou bien, lorsque $R^{10}$ est un groupe tertio-butoxy, par clivage, catalysé par un acide, dudit composé;

ou bien un dérivé amidique dans lequel X est un groupe hydroxyméthylène peut être obtenu ensuite par réduction du dérivé amidique correspondant dans lequel X est le groupe carbonyle;

ou bien un dérivé amidique dans lequel X est un groupe oximinométhylène peut être obtenu ensuite par réaction du dérivé amidique correspondant dans lequel X est le groupe carbonyle, avec l'hydroxylamine ou un dérivé d'hydroxylamine substitué sur l'atome de *O*, de formule $H_2NOR^{20}$ dans laquelle $R^{20}$ a la définition indiquée dans la revendication 1.

10. Composition pharmaceutique comprenant comme ingrédient actif au moins un dérivé amidique ou un sel de ce dérivé suivant l'une quelconque des revendications 1 à 8, en association avec un diluant ou support pharmaceutiquement acceptable.

**Revendications pour l'Etat contractant: AT**

1. Procédé de production d'un dérivé amidique de formule:

$$R^1—Y—NR^2—CHR^3—X—CHR^{11}—CHR^4—CO—NR^5—CR^6R^{16}—Q—R^{10}$$

dans laquelle $R^1$ est l'hydrogène ou un groupe alkyle ayant jusqu'à 15 atomes de carbone, qui n'est pas substitué ou qui porte un substituant choisi entre des substituants amino, benzyloxycarbonylamino, hydroxy, alkoxy, alkylthio et alkoxycarbonyle ayant chacun jusqu'à 5 atomes de carbone, et des substituants aryle, aryloxy et arylthio; ou qui porte deux ou trois substituants dont l'un est un substituant aryle, un autre est un substituant aryle, hydroxy, amino, benzyloxycarbonylamino, trifluorométhyle, aryloxy ou alkoxy ayant jusqu'à 5 atomes de carbone et dont le troisième, s'il est présent, est un substituant aryle ou trifluorométhyle;

ou bien $R^1$ est un groupe aryle ou, lorsque Y est un groupe carbonyle ou thiocarbonyle, $R^1$ est un groupe aryloxy, alkoxy ou arylalkoxy dont la portion alkoxy a jusqu'à 5 atomes de carbone;

ou bien $R^1$ est un groupe halogénalkyle ayant jusqu'à 6 atomes de carbone ou un groupe alcényle, halogénalcényle ou cycloalkyle, chacun ayant jusqu'à 6 atomes de carbone et n'étant pas substitué ou portant un substituant aryle;

ou bien $R^1$ est un substituant de formule $R^8CONHCHR^9$— ou $R^8COCH_2CHR^9$— où $R^8$ est un groupe alkyle ou cycloalkyle, chacun ayant jusqu'à 10 atomes de carbone, ou un groupe aryle et $R^9$ est l'hydrogène, ou un groupe alkyle ayant jusqu'à 5 atomes de carbone, qui n'est pas substitué ou qui porte un substituant aryle, ou bien $R^9$ est un groupe autre que ceux qui sont mentionnés ci-dessus, de manière que le composé $H_2NCHR^9COOH$ soit un amino-acide naturel; $R^2$ est l'hydrogène, un groupe alkyle ayant jusqu'à 5 atomes de carbone qui n'est pas substitué ou qui porte un substituant aryle, ou un groupe aryle;

$R^3$ est un groupe alkyle ou un groupe alcényle, chacun ayant jusqu'à 5 atomes de carbone, qui n'est pas substitué ou qui porte un substituant aryle, ou bien $R^3$ est un groupe aryle ou indolylméthyle;

$R^4$ est l'hydrogène ou un groupe alkyle ayant jusqu'à 5 atomes de carbone, qui n'est pas substitué ou qui porte un substituant aryle; $R^5$ est l'hydrogène ou un groupe aryle, ou un groupe alkyle ayant jusqu'à 5 atomes de carbone qui n'est pas substitué ou qui porte un substituant aryle, ou bien $R^5$ s'associe avec $R^6$ comme défini ci-dessous;

$R^6$ est l'hydrogène, un groupe aryle ou un groupe hétérocyclique, ou un groupe alkyle ayant jusqu'à 5 atomes de carbone qui n'est pas substitué ou qui porte un substituant hydroxy, aryle ou hétérocyclique;

ou bien $R^6$ et $R^5$ forment conjointement un groupe alkylène ou alcénylène de 2 à 5 atomes de carbone; ou un dérivé oxa, thia ou aza dudit alkylène ou alcénylène; ou un dérivé à substituant hydroxy ou oxo dudit alkylène ou alcénylène; ou bien $R^6$ et $R^{16}$, ou $R^6$, $R^{16}$ et $R^5$, ou $R^6$ et $R^{10}$ s'associent de la manière définie ci-dessous;

$R^{16}$ est l'hydrogène ou un groupe alkyle ayant jusqu'à 5 atomes de carbone;

ou bien $R^6$ et $R^{16}$ s'associent pour former un groupe alkylène ayant 2 à 5 atomes de carbone (c'est-à-dire pour former un groupe spirocycloalkyle);

ou bien $R^{16}$ forme conjointement avec le premier atome de carbone de $R^6$ une double liaison, $R^6$ désignant autre chose qu'un groupe alkyle, ou bien $R^6$ est un groupe alkyle autrement substitué comme défini ci-dessus, ou bien $R^6$ et $R^5$ s'associent d'une autre façon comme défini ci-dessus (c'est-à-dire de manière que $R^5$, $R^6$ et $R^{16}$ forment conjointement un groupe alkylidène);

Q est un groupe carbonyle (—CO—) ou méthylène (—$CH_2$—);

et $R^{10}$ est un groupe hydroxy, amino, aryloxy, arylthio, alkoxy, cycloalkoxy, alkylamino, dialkylamino, amino cyclique, hydroxyalkoxy, acyloxyalkoxy, aminoalkoxy, alkylaminoalkoxy, dialkylaminoalkoxy, aminoalkoxy cyclique, aminoalkylamino, alkylaminoalkylamino, dialkylaminoalkylamino, aminoalkylamino cyclique ou arylalkoxy, chaque radical alkyle ou alkoxy ayant jusqu'à 5 atomes de carbone et le groupe amino cyclique ayant jusqu'à 6 atomes de carbone;

ou bien $R^{10}$ et $R^6$ s'associent de façon telle que $R^{10}$ est un atome d'oxygène (—O—) lié à l'atome terminal de carbone de $R^6$ lorsque c'est un groupe alkyle;

$R^{11}$ est l'hydrogène ou un groupe alkyle ayant jusqu'à 3 atomes de carbone;

X est un groupe carbonyle (—CO—), hydroxyméthylène (—CHOH—), thiocarbonyle (—CS—) ou oximinométhylène (—C=N—$OR^{20}$) où $R^{20}$ est l'hydrogène ou un radical alkyle ayant jusqu'à 5 atomes de carbone, qui n'est pas substitué ou qui porte un substituant aryle);

et Y est un groupe carbonyle, thiocarbonyle, sulfonyle (—$SO_2$—) ou amido (—NHCO—);

ou un sel de ce dérivé dans les cas appropriés, caractérisé par:

(a) la réaction d'un acide de formule:

$$R^1—Y—NR^2—CHR^3—X—CHR^{11}—CHR^4—COOH$$

dans laquelle $R^1$, $R^2$, $R^3$, $R^4$, $R^{11}$, X et Y ont les définitions indiquées ci-dessus, ou d'un dérivé réactif de cet acide, avec une amine de formule:

$$HNR^5—CR^6R^{16}—Q—R^{12}$$

dans laquelle $R^5$, $R^6$, $R^{16}$ et Q ont les définitions données ci-dessus et $R^{12}$ a l'une quelconque des définitions indiquées ci-dessus pour $R^{10}$, excepté les définitions comprenent un groupe hydroxy ou amin libre; ou

(b) pour l'obtention d'un dérivé amidique dans lequel $R^2$ est l'hydrogène et X est le groupe carbonyle ou thiocarbonyle, la réaction d'une amine de formule:

$$H_2NCHR^3—X^1—CHR^{11}—CHR^4CONR^5—CR^6R^{16}—Q—R^{12}$$

dans laquelle $R^3$, $R^4$, $R^5$, $R^6$, $R^{11}$, $R^{12}$, $R^{16}$ et Q ont les définitions données ci-dessus et $X^1$ est le groupe carbonyle ou thiocarbonyle, avec un composé de formule $R^1$—Y—$Z^1$, dans lequel $R^1$ et Y ont les définitions indiquées ci-dessus et $Z^1$ est un halogène, un groupe alcanesulfonyle ou un groupe arène-sulfonyle déplaçable, ou bien $Z^1$ est le groupe hydroxy (auquel cas la réaction est conduite en présence d'un carbodiimide ou d'un chloroformiate d'alkyle), ou bien, lorsque Y est un groupe amido, avec un composé de formule $R^1NCO$, ou bien, lorsque Y est le groupe thiocarbonyle, avec un composé de formule $R^1CS—SCH_2COOH$; ou bien

(c) pour l'obtention d'un dérivé amidique dans lequel $R^2$ est l'hydrogène et X est un groupe carbonyle, la réaction d'un composé de formule:

$$Z^2COCHR^{11}CHR^4CONR^5CR^6R^{16}QR^{12}$$

dans laquelle $R^4$, $R^5$, $R^6$, $R^{11}$, $R^{12}$, $R^{16}$ et Q ont les définitions données ci-dessus et $Z^2$ est un atome d'halogène déplaçable, avec un composé de formule:

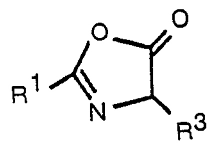

dans laquelle $R^1$ et $R^3$ ont les définitions indiquées ci-dessus, suivie de l'hydrolyse du noyau d'oxazolone; après quoi un dérivé amidique dans lequel $R^2$ est un groupe alkyle, arylalkyle ou aryle (et $R^{10}$ a la valeur de $R^{12}$) peut être obtenu par réaction du dérivé amidique correspondant dans lequel $R^2$ est l'hydrogène (et $R^{10}$ a la valeur de $R^{12}$) avec un composé de formule $R^2Z$, dans laquelle $R^2$ a la définition indiquée ci-dessus et Z est un halogène, un groupe alcanesulfonyle ou un groupe arènesulfonyle déplaçable;

ou bien un dérivé amidique dans lequel $R^3$ est un groupe alkyle non substitué ou substitué tel que défini ci-dessus peut être obtenu ensuite par hydrogénation du composé correspondant dans lequel $R^3$ est un groupe alcényle non substitué ou substitué tel que défini ci-dessus; ou bien un dérivé amidique dans lequel $R^{10}$ représente autre chose que le groupe hydroxy (c'est-à-dire dans lequel —Q—$R^{10}$ forme un groupe ester ou amide) peut être obtenu ensuite à partir de l'acide correspondant dans lequel —Q—$R^{10}$ représente —COOH par des moyens classiques de formation d'un ester ou d'un amide;

ou bien un dérivé amidique dans lequel Q est le groupe carbonyle et $R^{10}$ est le groupe hydroxy peut être obtenu ensuite par hydrolyse du dérivé amidique correspondant dans lequel $R^{10}$ est un groupe alkoxy ou bien, lorsque $R^{10}$ est un groupe tertio-butoxy, par clivage, catalysé par un acide, dudit composé:

ou bien un dérivé amidique dans lequel X est un groupe hydroxyméthylène peut être obtenu ensuite par réduction du dérivé amidique correspondant dans lequel X est le groupe carbonyle;

ou bien un dérivé amidique dans lequel X est un groupe oximinométhylène peut être obtenu ensuite par réaction du dérivé amidique correspondant dans lequel X est le groupe carbonyle, avec l'hydroxylamine ou un dérivé d'hydroxylamine substitué sur l'atome d'oxygène, de formule $H_2NOR^{20}$ dans laquelle $R^{20}$ a la définition indiquée ci-dessus.

2. Procédé de production d'un dérivé amidique de formule:

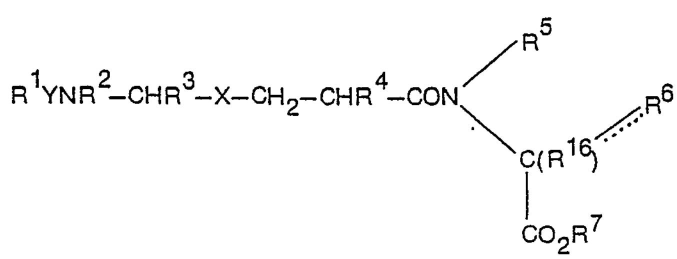

dans laquelle $R^1$ est un groupe alkyle ayant jusqu'à 15 atomes de carbone qui n'est pas substitué ou qui porte un substituant aryle, aryloxy ou arylthio, ou bien $R^1$ est un groupe aryle ou aryloxy, ou bien $R^1$ est un substituant de formule $R^8CONHCHR^9$— ou $R^8COCH_2CHR^9$— dans laquelle $R^8$ est un radical alkyle ou cycloalkyle, chacun ayant jusqu'à 10 atomes de carbone, ou un substituant aryle, et $R^9$ est l'hydrogène, un groupe alkyle ayant jusqu'à 5 atomes de carbone, qui n'est pas substitué ou qui porte un substituant aryle, ou bien $R^9$ est un groupe autre que ceux qui ont été indiqués ci-dessus, de telle sorte que le composé $H_2NCHR^9COOH$ soit un amino-acide naturel;

Y et $R^2$ ont les définitions données dans la revendication 1; $R^3$ est un groupe alkyle ou alcényle, chacun ayant jusqu'à 5 atomes de carbone, qui n'est pas substitué ou qui porte un substituant aryle, ou bien $R^3$ est un groupe aryle ou indolylméthyle; X est un groupe carbonyle, hydroxyméthylène, thiocarbonyle ou oximinométhylène (>C=N—OH);

$R^4$ est l'hydrogène ou un groupe alkyle ayant jusqu'à 5 atomes de carbone, qui n'est pas substitué ou qui porte un substituant aryle; $R^5$ est l'hydrogène, un groupe aryle ou un groupe alkyle ayant jusqu'à 5 atomes de carbone, qui n'est pas substitué ou qui porte un substituant aryle, $R^6$ est l'hydrogène, un groupe alkyle ayant jusqu'à 5 atomes de carbone qui n'est pas substitué ou qui porte un substituant aryle ou hétérocyclique, ou bien $R^6$ est un substituant hétérocyclique ou aryle et $R^{16}$ est l'hydrogène;

ou bien $R^5$ et $R^6$ s'associent pour former un groupe alkylène ou alcénylène ayant 2 à 4 atomes de carbone; ou un dérivé oxa, thia ou aza dudit alkylène ou alcénylène;

ou bien $R^6$, $R^{16}$ et $R^5$ s'associent de telle manière que $R^{16}$ forme avec le premier atome de carbone de $R^6$ une double liaison, $R^6$ et $R^5$ étant autrement liés ensemble comme définie ci-dessus;

et $R^7$ est l'hydrogène ou un groupe alkyle ayant jusqu'à 5 atomes de carbone;

ou d'un sel de ce dérivé, caractérisé par la réaction d'un acide de formule:

$$R^1—Y—NR^2—CHR^3—X—CH_2—CHR^4—COOH$$

dans laquelle $R^1$, $R^2$, $R^3$, $R^4$, X et Y ont les définitions données ci-dessus, ou d'un dérivé réactif de cet acide, avec une amine de formule:

$$HNR^5—\overset{R^6}{\overset{\vdots}{C}}(R^{16})—CO_2R^{12}$$

dans laquelle $R^5$, $R^6$ et $R^{16}$ ont les définitions données ci-dessus et $R^{12}$ est un groupe alkyle ayant jusqu'à 5 atomes de carbone; après quoi un dérivé amidique dans lequel $R^2$ est un groupe alkyle, arylalkyle ou aryle (et $R^7$ est un groupe alkyle) peut être obtenu par réaction du dérivé amidique correspondant dans lequel $R^2$ est l'hydrogène (et $R^7$ est un groupe alkyle) avec un composé de formule $R^2Z$ dans laquelle $R^2$ a la définition indiquée ci-dessus et Z est un halogène, un groupe alcanesulfonyle ou un groupe arènesulfonyle déplaçable;

ou bien un dérivé amidique dans lequel $R^7$ est l'hydrogène peut être obtenu ensuite par l'hydrolyse du dérivé amidique correspondant dans lequel $R^7$ est un groupe alkyle ou bien, lorsque $R^7$ est un groupe tertiobutyle, par clivage catalysé par un acide dudit composé;

ou bien un dérivé amidique dans lequel X est un groupe hydroxyméthylène peut être obtenu ensuite par réduction du dérivé amidique correspondant dans lequel X est le groupe carbonyle;

ou bien un dérivé amidique dans lequel X est un groupe oximinométhylène peut être obtenu ensuite par réaction du dérivé amidique correspondant dans lequel X est un groupe carbonyle, avec l'hydroxylamine.

3. Procédé de production d'un dérivé amidique de formule:

$$R^1CONH—CHR^3—COCH_2—CHR^4—CONR^5—CHR^6—COR^{10}$$

dans laquelle $R^1$ est le groupe méthyle, benzyle, β-phényléthyle, benzyloxy, phénoxyméthyle, phénylthiométhyle, phényle, tolyle, méthoxyphényle, méthylthiophényle, monochlorophényle, dichlorophényle, trifluorométhylphényle, acétamidophényle, acétylphényle, méthanesulfonylphényle, biphénylyle, naphtyle, benzamidométhyle, cyclopentanecarboxamidométhyle ou β-benzoyléthyle;

$R^3$ est un groupe benzyle qui n'est pas substitué ou qui porte un substituant méthyle, tertio-butyle, méthoxy, fluoro, chloro, bromo, nitro, cyano, trifluorométhyle, amino, acétamido, méthanesulfamido, trifluorométhanesulfamido ou phényle, ou bien $R^3$ est un groupe 3-phénylpropyle ou 3-phénylprop-2-ényle;

$R^4$ est l'hydrogène ou le groupe méthyle;

$R^5$ est l'hydrogène ou le groupe méthyle et $R^6$ est l'hydrogène, le groupe benzyle ou le groupe indole-3-ylméthyle, ou bien $R^5$ et $R^6$ forment conjointement un groupe triméthylène, tétraméthylène, pentaméthylène, 2-hydroxytriméthylène, 2-thiatriméthylène, 3-thiatétraméthylène, 3-oxatétraméthylène, 3-méthyl-3-azatétraméthylène; et

$R^{10}$ est un groupe hydroxy, amino, alkoxy ayant jusqu'à 5 atomes de carbone, ou β-diméthylaminoéthoxy ou β-morpholino-éthoxy;

ou d'un sel de ce dérivé, caractérisé par

(a) la réaction d'un acide de formule:

$$R^1CONH—CHR^3—COCH_2—CHR^4—COOH$$

dans laquelle $R^1$, $R^3$ et $R^4$ ont les définitions indiquées ci-dessus, ou d'un dérivé réactif de cet acide, avec une amine de formule:

$$HNR^5—CHR^6—COR^{12}$$

dans laquelle $R^5$ et $R^6$ ont les définitions indiquées ci-dessus et dans laquelle $R^{12}$ a l'une quelconque des définitions données ci-dessus pour $R^{10}$, excepté un groupe hydroxy et un groupe amino; ou

(b) la réaction d'une amine de formule:

$$H_2NCHR^3—COCH_2—CHR^4—CONR^5—CHR^6—COR^{12}$$

dans laquelle $R^3$, $R^4$, $R^5$, $R^6$ et $R^{12}$ ont les définitions indiquées ci-dessus, avec un composé de formule $R^1—COZ^1$, dans laquelle $R^1$ a la définition indiquée ci-dessus et $X^1$ est un halogène, un groupe alcanesulfonyle ou un groupe arènesulfonyle déplaçable, ou bien $Z^1$ est un groupe hydroxy (auquel cas la réaction est conduite en présence d'un carbodiimide ou d'un chloroformiate d'alkyle); ou

(c) is réaction d'un composé de formule:

$$Z^2COCH_2CHR^4CONR^5CHR^6COR^{12}$$

dans laquelle $R^4$, $R^5$, $R^6$ et $R^{12}$ ont les définitions indiquées ci-dessus et $Z^2$ est un atome d'halogène déplaçable, avec un composé de formule:

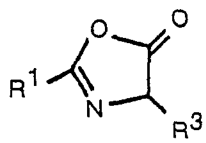

dans laquelle $R^1$ et $R^3$ ont les définitions indiquées ci-dessus, suivie de l'hydrolyse du noyau d'oxazolone; après quoi un dérivé amidique dans lequel $R^3$ est le groupe 3-phénylpropyle peut être obtenu par l'hydrogénation du composé correspondant dans lequel $R^3$ est le groupe 3-phénylprop-2-ényle; un dérivé amidique dans lequel $R^{10}$ est le groupe hydroxy peut être obtenu ensuite par l'hydrolyse du dérivé amidique correspondant dans lequel $R^{10}$ est un groupe alkoxy ou bien, lorsque $R^{10}$ est le groupe tertiobutoxy, par le clivage catalysé par un acide dudit composé; et un dérivé amidique dans lequel $R^{10}$ représente autre chose qu'un groupe hydroxy (c'est-à-dire dans lequel $COR^{10}$ forme un groupe ester ou amide) peut être obtenu ensuite à partir de l'acide correspondant dans lequel $—CO—R^{10}$ est un groupe $—COOH$, par des moyens classiques de formation d'un ester ou d'un amide.

4. Procédé suivant la revendication 3, dans lequel $R^1$, dans les matières de départ, est un groupe phényle, *p*-acétamidophényle ou *p*-cyanophényle, $R^3$ est un groupe benzyle, *p*-méthylbenzyle ou *p*-méthoxybenzyle, $R^4$ est l'hydrogène ou le groupe méthyle, $R^5$ et $R^6$ forment conjointement un groupe triméthylène et $R^{10}$ est un groupe hydroxy, amino, méthoxy, éthoxy, isopropoxy ou tertio-butoxy.